# EUROPEAN PATENT APPLICATION

(11) **EP 2 428 563 A1**
(43) Date of publication of application: **14.03.2012**
(21) Application number: 11160913.7
(22) Date of filing: 10.02.2006
(51) Int. Cl.: C12N 5/0775

(54) **Vascular/lymphatic endothelial cells**

(30) Priority: 10.02.2005 US 652015 P
(62) Divisional of application: 06720616.9
(71) Applicant: REGENTS OF THE UNIVERSITY OF MINNESOTA, Saint Paul, MN 55114-8658 (US); Proyecto De Biomedicina Cima, S.L., 31008 Navarra (ES)
(72) Inventor: Prosper, Felipe, Pamplona E-31008 Navarra (ES); Verfaillie, Catherine M., White Bear Lake, MN 55110 (US); Lopez-Aranguren, Xabier, E-31610 Pamplona Navarra (ES); Claver, Carlos Clavel, E-31009 Pamplona Navarra (ES); Luttun, Aernout, B-8750 Zwevezele (Wingene) (BE)
(74) Representative: Lucas, Brian Ronald

(57) **Abstract**

A method to differentiate cells comprising contacting an enriched population of stem cells that differentiate into more than one embryonic lineage with VEGF₁₆₅, VEGF-C, VEGF-D or a combination thereof, so as to increase expression of one or more of prox-1, podoplanin or lyve-1 relative to the initial population.

## Description

### Cross-Reference to Related Applications

This application claims priority under 35 U.S.C. 119(e) from U.S. Provisional Application Serial No. 60/652,015 filed February 10, 2005, which is herein incorporated by reference.

### Field of the Invention

This invention relates to methods and compositions for differentiation of non-embryonic stem cells to cells of the endothelial lineage, particularly a vascular endothelial lineage with arterial, venous and/or lymphatic endothelial characteristics, culture conditions therefor and uses thereof.

### Background of the Invention

The vascular system is a bipolar complex network of arteries that transport oxygen-rich blood to all tissues, and veins that bring oxygen-deprived blood back to the heart (Carmeliet, 2004). The vascular system is the first system to develop in the embryo. Vasculogenesis, the *in situ* differentiation of primitive endothelial progenitors, termed angioblasts, into endothelial cells that aggregate into a primary capillary plexus is responsible for the development of the vascular system during embryogenesis (Hirashima et al., 1999). In contrast, angiogenesis, defined as the formation of new blood vessels by a process of sprouting from preexisting vessels, occurs both during development and in postnatal life (Holash et al., 1999; Yang et al., 2001).

Previously, it was thought that blood vessel formation in post-natal life was mediated only by the "sprouting" of endothelial cells from existing vessels. However, recent studies have suggested that endothelial "stem cells" may persist into adult life, where they contribute to the formation of new blood vessels (Peichev et al., 2000; Lin et al., 2000; Gehling et al., 2000; Asahara et al., 1997; Shi et al., 1998), suggesting, that as it happens in normal development, neoangiogenesis in the adult, may at least in part depend on a process of vasculogenesis.

Aside from functional differences, arteries and veins feature several anatomical and molecular differences. Unlike venous endothelium, arterial endothelium is surrounded by several layers of smooth muscle cells (SMCs), separated by elastic laminae and embedded in a thick layer of fibrillar collagen (Torres-Vazquez et al., 2003). Arterial and venous ECs also have a different molecular signature, and such molecular specification occurs before the onset of blood flow (Jain, 2003). Arterio-venous (AV) specification of ECs is accomplished early in development and is associated with the expression of a specific complement of receptors and growth factors: venous endothelium is characterized by the expression of EphB4 (Bagley et al, 2003), Lefty-1 (Chi et al,. 2003), Lefty-2 (Chi et al., 2003), COUP-TFII (You et al., 2003) and MYO1-β (Chi et al., 2003), arterial ECs express high levels of Notch 1 and 4 (Villa et al., 2001), Dll-4 (Shutter et al. 2000), EphrinB1 and B2 (Bagley et al., 2003), Jagged-1 and 2 (Villa et al,. 2001), connexin-40, and Hey-2 (Gridlock zebrafish orthologue; Zhong et al., 2001; Zhong et al,. 2000), and lymphatic endothelial cells express and podoplanin, prox-1 and lyve-1 (Conway, 2001; Oettgen, 2001; Partanen, 2001).

Studies in *Xenopus,* zebrafish and mice have revealed that, besides blood flow (le Noble et al,. 2004), a number of vessel-intrinsic cues and, later in development, signals from outside the vasculature (Othman-Hassan et al., 2001; Mukouyama et al., 2002) are implicated in defining arterial or venous fate, such as members of the TGF-β pathway (Waite and Eng, 2003; Sorensen et al., 2003), VEGF isoforms (Mukouyama et al., 2002; Stalmans et al., 2002; Mukouyama et al., 2005; Cleaver and Krieg, 1998), neuropilins (Mukouyama et al,. 2005), angiopoietins (Moyon et al,. 2001), the Notch pathway (Villa et al., 2001; Zhong et al., 2001; Lawson et al., 2002; Liu et al., 2003), the patched pathway (Lawson et al., 2002) and COUP-TFII, a member of the orphan nuclear receptor superfamily (You et al., 2005). Although it has been shown that some of these pathways are well conserved from zebrafish to mouse, less information is available on whether they have a similar role in humans. Furthermore, the signals involved in arterial, venous or lymphatic endothelium fate are only starting to be unraveled.

### Stem Cells

The embryonic stem (ES) cell has unlimited self-renewal and can differentiate into all tissue types. ES cells are derived from the inner cell mass of the blastocyst or primordial germ cells from a post-implantation embryo (embryonic germ cells or EG cells). ES (and EG) cells can be identified by positive staining with antibodies to SSEA 1 (mouse) and SSEA 4 (human). At the molecular level, ES and EG cells express a number of transcription factors specific for these undifferentiated cells. These include Oct-4 and rex-1. Rex expression depends on Oct-4. Also found are the LIF-R (in mouse) and the transcription factors sox-2 and rox-1. Rox-1 and sox-2 are also expressed in non-ES cells. Another hallmark of ES cells is the presence of telomerase, which provides these cells with an unlimited self-renewal potential *in vitro.*

Oct-4 (Oct-3 in humans) is a transcription factor expressed in the pregastrulation embryo, early cleavage stage embryo, cells of the inner cell mass of the blastocyst, and embryonic carcinoma (EC) cells (Nichols J., et al 1998), and is down-regulated when cells are induced to differentiate. Expression of Oct-4 plays an important role in determining early steps in embryogenesis and differentiation. Oct-4, in combination with Rox-1, causes transcriptional activation of the Zn-finger protein Rex-1, also required for maintaining ES in an undifferentiated state (Rosfjord and Rizzino A. 1997; Ben-Shushan E, et al. 1998). In addition, sox-2, expressed in ES/EC, but also in other more differentiated cells, is needed together with Oct-4 to retain the undifferentiated state of ES/EC (Uwanogho D et al. 1995). Maintenance of murine ES cells and primordial germ cells requires LIF.

The Oct-4 gene (Oct-3 in humans) is transcribed into at least two splice variants in humans, Oct-3A and Oct-3B. The Oct-3B splice variant is found in many differentiated cells whereas the Oct-3A splice variant (also designated Oct-3/4) is reported to be specific for the undifferentiated embryonic stem cell (Shimozaki et al. 2003).

Stem cells derived from different tissues have demonstrated their potential to differentiate *in vitro* and *in vivo* to mature and functional endothelial cells (Asahara, 1997; Gehling, 2000; Salven, 2003; Bagley, 2003; Pelosi, 2002). However, the specific venous or arterial potential of different types of stem cells has not been analyzed. Furthermore, stem cells, with the capacity to differentiate into both endothelial cells and smooth muscle cells, are needed for the regrowth of vessels or the formation of new vessels (Jain, 2003) to treat diseases and/or disorders of the vascular system. For example, ischemic disorders, such as atherosclerosis, are generally caused by obstruction of the arterial part of the vasculature, which results in oxygen deprivation and ultimately death of the target tissue. Hence, methods for revascularization, including those that induce arterial growth are needed. Additionally, culture and/or differentiation methods are needed to obtain these cells.

### Summary of the Invention

The invention is directed to methods and compositions for differentiating stem cells into cells with a vascular endothelial phenotype. The phenotype includes, but is not limited to arterial, venous and lymphatic vascular endothelial.

One embodiment provides a method to differentiate cells comprising contacting an enriched population of stem cells that differentiate into more than one embryonic lineage with VEGF₁₆₅, VEGF₁₂₁ or a combination thereof, so as to increase expression of one or more of Hey-2, EphrinB 1, EphrinB2 or EphB4 relative to the initial population (e.g., prior to contacting the cells with, e.g., VEGF₁₆₅, VEGF₁₂₁ or a combination thereof). Another embodiment provides a method to differentiate cells comprising contacting an enriched population of stem cells that differentiate into more than one embryonic lineage with (a) at least one vascular endothelial growth factor (VEGF) and (b) one or more notch ligands, one or more patched ligands or a combination thereof, so as to increase expression of one or more of Hey-2, EphrinB1 or EphrinB2 relative to the initial population. Another embodiment further comprises a decrease in the expression of EphB4 relative to the initial population. In one embodiment, the VEGF comprises VEGF₁₆₅, VEGF₁₂₁ or a combination thereof. In one embodiment, the notch ligand comprises Dll-1, Dll-3, Dll-4, Jagged-1, Jagged-2 or a combination thereof. In another embodiment, the patched ligand comprises sonic hedgehog. In one embodiment, the VEGF comprises VEGF₁₆₅, the notch ligand comprises Dll-4 and the patched ligand comprises sonic hedgehog.

One embodiment provides a method to differentiate cells comprising contacting an enriched population of stem cells that differentiate into more than one embryonic lineage with VEGF₁₆₅, VEGF-C, VEGF-D or a combination thereof, so as to increase expression of one or more of prox-1, podoplanin or lyve-1 relative to the initial population.

In one embodiment, the enriched population of cells are embryonic stem cells. In another embodiment, the enriched population of cells are non-embryonic stem, non-germ, non-embryonic germ cells (e.g., MAPCs). In another embodiment, the enriched population of stem cells are mammalian cells. Another embodiment further provides comprising admixing the differentiated cells with a pharmaceutically acceptable carrier. One embodiment further provides administering the differentiated cells to a subject.

One embodiment provides a composition comprising VEGF₁₆₅, VEGF₁₂₁ or a combination thereof and an enriched population of stem cells that differentiate into more than one embryonic lineage. Another embodiment, the composition further comprises the comprising cells differentiated from the enriched population of cells, wherein the expression of one or more of Hey-2, EphrinB1, EphrinB2 or EphB4 is increased in the differentiated cells.

Another embodiment comprises a composition comprising at least one vascular endothelial growth factor (VEGF) and at least one notch ligand, at least one patched ligand or a combination thereof and an enriched population of stem cells that differentiate into more than one embryonic lineage. In another embodiment, the VEGF comprises VEGF₁₂₁, VEGF₁₆₅ or a combination thereof. In another embodiment, the notch ligand comprises Dll-1, Dll-3, Dll-4, Jagged-1, Jagged-2 or a combination thereof. In one embodiment, the patched ligand comprises sonic hedgehog. In another embodiment, the VEGF comprises VEGF₁₆₅, the notch ligand comprises Dll-4, and the patched ligand comprises sonic hedgehog. In another embodiment the composition further comprises a pharmaceutically acceptable carrier or cell culture media.

In one embodiment the enriched population of cells are embryonic stem cells. In another embodiment, the enriched population of cells are non-embryonic stem, non-germ, non-embryonic germ cells. In another embodiment, the enriched population of stem cells are mammalian. In another embodiment the composition further comprises a pharmaceutically acceptable carrier or cell culture media.

Another embodiment provides a composition comprising the cells differentiated from an enriched population of stem cells by the method of any one of claims 1-13 and cell culture media or a pharmaceutically acceptable carrier.

Another embodiment provides a method to prepare a composition comprising admixing VEGF₁₆₅, VEGF₁₂₁ or a combination thereof and an enriched population stem cells that differentiate into at least one embryonic lineage. One embodiment further comprises cells differentiated from the enriched population of stem cells, wherein the expression of one or more of Hey-2, EphrinB1, EphrinB2 or EphB4 is increased in the differentiated cells.

One embodiment provides a method to prepare a composition comprising admixing at least one vascular endothelial growth factor (VEGF) and at least one notch ligand, at least one patched ligand or a combination thereof, and an enriched population of stem cells that differentiate into at least one embryonic lineage.

Another embodiment provides a method to prepare a composition comprising admixing the cells differentiated from the enriched population of stem cells by the methods disclosed herein and a carrier. In embodiment the carrier is cell culture media, while in another embodiment, the carrier is a pharmaceutically acceptable carrier.

Another embodiment further comprises admixing culture medium to a prepare a composition. One embodiment further comprises admixing a pharmaceutically acceptable carrier.

One embodiment provides a method to treat a vascular condition comprising administering to a subject need of said treatment an effective amount of a) a population of cells enriched in stem cells that differentiate into at least one embryonic cell type; b) a population of cells differentiated from the enriched population of cells, wherein the expression of one of more of Hey-2, EphrinB1, EphrinB2 or EphB4 is increased in the differentiated cells relative to the undifferentiated cells; or (c) a combination of (a) and (b).

Another embodiment provides a method to increase vasculogenesis, angiogenesis or a combination thereof in a subject comprising administering to a subject in need of said treatment an effective amount of a) a population of cells enriched in stem cells that differentiate into at least one embryonic cell type; b) a population of cells differentiated from the enriched population of cells, wherein the expression of one of more of Hey-2, EphrinB 1, EphrinB2 or EphB4 is increased in the differentiated cells relative to the undifferentiated cells; or (c) a combination of (a) and (b), so that vasculogenesis, angiogenesis or a combination thereof is increased in the subject following administration. In one embodiment, the vasculogenesis, angiogenesis or a combination thereof is increased in ischemic tissue, including connective tissue, muscle tissue, nerve tissue and organ tissue (e.g., heart tissue) of the subject.

Another embodiment provides a method to provide arterial cells comprising administering to a subject in need thereof an effective amount of a) a population of cells enriched in stem cells that differentiate into at least one embryonic cell type; b) a population of cells differentiated from the enriched population cells, wherein the expression of one of more of Hey-2, EphrinB1, EphrinB2 or EphB4 is increased in the differentiated cells relative to the undifferentiated cells; or (c) a combination of (a) and (b), wherein the non-embryonic stem, non-germ, non-embryonic germ cells, the cells differentiated therefrom, or a combination thereof provide arterial cells in the subject following administration.

In one embodiment, the subject is a mammal, such as a human. In another embodiment, the subject is afflicted with a vascular condition, such as ischemia, atherosclerosis, congestive heart failure, peripheral vasculature disorder, coronary vascular disease, hypertension, stroke, aneurysm, thrombosis, arrhythmia, tachycardia, or surgical or physical trauma. In one embodiment, the vascular condition is a myocardial infarction.

One embodiment provides a medical device comprising the cells differentiated from the enriched population of cells by any of the methods disclosed herein. In one embodiment, the device comprises a valve, stent, shunt or graft (e.g., an artificial vessel graft).

One embodiment provides for the use of the cells differentiated from the enriched population of cells by the methods disclosed herein to prepare a medicament for treating a vascular condition, including ischemia, atherosclerosis, congestive heart failure, peripheral vasculature disorder, coronary vascular disease, hypertension, stroke, aneurysm, thrombosis, arrhythmia or tachycardia, or surgical or physical trauma. In one embodiment, the medicament comprises a physiologically acceptable carrier.

Although directed to the differentiation of MAPCs into arterial, venous and/or lymphatic endothelial cells, the methods and uses of the invention described herein are applicable to other stem cells, including embryonic stem cells.

### Brief Description of the Figures

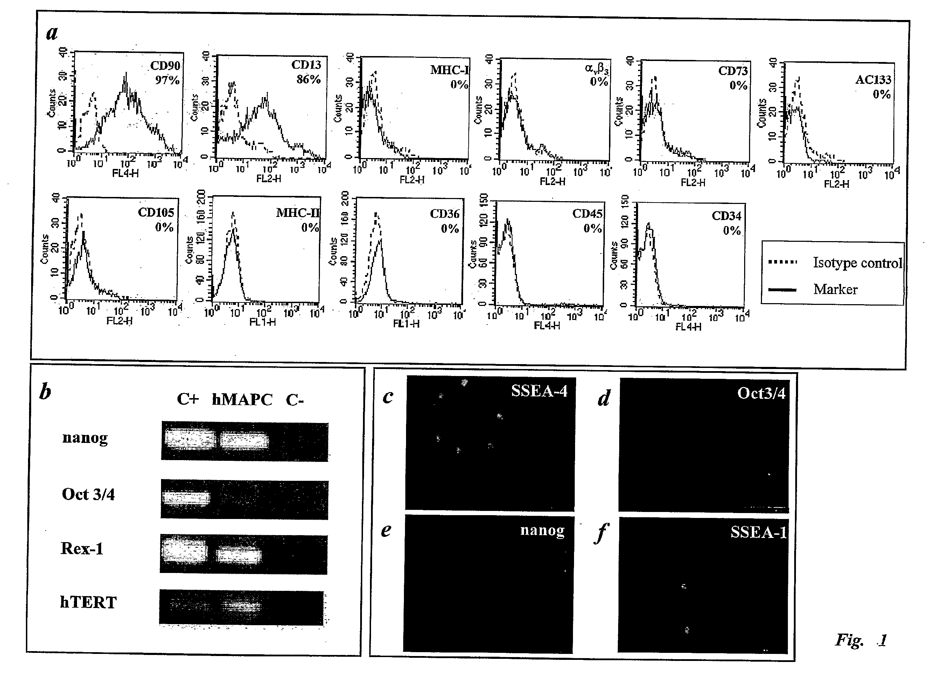

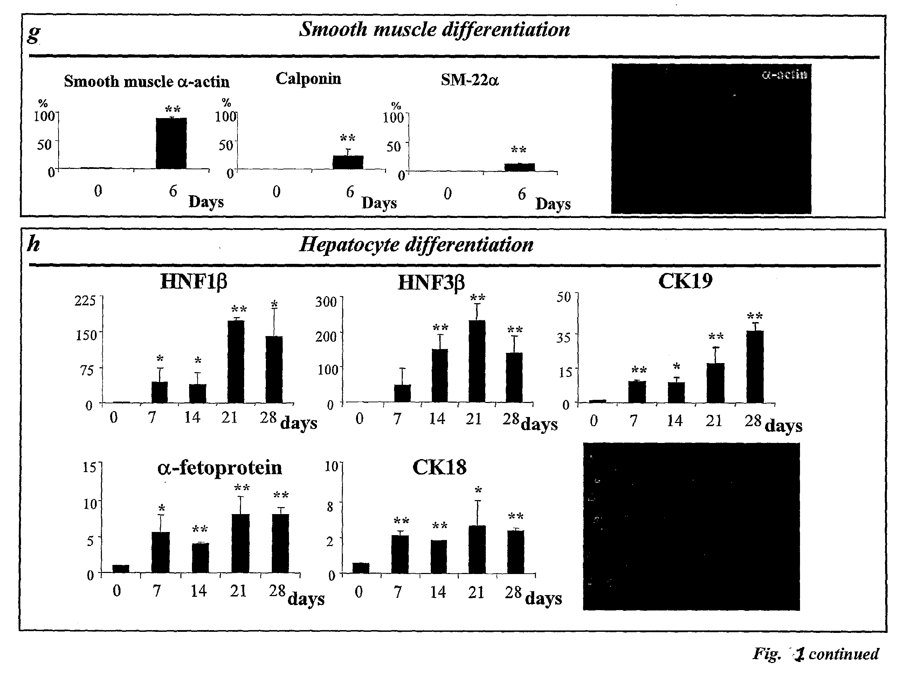

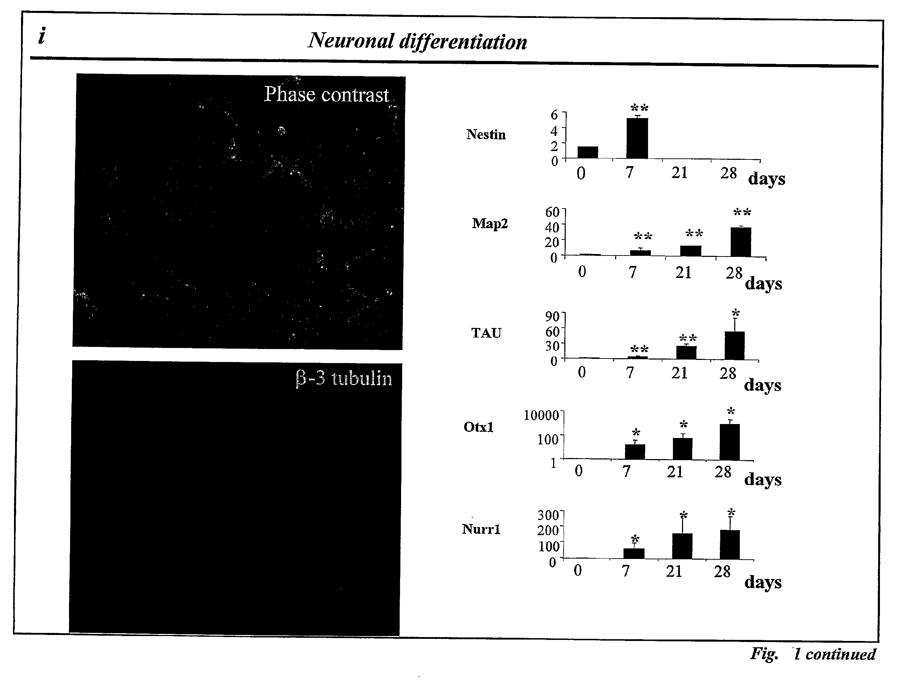

Figure 1. Characterization of hMAPCs: a, FACS phenotype of hMAPCs at 50 population doublings (PDs). Plots show isotype control IgG-staining profile (black line) versus specific antibody staining profile (red line). A representative phenotype of more than 5 experiments is shown; b, RT-PCR blot showing the expression profile of pluripotency markers in hMAPCs at 50 PDs. C+: total RNA (BD), C-: H₂O. *c*-*f*, Immunofluorescent staining for pluripotency markers on hMAPCs at 50 PDs. hMAPCs show expression of SSEA-4 (*c*), OCT3/4 (*d*) and nanog (*e*), but not SSEA-1 (f). A representative experiment of more than 5 experiments is shown. g, Smooth muscle differentiation from hMAPCs: hMAPCs treated with TGF-β1 for 6 days upregulated expression of SMC markers, shown by Q-RT-PCR (presented as % expression in comparison with SMCs derived from umbilical artery) and immunoflourescence (α-actin, green). *h*, Hepatocyte differentiation from hMAPCs: expression of hepatocyte markers was upregulated after 7-28 days in the presence of HGF and FGF-4, shown by Q-RT-PCR (presented as fold increase compared to day 0) and immunoflourescence (albumin, red). *i*, Neuronal differentiation from hMAPCs: expression of neuronal markers was upregulated after 7-28 days in the presence ofbFGF (week 1), Shh, FGF8 (week 2) and BDNF(week 3) (Jiang et al., 2003), as shown by Q-RT-PCR (presented as fold increase compared to day 0) and immunoflourescence (β3-tubulin, green). Q-RT-PCR results are expressed as the mean (± SEM) of three different experiments in triplicates, and immunofluorescence panels are representative of more than 3 experiments. DAPI (blue) in panels *c, f-i* was used for nuclear staining. In panels *g-i:* **P*<0.05; ***P*<0.01 *versus* day 0. Magnification of staining panels: *c*-*f*, 40x, *g,h,* 20x, i, 40x.

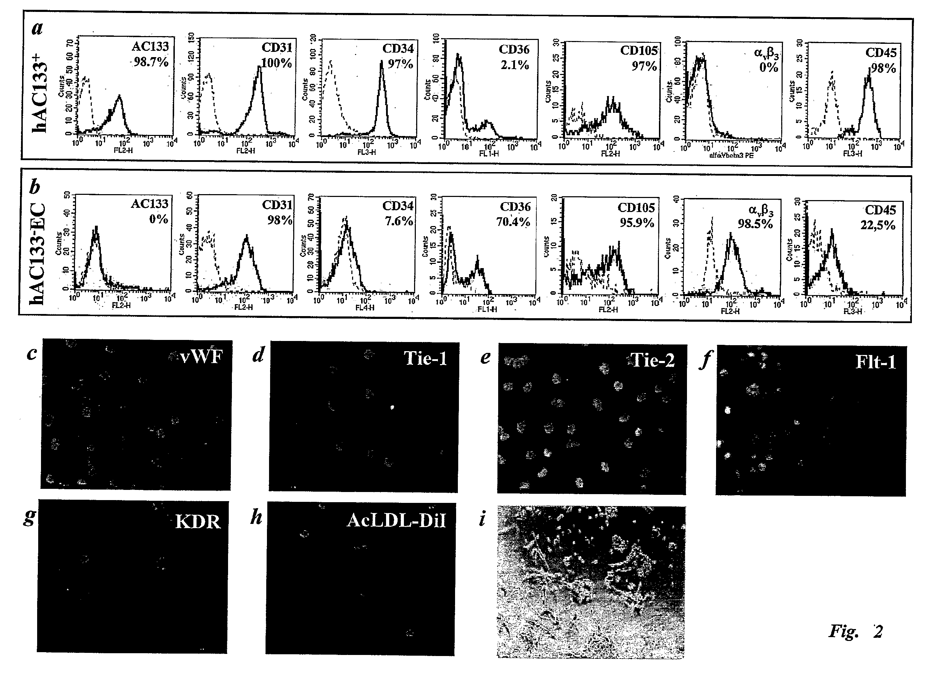

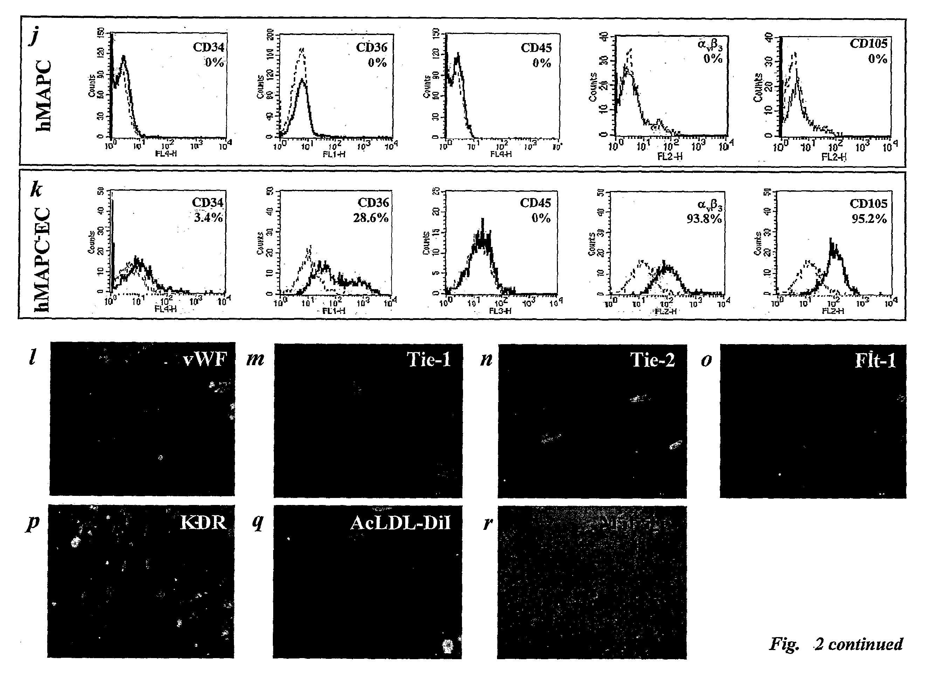

Figure 2. hAC133⁺ cells and hMAPCs differentiate into functional ECs: *a,b,* Flow cytometric analysis of hAC133⁺ cells before (a) and 14 days after the start of the differentiation process (*b*). After 14 days of differentiation, hAC133⁺ cells downregulated hematopoietic markers (like AC133, CD45 and CD34), while EC markers (including CD31, CD36, CD105 and αᵥβ₃) were upregulated. A representative experiment of more than 5 experiments is shown. *c*-*g*, Immunofluorescence of hAC133⁺ derived ECs. After 14 days of differentiation, hAC133⁺ cell-derived ECs stained positive for EC markers, including vWF (*c*), Tie-1 (*d*), Tie-2 (*e*), Flt-1 (*f*) and KDR (*g*). *h,i,* Functionality of hAC133⁺ derived ECs. hAC133⁺ cell-derived ECs were functional as shown by their ability to take up AcLDL (*h*) and to form vascular tubes in matrigel (*i*). A representative experiment of more than 5 experiments is shown. *j,k,* Flow cytometric analysis of hMAPCs before (*j*) and 21 days after the start of the differentiation process (k). After 21 days of differentiation, hMAPCs upregulated several EC markers (including CD34, CD36, CD 105 and αᵥβ₃). *l*-*p*, Immunofluorescence of hMAPC derived ECs. After 14 days of differentiation, hMAPC-derived ECs expressed several EC markers including vWF (*l*), Tie-1 (*m*), Tie-2 (*n*), Flt-1 (*o*) and KDR (*p*). A representative experiment of more than 5 experiments is shown. *q,r,* Functionality of hMAPC-derived ECs. hMAPC-derived ECs were functional as shown by their ability to take up AcLDL (*q*) and to form vascular tubes in matrigel (*r*). Note the difference in morphology between ECs and vascular tubes derived from hAC133⁺ cells or hMAPCs. A representative experiment of more than 5 experiments is shown. FACS plots in panels *a,b,j,* and *k* show isotype control IgG-staining profile (black line) versus specific antibody staining profile (red line). Percentages of positive cells are shown. In panels *c*-*i* and *l-r*, DAPI was used for nuclear staining. Magnifications: *c-h* and *l-q* 40x, i and r 10x.

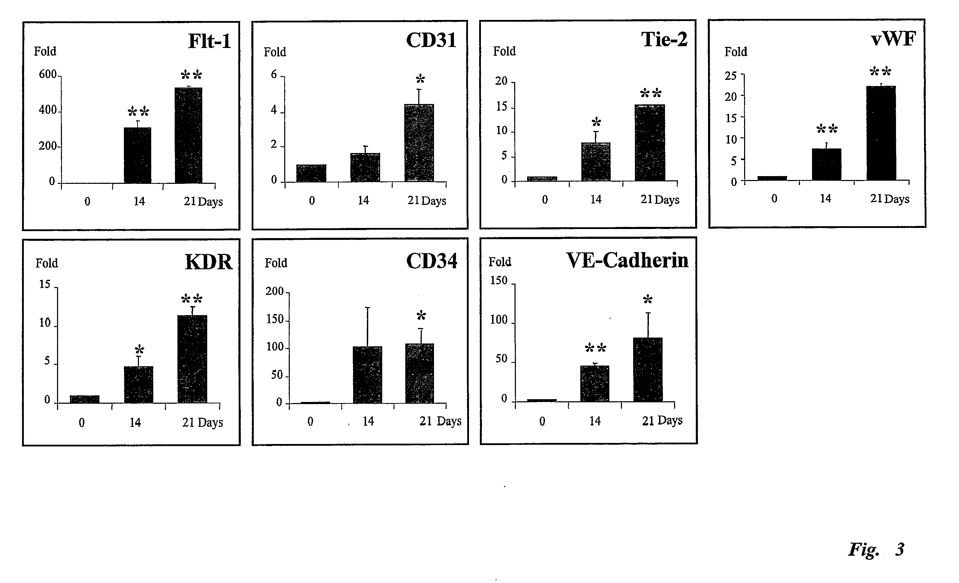

Figure 3. VEGF₁₆₅ induces high expression levels of EC markers in hMAPCs: Q-RT-PCR analysis of EC markers before and 14 or 21 days after differentiation of hMAPCs. Note that the increase in mRNA expression varied between the different genes from 5-fold (CD31) to over 600-fold increase (Flt-1) versus undifferentiated state. mRNA levels in all panels are expressed as fold increase compared with day 0 and were normalized using GAPDH as housekeeping gene. The mean and SEM of three different experiments is shown. **P*<0.05; ***P*<0.01 *versus* day 0.

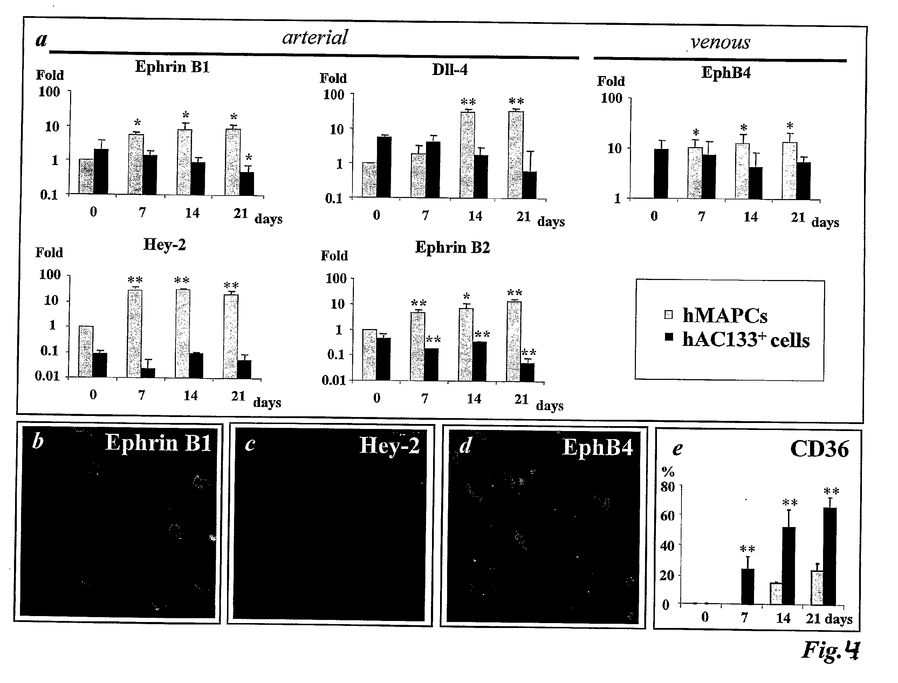

Figure 4. VEGF₁₆₅ induces arterial specification of hMAPCs but not hAC133⁺ cells: *a*, Q-RT-PCR for arterial (EphrinB1, Dll-4, Hey-2, EphrinB2) and venous markers (EphB4) on hAC133⁺ cell-derived ECs (black bars) or hMAPC-derived ECs (grey bars) at different time points (0, 7, 14 and 21 days) after the start of the differentiation process. While hMAPCs upregulated arterial and venous markers during the differentiation process, hAC133⁺ cell-derived ECs showed reduced arterial marker expression. Expression levels are presented as fold increase (in logarithmic scale) in comparison to baseline levels and were normalized by using GAPDH as housekeeping gene. mRNA levels in undifferentiated hMAPC were considered as 1. Expression between baseline levels and day 7, 14 and 21 for each cell population were compared (**P*<0.05; ***P*<0.01). *b*-*d*, Immunofluorescent staining of hMAPC-derived ECs. After 14 days, hMAPCs were positive for arterial markers EphrinB1 (*b*), Hey-2 (*c*), and venous marker EphB4 (*d*) (see text for percentage of positive cells). A representative example from three different donors is shown. *e*, Comparative expression, based on FACS analysis, of the microvascular specific marker CD36 in hMAPC (grey bars) and hAC133⁺ cell-derived ECs (black bars) (***P*<0.01 hAC133-ECs *versus* hMAPC-ECs). CD36 cells are expressed as % of total number of cells. The mean (±SEM) of three (*a*) or 5 (*e*) different experiments in triplicates is shown. Magnification: 40x in panels *b-d.*

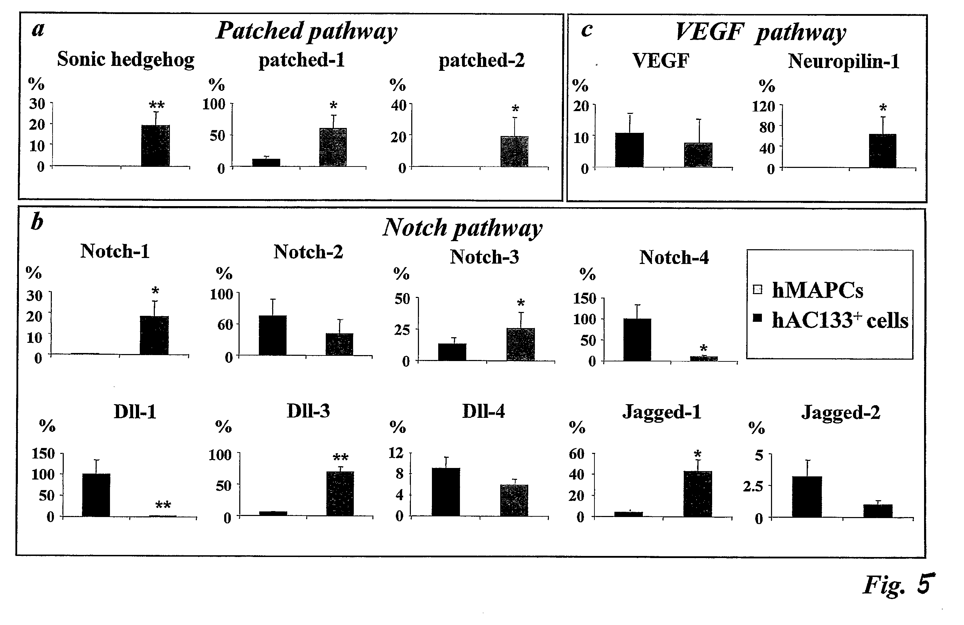

Figure 5. Notch and patched pathway members are differentially expressed in hMAPCs and hAC133⁺ cells: a-c, Q-RT-PCR analysis of members of patched pathway (Shh, Patched-1 and Patched-2) (a), Notch pathway (notch-1, -2, -3 and -4 and ligands Dll-1, -3, -4, and Jagged-1 and -2 (b) and VEGF pathway (VEGF₁₆₅ and neuropilin-1) (c), known to be involved in AV specification. Note differences in expression (see text) between hMAPCs compared to hAC133⁺ cells. mRNA levels in all panels are expressed in % versus a positive control (total RNA, BD) and were normalized by using GAPDH as housekeeping gene. The mean (±SEM) of three different experiments in triplicates is shown. *P<0.05; **P<0.01 versus hAC133⁺ cells.

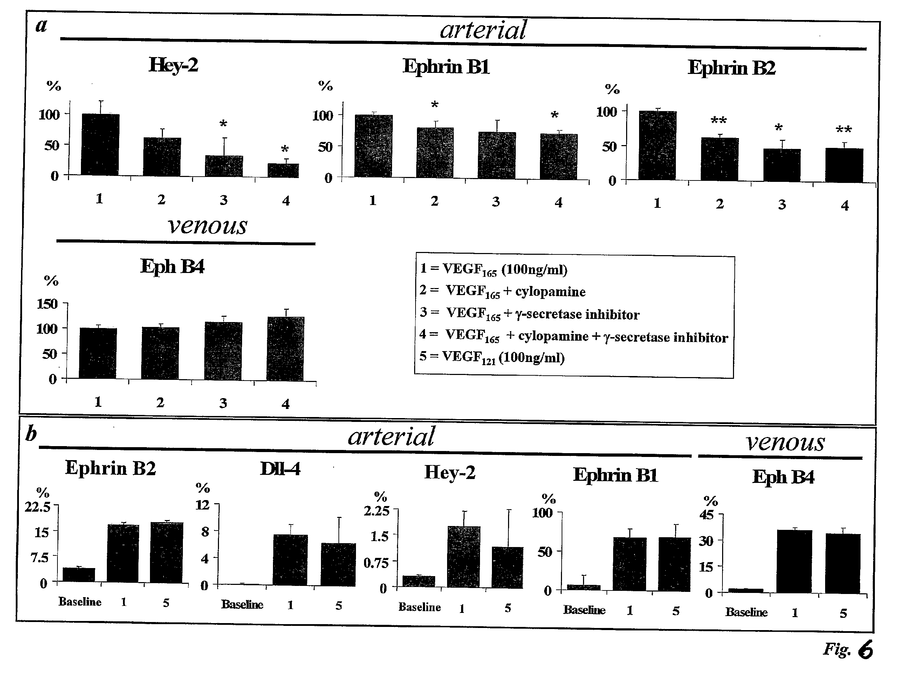

Figure 6. Notch and patched pathway blocking (*e*.*g*., negative modulation) attenuates arterial EC differentiation in hMAPCs: *a*,*b*, Q-RT-PCR analysis for arterial (EphrinB 1, Hey-2, Dll-4, EphrinB2) and venous markers (EphB4) on hMAPC-derived ECs (grey bars) after 14 days of differentiation using different treatments (as indicated on the X-axis and in the legend box). Shh blocking, Notch blocking or a combination of both, significantly reduced expression of arterial EC markers paralleled by an increase in venous marker expression (*a*). Equal concentrations of VEGF₁₂₁ and VEGF₁₆₅ induced arterial (and venous) marker expression to the same extent (*b*). mRNA levels in panels are expressed in % *versus* a expression levels with VEGF₁₆₅ alone *(a)* or as mean % of a positive control (as indicated on the Y axis: Human Umbilical Arteries Endothelial Cells -HUAECs- for arterial markers and Human Umbilical Vein Endothelial Cells - HUVECs- for venous markers) (*b*) and were normalized using GAPDH as housekeeping gene. The mean (± SEM) of three different experiments in triplicates is shown. **P*<0.05; ***P*<0.01 *versus* treatment '1').

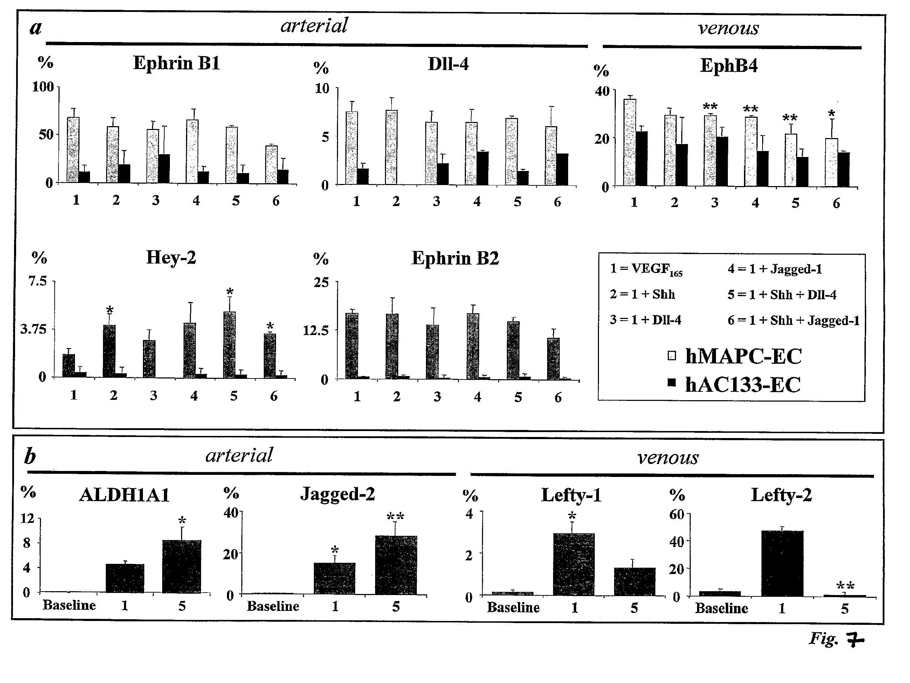

Figure 7. Simultaneous Notch and patched activation boosts arterial EC fate in hMAPCs, but not in hAC133⁺ cells: *a*, Q-RT-PCR analysis of arterial (EphrinB1, Dll-4, Hey-2, EphrinB2) and venous (EphB4) markers in ECs derived from hAC133⁺ cells (black bars) or hMAPCs (grey bars) after 14 days of differentiation using different cytokine cocktails (as indicated on the X-axis and in the legend box). Note the increased expression of arterial marker Hey-2 and the downregulation of venous marker EphB4 with a combination of VEGF₁₆₅, Shh and Dll-4; **P*<0.05; ***P*<0.01 *versus* VEGF₁₆₅ alone. ***b,*** Q-RT-PCR analysis of additional arterial (ALDH1A1, Jagged-2) and venous markers (Lefty-1, Lefty-2) in hMAPC-derived ECs cultured in VEGF₁₆₅ alone or combined with Shh and Dll-4. Note the upregulation of arterial markers and simultaneous downregulation of venous markers in the combination cocktail as compared to VEGF₁₆₅ alone. mRNA levels in all panels are expressed as mean % of a positive control (as indicated on the Y axis: Human Umbilical Arteries Endothelial Cells -HUAECs- for arterial markers and Human Umbilical Vein Endothelial Cells -HUVECs- for venous markers) and were normalized using GAPDH as housekeeping gene. The mean (± SEM) of three different experiments is shown.

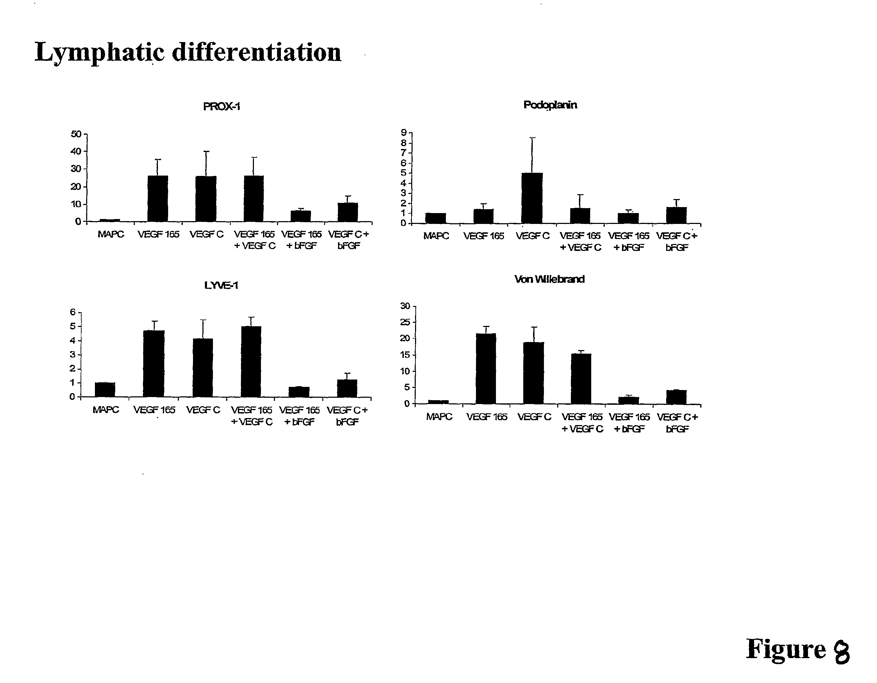

Figure 8. Quantification of lymphatic endothelium specific markers (Prox-1, Lyve-1 and Podoplanin) by real time PCR using differentiation media consisting of: VEGF₁₆₅, VEGF-C, VEGF₁₆₅ + VEGF-C, VEGF₁₆₅ + bFGF and VEGF-C + bFGF.

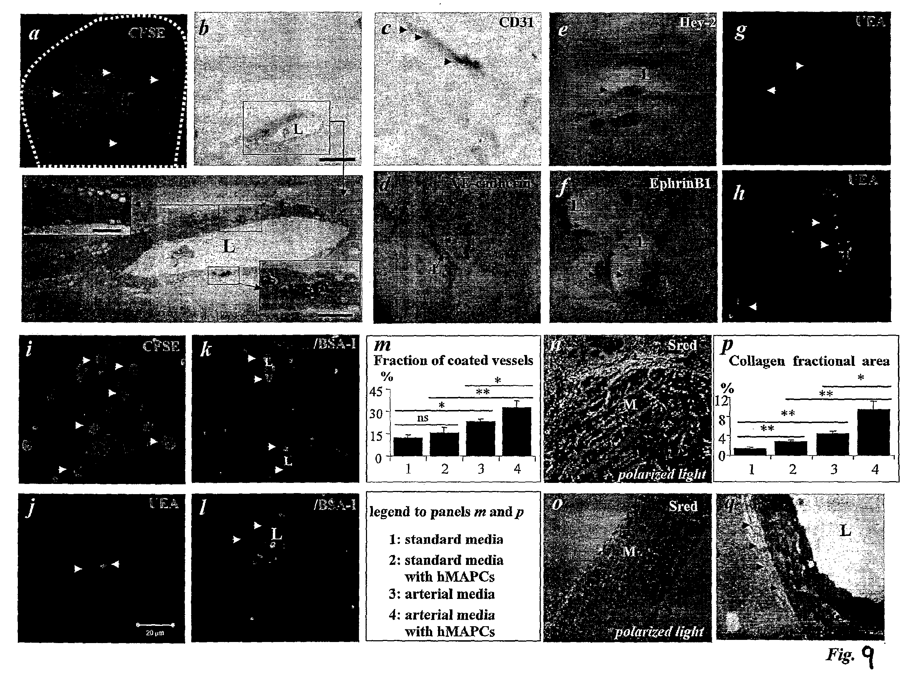

Figure 9. Shh and Dll-4 induce arterial hMAPC-EC differentiation and arterial-like vessel growth *in vivo*: *a,* Live *in vivo* imaging of a matrigel plug containing VEGF₁₆₅ and hMAPCs labeled with CFSE, 10 days after subcutaneous implantation. Note the localized CFSE labeled area (outlined by a dashed white line) located in the matrigel in the vicinity of a large vascular tree (arrowheads) from the overlying host skin. *b-q,* Histological analysis on cross-sections through matrigel plugs containing hMAPCs and VEFG₁₆₅ *(b-d, l,* and *o*) or hMAPCs and VEGF₁₆₅+Shh+Dll-4 ('arterial cytokine mix'; *e-k, n* and *q*). *b,* Electron microscopy showing a capillary comprised of a Resovist labeled hMAPC derived EC in matrigel plugs. Semithin section (magnification 100x); ultrathin section and a detail of iron particles (inset). *c*-*f* Immunohistochemical staining of 3 µm paraffin cross-sections through matrigel plugs for human-specific CD31 (*c*) and human-specific VE-Cadherin (*d*) (both indicating their EC identity), and human-specific Hey-2 (*e*) and EphrinB1 (*f*), both indicating their arterial EC identity. *g*,*h*, Double confocal immunofluorescence staining of 40 µm cryopreserved cross-sections though matrigel plugs with human endothelial specific lectin UEA (green) and Hey2 (red) (*g*), UEA (green) and EphrinB2 (red) (*h*) Topro (blue) was used for nuclear staining. *i*, High resolution live *in vivo* imaging of a matrigel plug containing VEGF₁₆₅ and hMAPCs labeled with CFSE, 10 days after subcutaneous implantation and 30 min after IV injection of UEA lectin. Note co-localization (yellow; indicated by arrowheads) of CFSE labeled cells (green) and UEA lectin (red) area, indicating that the vessels containing CFSE labeled cells were connected to the host vascular system. *j*, Double confocal immunofluorescence staining of 40 µm cryopreserved cross-sections through matrigel plugs with human endothelial specific lectin UEA (green) and α-actin (red) (Magnification 40x), showing hMAPC-ECs (arrowheads) coated by α-actin⁺ SMCs. Topro (blue) was used for nuclear staining. *k*,*l*, Double immunofluorescent staining of 3 µm paraffin cross-sections though matrigel plugs stained with SMC α-actin (red) and BS-I lectin (green), showing more SMC coated (indicated by arrowheads) vessels when the arterial media was used (*k*) in comparison to standard media (/). *m*, Diagram comparing the fraction of SMC-coated vessels (expressed as % (± SEM) *versus* the total number of vessels) for the conditions outlined in the legend box; **P*<0.05; ***P*<0.01. *n,o,* Sirius Red staining (visualized by polarized light microscopy indicating abundant and thick (orange-red birefringent) fibrillar collagen around vessels in matrigels containing hMAPCs combined with the arterial mix (*n*) as compared to the less abundant and thinner collagen in matrigels containing hMAPCs combined with VEGF₁₆₅ alone (*o*), dashed lines indicate the edge of the matrigel. *p*, Diagram comparing the collagen fractional area (expressed as % (± SEM) *versus* the total area) for the conditions outlined in the legend box; **P*<0.05; ***P*<0.01. *q*, Ultrastructural analysis of a matrigel plug injected subcutaneously with hMAPCs and arterial cytokines showing that collagen/elastin is associated with peri-endothelial cells in the vessel wall of an artery-like tube. Magnifications 63x in panels *e,f*; 40x in panels *c,d, j-l*; 10x in panels *n,o.* 'L' in panels *b,e,f,k,l,* and *q* indicates the vessel lumen. Scale bar 10 µm (*b*, semithin); 2.5 µm (*b*, ultrathin); 1 µm (*b*, upper inset); 0.5 µm (*b*, lower inset), 0.2 µm in *q*.

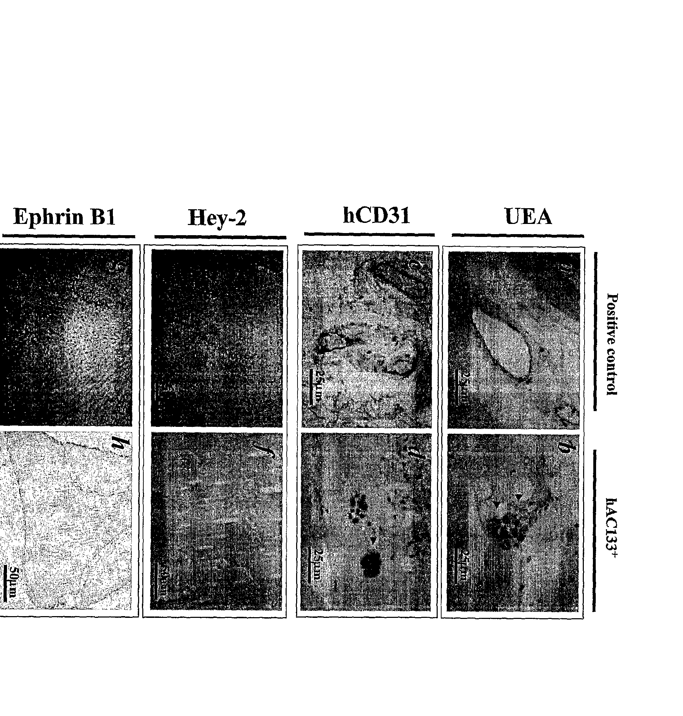

Figure 10. hAC133⁺ cells do not form arterial ECs *in vivo*: *a-h,* Histological analysis on cross sections through matrigel plugs containing hAC133⁺ cells, 14 days after implantation. Immunohistochemical staining of 3 µm paraffin cross-sections through matrigel plugs. hAC133⁺ derived cells (arrowheads) stained positive for EC markers (human-specific UEA lectin (*b*) and CD31 (*d*); both indicating their EC identity), but negative for arterial EC markers (human-specific Hey-2 (*f*) and EphrinB1 (*h*), both indicating lack of arterial EC identity). Human muscle (*a*,*c*) and umbilical chord biopsies (*e*,*g*) were used as positive control. Scale bars: 25 µm in *a-c*, 50 µm in *e-h.*

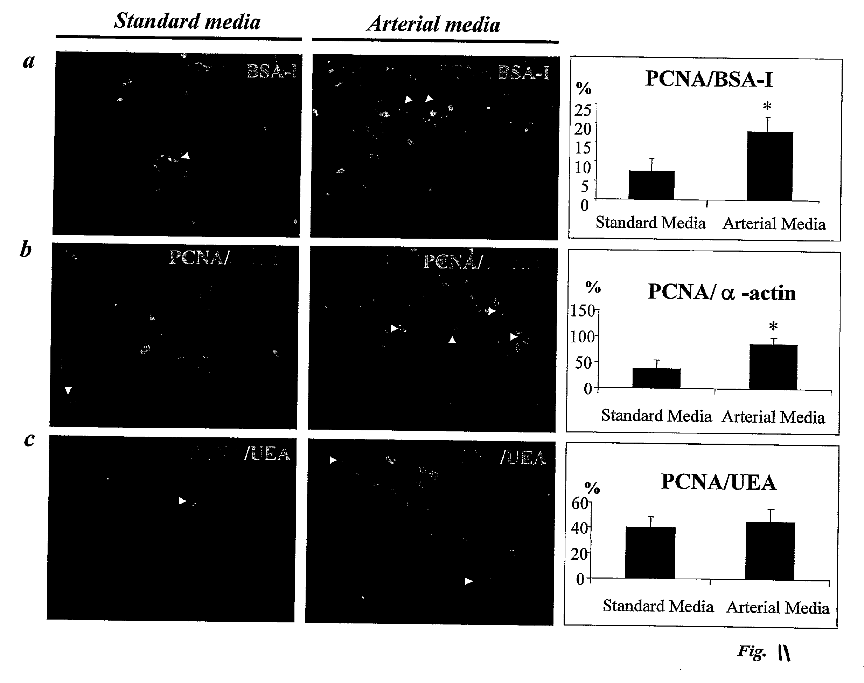

Figure 11. Shh and Dll-4 boost host cell proliferation: *a*-*c*, 3 µm paraffin cross-sections through matrigel plugs injected with hMAPCs combined with standard media (left panels) or arterial media (middle panels), double-stained with proliferation marker PCNA (red in (*a,c*) and green in (*b*)) and BS-I lectin (*a*, green, corresponding to host ECs), α-actin (*b*, red, corresponding to host SMCs) and UEA lectin (*c*, green, corresponding to hMAPC-ECs). Proliferating cells are indicated by arrowheads. Panels on the right show a numeric representation, where the number of proliferating cells is expressed as a % of the total number of each cell type of interest. Note that significantly more host derived ECs and SMCs were proliferating in the matrigel plugs injected with arterial media compared to standard media (**P*<0.05). No difference in hMAPC-EC proliferation was observed between arterial and standard media. DAPI (blue) was used in all panels for nuclear counterstaining. Magnifications: 20x in all panels.

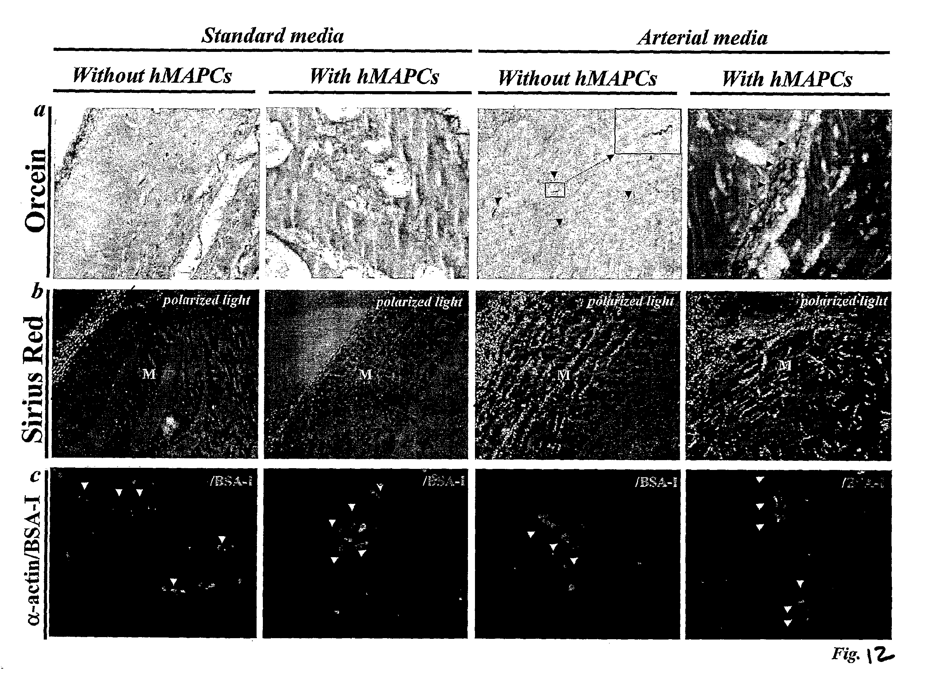

Figure 12. Shh and Dll-4 stimulate formation of vessels with artery-like characteristics: *a*-*c*, 3 µm paraffin cross-sections through matrigel plugs injected with arterial or standard media, combined or not with hMAPCs, stained for orcein (*a*, corresponding to elastin), Sirius red (*b*, corresponding to fibrillar collagen), and double-stained with α-actin (red) and BS-I lectin (green) (*c*, corresponding to SMC coated vessels). Orcein staining shows abundant elastin (visualized as dark purple fibers indicated by arrowheads and inbox) only around vessels in matrigels containing the arterial mix, which was more elaborate in the presence of hMAPCs. *b*, Sirius red staining shows significantly more collagen (visualized by polarized light microscopy) in conditions where the arterial mix was used, as compared to the standard mix. Moreover, there was more abundant and thick fibrillar collagen (appearing as orange-red birefringent) around vessels in matrigels containing hMAPCs and the arterial mix in comparison with the other conditions. For quantification of collagen content, see Fig. 9p. Dashed lines indicate the edge of the matrigel. *c*, Double-staining for α-actin and BS-I lectin revealed a significantly higher fraction of SMC coated vessels (arrowheads) in matrigel plugs containing arterial media as compared to standard media. Moreover, when the arterial mix was combined with hMAPCs, SMC coating was even more pronounced and vessels had larger diameters. For quantification of collagen content, see Fig. 9m. DAPI was used in panel (*c*) for nuclear staining. Magnifications: 40x in (*a*), 10x in (*b*), and 40x in (*c*).

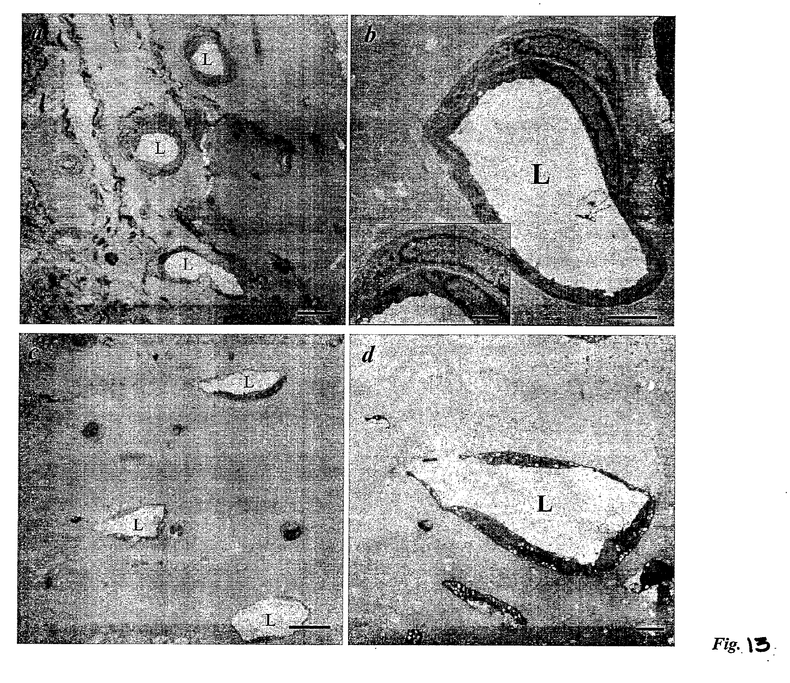

Figure 13. Ultrastructural comparison between the vessel make-up in matrigel plugs injected with hMAPCs in arterial or standard media: Ultrastructural analysis of matrigel plugs injected subcutaneously with hMAPCs combined with arterial (*a,b*) or standard media (*c*,*d*). Semithin sections of a matrigel plug (*a,c*), ultrathin section of an artery-like tube (*b*) with a detail showing an SMC around an EC (inset) and a vein-like tube (*d*). 'L' in panels *a*-*d* indicates the vessel lumen. Scale bar 10 µm (*a,c*); 2.5 µm (*b*); 2 µm (*d*); 1 µm (*b*, inset).

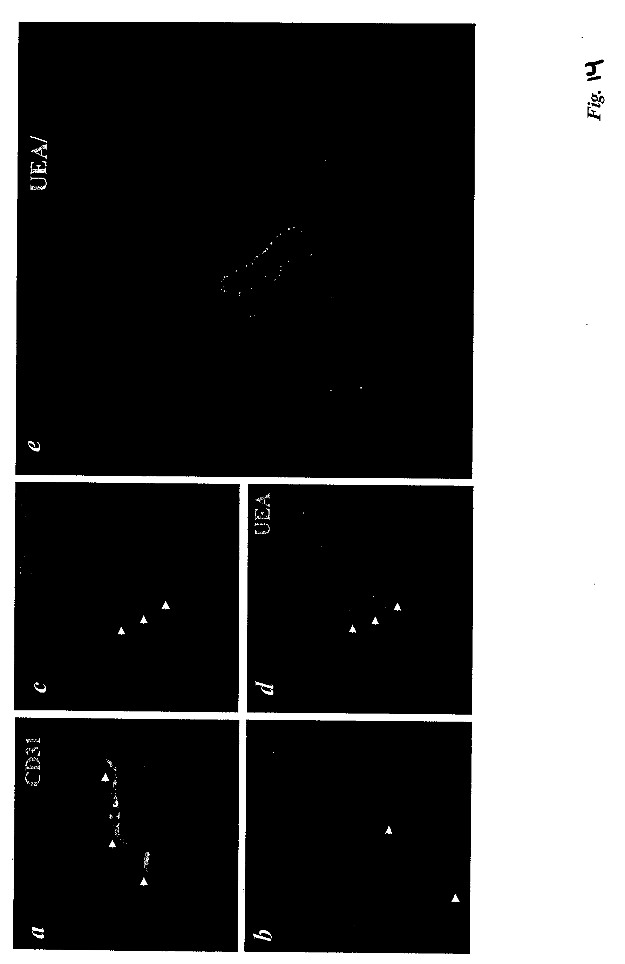

Figure 14. hMAPCs differentiate into arterial ECs in ischemic hind limbs: Confocal immunofluorescent imaging on 30 µm cross-sections through the quadriceps muscle of an ischemic mouse limb, one month after injection of hMAPCs, stained with human CD31 (green) (*a*), UEA lectin (red in *b*, green in *d*) and EphrinB1 (red in *c* and *e*) showing capillaries containing hMAPC-derived (arrowheads) UEA lectin⁺ (*b*) and human CD31⁺ (*a*) ECs and arterioles containing hMAPC-derived EphrinB1⁺ UEA⁺ arterial ECs (*e*). Topro (blue) was used as nuclear counterstain in all panels. Scale bar: 50 µm (*e*) and 20 µm (*a-d*).

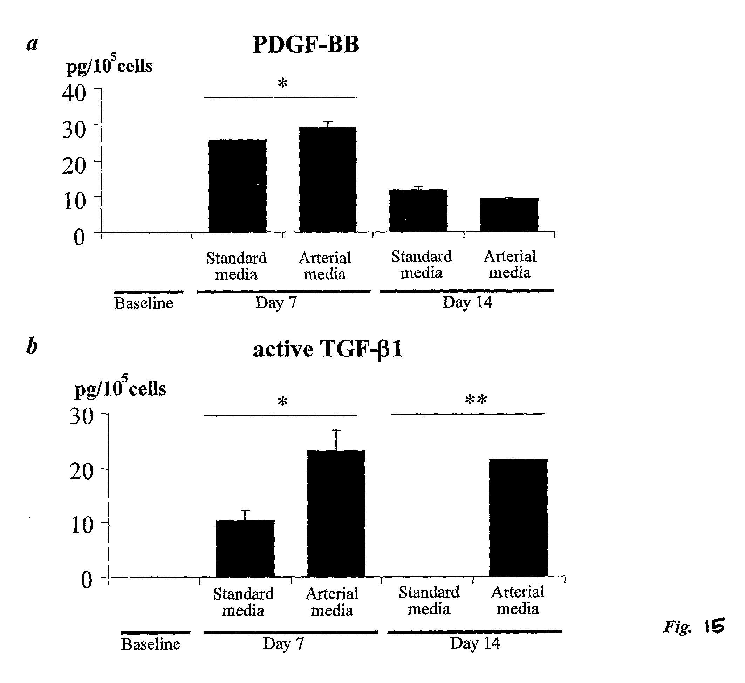

Figure 15. hMAPC-ECs secrete PDGF-BB and active TGF-β1: *a,b,* ELISA for PDGF-BB *(a)* and active TGF-β1 (*b*) on cell supernatants of undifferentiated hMAPCs ('baseline') or differentiated for 7 or 14 days to ECs either in standard media or arterial media. While PDGF-BB production was only slightly and temporarily higher, active TGFβ1 production was significantly higher in arterial media *versus* standard media. **P*<0.05; ***P*<0.01. Data are expressed as pg per 10⁵ cells and represent the mean±SEM of experiments performed in triplicates.

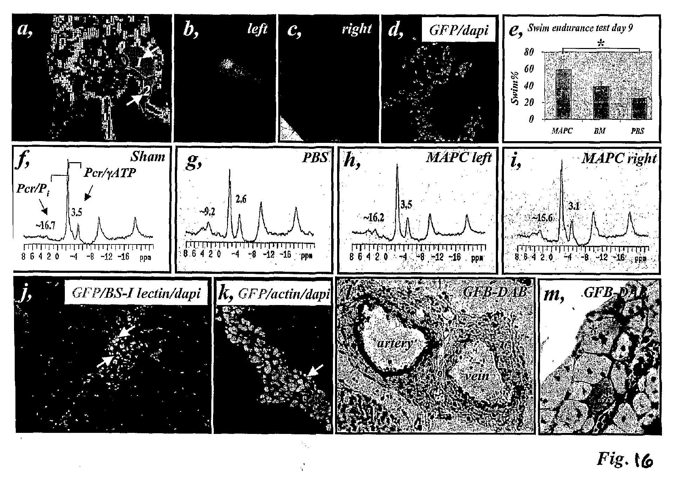

Figure 16. Mouse model of hind limb ischemia: panel (a), mouse model of hind limb ischemia, showing bilateral ligation of the deep femoral artery and transplantation (arrows) of cells into the left adductor muscle (1) and the left gastrocnemius muscle (2); panel (*b*-*c*), Live imaging on the left adductor (*b*) and right adductor (*c*) after exposure of the muscle, showing a GFP-signal derived from transplanted GFP-overexpressing mouse MAPCs located next to the left femoral artery (*b*), but not the right femoral artery (c); panel (*d*), Fluorescent image of a cross-section through the left adductor muscle of a mouse transplanted with GFP-overexpressing MAPCs showing robust engraftment of GFP-positive cells (green) between the muscle fibers; Dapi (blue) was used as nuclear counterstain; panel (*e*), Swim endurance test results, expressed as a percentage of swim time 9 days after bilateral femoral artery ligation versus swim time before ligation; N=8-10; **P* < 0.05; panel (*f-i*), Magnetic Resonance Imaging (MRI) spectra, showing the energetic status of the gastrocnemic muscle 9 days after ligation, in a sham operated mouse ((*f*); left leg)), a vehicle treated mouse ((*g*); left leg), a MAPC treated mouse ((*h*); left leg; (*i*): right leg). Energetic status is expressed as two different ratios: Pcr/Pi (phospho-creatine kinase/inorganic phosphate) and Pcr/gammaATP; a bad energetic status is manifested when values of both ratios are low, such as in the vehicle treated animal in panel (*g*); panel (*j*): Immunofluorescent staining on a cross-section of the left adductor muscle of a MAPC transplanted mouse showing co-localization (yellow; arrows) of endothelial marker BS-I lectin (red) and GFP (green); panel (*k*), Immunofluorescent confocal image of a cross-section of the left adductor muscle of a MAPC transplanted mouse showing co-localization (yellow; arrows) of smooth muscle cell marker alpha-actin (red) and GFP (green); panel (*l*), DAB staining for GFP on a cross-section from the left adductor muscle of a MAPC transplanted mouse, showing positive signals in the endothelial and smooth muscle cell layer of the artery, but not the vein; panel (*m*), DAB staining for GFP on a cross-section from the left gastrocnemic muscle of a mouse transplanted with MAPCs, showing positive signal (asterisks) in some of the muscle cells. Note the centrally localized nucleus as a hallmark for regenerating muscle fibers.

### Detailed Description of the Invention

### Definitions

As used herein, the terms below are defined by the following meanings:
"MAPC" is an acronym for "multipotent adult progenitor cell." It is used herein to refer to a non-embryonic stem (non-ES), non-germ, non-embryonic germ (non-EG) cell that can give rise to cell types of more than one embryonic lineage. It can form cell lineages of at least two germ layers (i.e., endoderm, mesoderm and ectoderm) upon differentiation. Like embryonic stem cells, MAPCs from humans were reported to express telomerase, Oct-3/4 (i.e., Oct-3A), rex-1, rox-1 and sox-2 (Jiang, Y. et al. 2002). Telomerase or Oct-3/4 have been recognized as genes that are primary products for the undifferentiated state. Telomerase is necessary for self renewal without replicative senescence. MAPCs derived from human, mouse, rat or other mammals appear to be the only normal, non-malignant, somatic cell (*i.e.*, non-germ cell) known to date to express telomerase even in late passage cells. The telomeres are not sequentially reduced in length in MAPCs. MAPCs are karyotypically normal. MAPCs may express SSEA-4 and nanog. The term "adult," with respect to MAPC is non-restrictive. It refers to a non-embryonic somatic cell.

MAPCs injected into a mammal can migrate to and assimilate within multiple organs. This shows that MAPCs are self-renewing. As such, they have utility in the repopulation of organs, either in a self-renewing state or in a differentiated state compatible with the organ of interest. They have the capacity to replace cell types that have been damaged (due to disease or injury), died, or otherwise have an abnormal function because of genetic or acquired disease. Or, as discussed below, they may contribute to preservation of healthy cells or production of new cells in a tissue.

"Multipotent," with respect to MAPC, refers to the ability to give rise to cell types of more than one embryonic lineage. MAPC can form cell lineages of all three primitive germ layers (*i.e.*, endoderm, mesoderm and ectoderm).

"Expansion" refers to the propagation of cells without differentiation.

"Progenitor cells" are cells produced during differentiation of a stem cell that have some, but not all, of the characteristics of their terminally-differentiated progeny.

Defined progenitor cells, such as "vascular endothelial progenitor cells," are committed to a lineage, but not to a specific or terminally-differentiated cell type. The phrase "vascular endothelial cells" encompasses not only terminally-differentiated vascular cells types, but also cells that are committed to a vascular lineage (*e.g.*, venous and/or arterial lineage), but are not terminally-differentiated. The term "progenitor" as used in the acronym "MAPC" does not limit these cells to a particular lineage.

"Self-renewal" refers to the ability to produce replicate daughter cells having differentiation potential that is identical to those from which they arose. A similar term used in this context is "proliferation."

"Engraft" or "engraftment" refers to the process of cellular contact and incorporation into an existing tissue or site of interest. In one embodiment, greater than about 5%, greater than about 10%, greater than about 15%, greater than about 20%, greater than about 25%, greater than about 30%, greater than about 35%, greater than about 40%, greater than about 45%, greater than about 50%, greater than about 55%, greater than about 60%, greater than about 65%, greater than about 70%, greater than about 75%, greater than about 80%, greater than about 85%, greater than about 90%, greater than about 95% or about 100% of administered MAPCs or progeny derived therefrom engraft in tissues (e.g., vasculature) of the subject.

Persistence refers to the ability of cells to resist rejection and remain or increase in number over time (e.g., days, weeks, months, years) *in vivo.* Thus, by persisting, the MAPC or progeny can populate the vasculature or other tissues or remain *in vivo,* such as in barrier devices or other encapsulated forms.

"Immunologic tolerance" refers to the survival (in amount and/or length of time) of foreign (e.g., allogeneic or xenogeneic) tissues, organs or cells in recipient subjects. This survival is often a result of the inhibition of a graft recipient's ability to mount an immune response that would otherwise occur in response to the introduction of foreign cells. Immune tolerance can encompass durable immunosuppression of days, weeks, months or years. Included in the definition of immunologic tolerance is NK-mediated immunologic tolerance. This term also encompasses instances where the graft is tolerant of the host.

The term "isolated" refers to a cell or cells which are not associated with one or more cells or one or more cellular components that are associated with the cell or cells *in vivo* or in primary cultures. An "enriched population" means a relative increase in numbers of the cell of interest, such as MAPCs, relative to one or more other cell types, such as non-MAPC cell types, *in vivo* or in primary culture.

"Cytokines" refer to cellular factors that induce or enhance cellular movement, such as homing of MAPCs or other stem cells, progenitor cells or differentiated cells. Cytokines may also stimulate such cells to divide.

"Differentiation factors" refer to cellular factors, preferably growth factors or angiogenic factors, that induce lineage commitment.

A "subject" is a vertebrate, preferably a mammal, more preferably a human. Mammals include, but are not limited to, humans, farm animals, sport animals and companion animals. Included in the term "animal" is dog, cat, fish, gerbil, guinea pig, hamster, horse, rabbit, swine, mouse, hamster, monkey (*e.g.*, ape, gorilla, chimpanzee, orangutan), rat, sheep, goat, cow and bird.

Subjects that can benefit from the vascular endothelial cells and methods of the invention can include, but are not limited to, those suffering from a loss and/or function of vascularization as a result of physical or disease related damage. Disease states characterized by a loss of vascularization and/or function, and that benefit from methods of the present invention include vascular conditions, such as ischemia (including ischemia-reperfusion injury and critical limb ischemia), congestive heart failure, peripheral vasculature disorder, myocardial infarction, coronary vascular disease, hypertension, stroke, aneurysm, thrombosis, arrhythmia, tachycardia, or surgical or physical (*e.g.*, wounding) trauma.

As used herein, "treat," "treating" or "treatment" includes treating, reversing, preventing, ameliorating, or inhibiting an injury or disease-related condition or a symptom of an injury or disease-related condition.

An "effective amount" generally means an amount which provides the desired effect. For example, an effective dose is an amount sufficient to effect a beneficial or desired result, including a clinical result. The dose could be administered in one or more administrations and can include any preselected amount of cells. The precise determination of what would be considered an effective dose may be based on factors individual to each subject, including size, age, injury or disease being treated and amount of time since the injury occurred or the disease began. One skilled in the art, particularly a physician, would be able to determine the number of cells that would constitute an effective dose. Doses can vary depending on the mode of administration, e.g., local or systemic; free or encapsulated. The effect can be engraftment or other clinical endpoints, such as reversal or treatment of ischemia. Other effects can include providing vascular endothelial cells, recruiting endogenous cells, effecting angiogenesis or vasculogenesis, and/or providing vasculature.

"Co-administer" can include simultaneous and/or sequential administration of two or more agents (including cells).

Administered MAPCs or progeny may contribute to the generation of vascular tissue by differentiating into various cells in vivo. Alternatively, or in addition, administered cells may contribute to the generation of vascular tissue by secreting cellular factors that aid in homing and recruitment of endogenous MAPCs or other stem cells, or other more differentiated cells. Alternatively, or in addition, MAPCs or progeny may secrete factors that act on endogenous stem or progenitor cells causing them to differentiate. Further, MAPCs or progeny may secrete factors that act on stem, progenitor or differentiated cells, causing them to divide. Thus, MAPCs or progeny may provide benefit through trophic influences. Examples of trophic influences include, but are not limited to, improving cell survival and homing of cells to desired sites. Additionally, MAPCs or progeny may provide for angiogenesis, vasculogenesis or reduce or prevent apoptosis. Therapeutic benefit may be achieved by a combination of the above pathways.

The terms "comprises," "comprising," and the like can have the meaning ascribed to them in U.S. Patent Law and can mean "includes," "including" and the like. As used herein, "including" or "includes" or the like means including, without limitation.

### MAPCs

MAPCs are non-embryonic (non-ES), non-germ and non-embryonic germ (non-EG) cells that can differentiate into ectodermal, endodermal and mesodermal cells types. MAPCs can be positive for telomerase. They can also be positive for Oct-3A (Oct-3/4). MAPCs can differentiate *in vivo* where they can form vascular cells, such as arterial or venous cells. Alternatively, differentiated progeny of MAPCs, formed *ex vivo*, can be used to provide vascular cells. MAPCs or their differentiated progeny can be administered to a subj ect.

Human MAPCs are described in U.S. Patent Application Serial Nos. 10/048,757 (PCT/US00/21387 (published as WO 01/11011)) and 10/467,963 (PCT/US02/04652 (published as WO 02/064748)), the contents of which are incorporated herein by reference for their description of MAPCs. MAPCs have been identified in other mammals. Murine MAPCs, for example, are also described in U.S. Patent Application Serial Nos. 10/048,757 and 10/467,963, the contents of which are incorporated herein by reference for their description of murine MAPCs. Rat MAPCs are also described in U.S. Patent Application Serial No. 10/467,963, the contents of which are incorporated herein by reference for their description of rat MAPCs. Swine MAPCs are described in Patent Application No. PCT/US2005/038979, the contents of which are incorporated herein by reference for their description of swine MAPCs.

MAPCs have the ability to regenerate all primitive germ layers (endodermal, mesodermal and ectodermal) *in vitro* and *in vivo.* The biological potency of MAPCs has been proven in various animal models (Reyes, M. and C.M. Verfaillie 2001; Jiang, Y. et al. 2002). Single genetically marked MAPC were injected into mouse blastocysts, blastocysts implanted, and embryos developed to term (Jiang, Y. et al. 2002). Post-natal analysis in chimeric animals showed reconstitution of all tissues and organs, including liver.

MAPCs are capable of extensive culture without loss of differentiation potential and show efficient, long term, engraftment and differentiation along multiple developmental lineages in NOD-SCID mice, without evidence of teratoma formation (Reyes, M. and C.M. Verfaillie 2001). This includes endothelial lineage differentiation Verfaillie, C.M. 2002; Jahagirdar, B.N. et al. 2001).

Adherent cells from bone tissue are enriched in media as described herein, and grown to high population doublings. At early culture points more heterogeneity is detected in the population. Then, many adherent stromal cells undergo replicative senescence around cell doubling 30 and a more homogenous population of cells continues to expand and maintain long telomeres.

### Isolation and Growth

Methods of MAPC isolation for humans and mouse are described in U.S. Patent Application Serial No. 10/048,757 (PCT/US00/21387 (published as WO 01/11011)) and for rat in U.S. Patent Application Serial No. 10/467,963 (PCT/US02/04652 (published as WO 02/064748)), and these methods, along with the characterization of MAPCs disclosed therein, are incorporated herein by reference.

MAPCs were initially isolated from bone marrow, but were subsequently established from other tissues, including brain and muscle (Jiang, Y., et al., 2002). Thus, MAPCs can be isolated from multiple sources, including bone marrow, placenta, umbilical cord and cord blood, muscle, brain, liver, spinal cord, blood or skin. For example, MAPCs can be derived from bone marrow aspirates, which can be obtained by standard means available to those of skill in the art (see, for example, Muschler, G.F., et al., 1997; Batinic, D., et al., 1990). It is therefore now possible for one of skill in the art to obtain bone marrow aspirates, brain or liver biopsies and other organs, and isolate the cells using positive or negative selection techniques available to those of skill in the art, relying upon the genes that are expressed (or not expressed) in these cells (e.g., by functional or morphological assays, such as those disclosed in the above-referenced applications, which have been incorporated herein by reference for teaching such assays).

### MAPCs from Human Bone Marrow as Described in U.S. 10/048,757

Bone marrow mononuclear cells were derived from bone marrow aspirates, which were obtained by standard means available to those of skill in the art (see, for example, Muschler, G.F. et al. 1997; Batinic, D. et al. 1990). Multipotent adult stem cells are present within the bone marrow (and other organs such as liver or brain), but do not express the common leukocyte antigen CD45 or erythroblast specific glycophorin-A (Gly-A). The mixed population of cells was subjected to a Ficoll Hypaque separation. The cells were then subjected to negative selection using anti-CD45 and anti-Gly-A antibodies, depleting the population of CD45⁺ and Gly-A⁺ cells, and the remaining approximately 0.1% of marrow mononuclear cells were then recovered. Cells could also be plated in fibronectin-coated wells and cultured as described below for 2-4 weeks to deplete the cell population of CD45⁺ and Gly-A⁺ cells.

Alternatively, positive selection can be used to isolate cells via a combination of cell-specific markers. Both positive and negative selection techniques are available to those of skill in the art, and numerous monoclonal and polyclonal antibodies suitable for negative selection purposes are also available in the art (see, for example, Leukocyte Typing V, Schlossman, et al., Eds. (1995) Oxford University Press) and are commercially available from a number of sources.

Techniques for mammalian cell separation from a mixture of cell populations have also been described by, for example, Schwartz, et al., in U. S. Patent No. 5,759,793 (magnetic separation), Basch et al. 1983 (immunoaffinity chromatography), and Wysocki and Sato 1978 (fluorescence-activated cell sorting).

Recovered CD45⁻/GlyA⁻ cells were plated onto culture dishes coated with about 5-115 ng/ml (about 7-10 ng/ml can be used) serum fibronectin or other appropriate matrix coating. Cells were maintained in Dulbecco's Minimal Essential Medium (DMEM) or other appropriate cell culture medium, supplemented with about 1-50 ng/ml (about 5-15 ng/ml can be used) platelet-derived growth factor-BB (PDGF-BB), about 1-50 ng/ml (about 5-15 ng/ml can be used) epidermal growth factor (EGF), about 1-50 ng/ml (about 5-15 ng/ml can be used) insulin-like growth factor (IGF), or about 100-10,000 IU (about 1,000 IU can be used) LIF, with about 10⁻¹⁰ to about 10⁻⁸ M dexamethasone or other appropriate steroid, about 2-10 µg/ml linoleic acid, and about 0.05-0.15 µM ascorbic acid. Other appropriate media include, for example, MCDB, MEM, IMDM and RPMI. Cells can either be maintained without serum, in the presence of about 1-2% fetal calf serum, or, for example, in about 1-2% human AB serum or autologous serum.

When re-seeded at about 2x10³ cells/cm² about every 3 days, >40 cell doublings were routinely obtained, and some populations underwent >70 cell doublings. Cell doubling time was about 36-48h for the initial 20-30 cell doublings. Afterwards cell-doubling time was extended to as much as 60-72h.

Telomere length of MAPCs from 5 donors (age about 2 years to about 55 years) cultured at re-seeding densities of about 2x10³ cells/cm² for about 23-26 cell doublings was between about 11-13 KB. This was about 3-5 KB longer than telomere length of blood lymphocytes obtained from the same donors. Telomere length of cells from 2 donors evaluated after about 23 and about 25 cell doublings, respectively, and again after about 35 cells doublings, was unchanged. The karyotype of these MAPCS was normal.

### Phenotype of Human MAPCs Under Conditions Described in U.S. 10/048,757

Immunophenotypic analysis by FACS of human MAPCs obtained after about 22-25 cell doublings showed that the cells do not express CD31, CD34, CD36, CD38, CD45, CD50, CD62E and -P, HLA-DR, Muc18, STRO-1, cKit, Tie/Tek; and express low levels of CD44, HLA-class I and β2-microglobulin, and express CD10, CD13, CD49b, CD49e, CDw90, Flk1 (N>10).

Once cells underwent >40 doublings in cultures re-seeded at about 2x10³ cells/cm², the phenotype became more homogenous and no cell expressed HLA class-I or CD44 (n=6). When cells were grown at higher confluence, they expressed high levels of Muc18, CD44, HLA class I and β2-microglobulin, which is similar to the phenotype described for MSC (N=8) (Pittenger, 1999).

Immunohistochemistry showed that human MAPCs grown at about 2x10³ cells/cm² seeding density express EGF-R, TGF-R1 and -2, BMP-R1A, PDGF-R1A and - B, and that a small subpopulation (between about 1 and about 10%) of MAPCs stain with anti-SSEA4 antibodies (Kannagi, R 1983).

Using Clontech cDNA arrays the expressed gene profile of human MAPCs cultured at seeding densities of about 2x10³ cells/cm² for about 22 and about 26 cell doublings was determined:
A. MAPCs did not express CD31, CD36, CD62E, CD62P, CD44-H, cKit, Tie, receptors for IL1, IL3, IL6, IL11, G CSF, GM-CSF, Epo, Flt3-L, or CNTF, and low levels of HLA-class-I, CD44-E and Muc-18 mRNA.
B. MAPCs expressed mRNA for the cytokines BMP1, BMP5, VEGF, HGF, KGF, MCP1; the cytokine receptors Flk1, EGF-R, PDGF-R1α, gp130, LIF-R, activin-R1 and - R2, TGFR-2, BMP-R1A; the adhesion receptors CD49c, CD49d, CD29; and CD10.
C. MAPCs expressed mRNA for hTRT and TRF1; the POU domain transcription factor Oct-4, sox-2 (required with Oct-4 to maintain undifferentiated state of ES/EC, Uwanogho D. 1995), sox 11 (neural development), sox 9 (chondrogenesis) (Lefebvre V. 1998); homeodeomain transcription factors: Hoxa4 and -a5 (cervical and thoracic skeleton specification; organogenesis of respiratory tract) (Packer, A.I. 2000), Hox-a9 (myelopoiesis) (Lawrence, H. 1997), Dlx4 (specification of forebrain and peripheral structures of head) (Akimenko, M.A. 1994), MSX1 (embryonic mesoderm, adult heart and muscle, chondro- and osteogenesis) (Foerst-Potts, L. 1997), PDX1 (pancreas) (Offield, M.F. 1996).
D. Presence of Oct-4, LIF-R, and hTRT mRNA was confirmed by RT-PCR.
E. In addition, RT-PCR showed that Rex-1 mRNA and Rox-1 mRNA were expressed in MAPCs.

Oct-4, Rex-1 and Rox-1 were expressed in MAPCs derived from human and murine marrow and from murine liver and brain. Human MAPCs expressed LIF-R and stained positive with SSEA-4. Finally, Oct-4, LIF-R, Rex-1 and Rox-1 mRNA levels were found to increase in human MAPCs cultured beyond 30 cell doublings, which resulted in phenotypically more homogenous cells. In contrast, MAPCs cultured at high density lost expression of these markers. This was associated with senescence before about 40 cell doublings and loss of differentiation to cells other than chondroblasts, osteoblasts and adipocytes.

### Culturing MAPCs as Described in U.S. 10/048,757

MAPCs isolated as described herein can be cultured using methods disclosed herein and in U.S. 10/048,757, which is incorporated by reference for these methods.

Briefly, for the culture of MAPCs, culture in low-serum or serum-free medium was preferred to maintain the cells in the undifferentiated state. Medium used to culture the cells, as described herein, was supplemented as described in Table 1. Human MAPCs do not require LIF.

**Table 1**

| | |
|---|---|
| Insulin | about 10 - 50 µg/ml (about 10 µg/ml)* |
| Transferrin | about 0 - 10 µg/ml (about 5.5 µg/ml) |
| Selenium | about 2 -10 ng/ml (about 5 ng/ml) |
| Bovine serum albumin (BSA) | about 0.1 - 5 µg/ml (about 0.5 µg/ml) |
| Linoleic acid | about 2 - 10 µg/ml (about 4.7 µg/ml) |
| Dexamethasone | about 0.005 - 0.15 µM (about 0.01 µM) |
| L-ascorbic acid 2-phosphate | about 0.1 mM |
| Low-glucose DMEM (DMEM-LG) | about 40 - 60% (about 60%) |
| MCDB-201 | about 40 - 60% (about 40%) |
| Fetal calf serum | about 0-2% |
| Platelet-derived growth | about 5 - 15 ng/ml (about 10 ng/ml) |
| Epidermal growth factor | about 5 - 15 ng/ml (about 10 ng/ml) |
| Insulin like growth factor | about 5 - 15 ng/ml (about 10 ng/ml) |
| Leukemia inhibitory factor | about 10-10,000IU (about 1,000 IU) |

| | |
|---|---|
| * Preferred concentrations are shown in parentheses. | |

Addition of about 10 ng/mL LIF to human MAPCs did not affect short-term cell growth (same cell doubling time till 25 cell doublings, level of Oct-4 (Oct-3/4) expression). In contrast to what was seen with human cells, when fresh murine marrow mononuclear cells, depleted on day 0 of CD45⁺ cells, were plated in MAPC culture, no growth was seen. When murine marrow mononuclear cells were plated, and cultured cells 14 days later depleted of CD45⁺ cells, cells with the morphology and phenotype similar to that of human MAPCs appeared. This suggested that factors secreted by hematopoietic cells were needed to support initial growth of murine MAPCs. When cultured with PDGF-BB and EFG alone, cell doubling was slow (>6 days) and cultures could not be maintained beyond about 10 cell doublings. Addition of about 10 ng/mL LIF significantly enhanced cell growth.

Once established in culture, cells can be frozen and stored as frozen stocks, using DMEM with about 40% FCS and about 10% DMSO. Other methods for preparing frozen stocks for cultured cells are also available to those of skill in the art.

Thus, MAPCs can be maintained and expanded in culture medium that is available to the art. Such media include, but are not limited to, Dulbecco's Modified Eagle's Medium® (DMEM), DMEM F12 medium®, Eagle's Minimum Essential Medium®, F-12K medium®, Iscove's Modified Dulbecco's Medium®, RPMI-1640 medium®. Many media are also available as a low-glucose formulation, with or without sodium pyruvate.

Also contemplated is supplementation of cell culture medium with mammalian sera. Sera often contain cellular factors and components that are necessary for viability and expansion. Examples of sera include fetal bovine serum (FBS), bovine serum (BS), calf serum (CS), fetal calf serum (FCS), newborn calf serum (NCS), goat serum (GS), horse serum (HS), human serum, chicken serum, porcine serum, sheep serum, rabbit serum, serum replacements, and bovine embryonic fluid. It is understood that sera can be heat-inactivated at about 55-65°C if deemed necessary to inactivate components of the complement cascade.

Additional supplements can also be used advantageously to supply the cells with the trace elements for optimal growth and expansion. Such supplements include insulin, transferrin, sodium selenium and combinations thereof. These components can be included in a salt solution such as, but not limited to Hanks' Balanced Salt Solution® (HBSS), Earle's Salt Solution®, antioxidant supplements, MCDB-201® supplements, phosphate buffered saline (PBS), ascorbic acid and ascorbic acid-2-phosphate, as well as additional amino acids. Many cell culture media already contain amino acids, however some require supplementation prior to culturing cells. Such amino acids include, but are not limited to, L-alanine, L-arginine, L-aspartic acid, L-asparagine, L-cysteine, L-cystine, L-glutamic acid, L-glutamine, L-glycine, L-histidine, L-isoleucine, L-leucine, L-lysine, L-methionine, L-phenylalanine, L-proline, L-serine, L-threonine, L-tryptophan, L-tyrosine and L-valine. It is well within the skill of one in the art to determine the proper concentrations of these supplements.

Antibiotics are also typically used in cell culture to mitigate bacterial, mycoplasmal and fungal contamination. Typically, antibiotics or anti-mycotic compounds used are mixtures of penicillin/streptomycin, but can also include, but are not limited to, amphotericin (Fungizone®), ampicillin, gentamicin, bleomycin, hygromycin, kanamycin, mitomycin, mycophenolic acid, nalidixic acid, neomycin, nystatin, paromomycin, polymyxin, puromycin, rifampicin, spectinomycin, tetracycline, tylosin and zeocin. Antibiotic and antimycotic additives can be of some concern, depending on the type of work being performed. One possible situation that can arise is an antibiotic-containing media wherein bacteria are still present in the culture, but the action of the antibiotic performs a bacteriostatic rather than bacteriocidal mechanism. Also, antibiotics can interfere with the metabolism of some cell types.

Hormones can also be advantageously used in cell culture and include, but are not limited to, D-aldosterone, diethylstilbestrol (DES), dexamethasone, β-estradiol, hydrocortisone, insulin, prolactin, progesterone, somatostatin/human growth hormone (HGH), thyrotropin, thyroxine and L-thyronine.

Lipids and lipid carriers can also be used to supplement cell culture media, depending on the type of cell and the fate of the differentiated cell. Such lipids and carriers can include, but are not limited to cyclodextrin (α, β, γ), cholesterol, linoleic acid conjugated to albumin, linoleic acid and oleic acid conjugated to albumin, unconjugated linoleic acid, linoleic-oleic-arachidonic acid conjugated to albumin, oleic acid unconjugated and conjugated to albumin, among others.

Also contemplated is the use of feeder cell layers. Feeder cells are used to support the growth of fastidious cultured cells, including stem cells. Feeder cells are normal cells that have been inactivated by γ-irradiation. In culture, the feeder layer serves as a basal layer for other cells and supplies cellular factors without further growth or division of their own (Lim, J.W. and Bodnar, A., 2002). Examples of feeder layer cells are typically human diploid lung cells, mouse embryonic fibroblasts, Swiss mouse embryonic fibroblasts, but can be any post-mitotic cell that is capable of supplying cellular components and factors that are advantageous in allowing optimal growth, viability and expansion of stem cells. In many cases, feeder cell layers are not necessary to keep the ES cells in an undifferentiated, proliferative state, as leukemia inhibitory factor (LIF) has anti-differentiation properties. Therefore, supplementation with LIF could be used to maintain MAPC in some species in an undifferentiated state.

Cells in culture can be maintained either in suspension or attached to a solid support, such as extracellular matrix components and synthetic or biopolymers. Stem cells often require additional factors that encourage their attachment to a solid support, such as type I, type II and type IV collagen, concanavalin A, chondroitin sulfate, fibronectin, "superfibronectin" and fibronectin-like polymers, gelatin, laminin, poly-D and poly-L-lysine, thrombospondin and vitronectin.

The maintenance conditions of stem cells can also contain cellular factors that allow stem cells, such as MAPCs, to remain in an undifferentiated form. It is advantageous under conditions where the cell must remain in an undifferentiated state of self-renewal for the medium to contain epidermal growth factor (EGF), platelet derived growth factor (PDGF), leukemia inhibitory factor (LIF; in selected species), and combinations thereof. It is apparent to those skilled in the art that supplements that allow the cell to self-renew but not differentiate should be removed from the culture medium prior to differentiation.

Stem cell lines and other cells can benefit from co-culturing with another cell type. Such co-culturing methods arise from the observation that certain cells can supply yet-unidentified cellular factors that allow the stem cell to differentiate into a specific lineage or cell type. These cellular factors can also induce expression of cell-surface receptors, some of which can be readily identified by monoclonal antibodies. Generally, cells for co-culturing are selected based on the type of lineage one skilled in the art wishes to induce, and it is within the capabilities of the skilled artisan to select the appropriate cells for co-culture.

MAPCs and progeny differentiated from MAPCs are useful as a source of cells for specific vascular lineages. The maturation, proliferation and differentiation of MAPCs may be effected through many pathways, including but not limited to activation or inhibition of Notch and/or patched receptors, such as by exposing MAPCs to appropriate factors, *in vitro* or *in vivo,* including, but not limited to, one or more members of the TGF-*β* pathway ((Waite and Eng, 2003; Sorensen et al., 2003) including but not limited to TGF-*β*, TGF-*β*1, TGF-*β*2, TGF-*β*3, Alk-1, Alk-5, Bone Morphogenic Proteins (BMP), Activins, deapentaplegic (DPP) and GFD5 (Growth and differentiation factor 5)), one or more VEGF ((Yancopoulos, G.D., et. al., 2000; Shima and Mailhos, 2000; Robinson and Stringer, 2001; Mukouyama et al., 2002; Stalmans et al., 2002; Mukouyama et al., 2005; Cleaver and Krieg, 1998)) including, but not limited to, VEGF_{A}, VEGF_{B}, VEGF_{C}, VEGF_{D}, VEGF_{E} (not mammalian), VEGF₁₂₁, VEGF₁₄₅, VEGF₁₆₅, VEGF₁₈₉, VEGF₁₈₃, VEGF₂₀₆, P1GF; VEGF_{C} and VEGF_{D} may be useful in the development of lymphatic endothelium) one or more neuropilin ((Mukouyama et al,. 2005) including, but not limited to, NP-1 and NP-2), one or more angiopoietin ((Moyon et al,. 2001) including but not limited to Ang-1, Ang-2, Ang-3 (mouse) and Ang-4 (the human ortholog of mouse Ang-3)), one or more members of the Notch pathway ((Villa et al., 2001; Zhong et al., 2001; Lawson et al., 2002; Liu et al., 2003) such as ligands, including, but not limited to, delta-like (including but not limited to D11-1, Dll-3 and Dll-4), Jagged (including but not limited to Jagged-1 and Jagged-2), delta, serrate, scabrous and Fringe proteins (the Notch receptors include but are not limited to Notch-1, Notch-2, Notch-3 and Notch-4)), one or more members of the patched pathway ((Lawson et al., 2002) such as ligands, including, but not limited to, sonic hedgehog (Shh; Chuong et a., 2000; Cohen MM Jr., 2004), Indian hedgehog (Ihh) and Desert hedgehog (Dhh)) (patched receptors include but are not limited to ptc-1 and ptc-2), one or more agents that inhibits sonic hedgehog activity (including, but not limited to, cyclopamine and anti-SHH antibody), Notch pathway activity or patched pathway activity, or with stromal cells or other cells which secrete factors responsible for stem cell regeneration, commitment and differentiation (the citations of which are incorporated herein by reference for their description of the factors/receptors). Exposing a cell to one or more of the factors includes not only exposure to an exogenous factor, but alo to an endogenous factor. The endogenous factor can be activated or increased in the cell by methods know in the art. The latter includes homolgous recombination (e.g., U.S. 5,641,670), non-homologus recombination (e.g., U.S. 6,602,686; RAGE-PE^{™} (Random Activation of Gene Expression for Protein Expression) technology; Athersys, Inc. (Cleveland, Ohio)), or other endogenous expression techniques available to the art worker (the above mentioned patents are incorporated by reference for teaching of methods of endogenous gene activation). Useful concentration ranges for factors of use in the invention are generally between about 5 ng/ml to about 500 ng/ml, including about 5 ng/ml to about 100 ng/ml, about 10 ng/ml to abut 100 ng/ml, about 5 ng/ml to about 50 ng/ml and about 5 ng/ml to about 20 ng/ml.

In addition to the factors/genes described herein, variants, homologs or orthologs of the factors/genes, which have the same biological function/acitivty, can be used or assayed for in methods of the invention. For example, variants, homolog or orthologs of use in the present invention may be homologous or have sequence identity (nucleotide or amino acid sequence) with factors/genes involved in the Notch and patched pathways and others, including those factors/genes provided herein. Examples of assays and programs to determine if a factor/gene is homolgous is provided herein and is known in the art. "Homology" refers to the percent identity between two polynucleotide or two polypeptide sequences. Two DNA or polypeptide sequences are "substantially homologous" to each other when the sequences exhibit at least about 75% to 85%, preferably at least about 90%, and most preferably at least about 95% to 98% contiguous sequence identity over a defined length of the sequences.

The following terms are used to describe the sequence relationships between two or more nucleic acids or polynucleotides: (a) "reference sequence", (b) "comparison window", (c) "sequence identity", (d) "percentage of sequence identity", and (e) "substantial identity".
(a) As used herein, "reference sequence" is a defined sequence used as a basis for sequence comparison. A reference sequence may be a subset or the entirety of a specified sequence; for example, as a segment of a full length cDNA or gene sequence, or the complete cDNA or gene sequence.
(b) As used herein, "comparison window" makes reference to a contiguous and specified segment of a polynucleotide sequence, wherein the polynucleotide sequence in the comparison window may comprise additions or deletions (*i.e.*, gaps) compared to the reference sequence (which does not comprise additions or deletions) for optimal alignment of the two sequences. Generally, the comparison window is at least 20 contiguous nucleotides in length, and optionally can be 30, 40, 50, 100, or longer. Those of skill in the art understand that to avoid a high similarity to a reference sequence due to inclusion of gaps in the polynucleotide sequence a gap penalty is typically introduced and is subtracted from the number of matches.

Methods of alignment of sequences for comparison are well known in the art. Thus, the determination of percent identity between any two sequences can be accomplished using a mathematical algorithm. Computer implementations of these mathematical algorithms can be utilized for comparison of sequences to determine sequence identity. Such implementations include, but are not limited to: CLUSTAL in the PC/Gene program (available from Intelligenetics, Mountain View, California); the ALIGN program (Version 2.0) and GAP, BESTFIT, BLAST, FASTA, and TFASTA in the Wisconsin Genetics Software Package, Version 8 (available from Genetics Computer Group (GCG), 575 Science Drive, Madison, Wisconsin, USA). Alignments using these programs can be performed using the default parameters.

Software for performing BLAST analyses is publicly available through the National Center for Biotechnology Information (http://www.ncbi.nlm.nih.gov/). This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul et al., 1990). These initial neighborhood word hits act as seeds for initiating searches to find longer HSPs containing them. The word hits are then extended in both directions along each sequence for as far as the cumulative alignment score can be increased. Cumulative scores are calculated using, for nucleotide sequences, the parameters M (reward score for a pair of matching residues; always > 0) and N (penalty score for mismatching residues; always < 0). For amino acid sequences, a scoring matrix is used to calculate the cumulative score. Extension of the word hits in each direction are halted when the cumulative alignment score falls off by the quantity X from its maximum achieved value, the cumulative score goes to zero or below due to the accumulation of one or more negative-scoring residue alignments, or the end of either sequence is reached.

In addition to calculating percent sequence identity, the BLAST algorithm also performs a statistical analysis of the similarity between two sequences. One measure of similarity provided by the BLAST algorithm is the smallest sum probability (P(N)), which provides an indication of the probability by which a match between two nucleotide or amino acid sequences would occur by chance. For example, a test nucleic acid sequence is considered similar to a reference sequence if the smallest sum probability in a comparison of the test nucleic acid sequence to the reference nucleic acid sequence is less than about 0.1, more preferably less than about 0.01, and most preferably less than about 0.001.

To obtain gapped alignments for comparison purposes, Gapped BLAST (in BLAST 2.0) can be utilized. Alternatively, PSI-BLAST (in BLAST 2.0) can be used to perform an iterated search that detects distant relationships between molecules. When utilizing BLAST, Gapped BLAST, PSI-BLAST, the default parameters of the respective programs (e.g. BLASTN for nucleotide sequences, BLASTX for proteins) can be used. The BLASTN program (for nucleotide sequences) uses as defaults a wordlength (W) of 11, an expectation (E) of 10, a cutoff of 100, M=5, N=-4, and a comparison of both strands. For amino acid sequences, the BLASTP program uses as defaults a wordlength (W) of 3, an expectation (E) of 10, and the BLOSUM62 scoring matrix. See http://www.ncbi.nlm.nih.gov. Alignment may also be performed manually by inspection.

For purposes of the present invention, comparison of nucleotide sequences for determination of percent sequence identity to the factors/markers of use with the invention is preferably made using the BlastN program (version 1.4.7 or later) with its default parameters or any equivalent program. By "equivalent program" is intended any sequence comparison program that, for any two sequences in question, generates an alignment having identical nucleotide or amino acid residue matches and an identical percent sequence identity when compared to the corresponding alignment generated by the preferred program.
(c) As used herein, "sequence identity" or "identity" in the context of two nucleic acid or polypeptide sequences makes reference to the residues in the two sequences that are the same when aligned for maximum correspondence over a specified comparison window. When percentage of sequence identity is used in reference to proteins it is recognized that residue positions which are not identical often differ by conservative amino acid substitutions, where amino acid residues are substituted for other amino acid residues with similar chemical properties (*e.g.*, charge or hydrophobicity) and therefore do not change the functional properties of the molecule. When sequences differ in conservative substitutions, the percent sequence identity may be adjusted upwards to correct for the conservative nature of the substitution. Sequences that differ by such conservative substitutions are said to have "sequence similarity" or "similarity." Means for making this adjustment are well known to those of skill in the art. Typically this involves scoring a conservative substitution as a partial rather than a full mismatch, thereby increasing the percentage sequence identity. Thus, for example, where an identical amino acid is given a score of 1 and a non-conservative substitution is given a score of zero, a conservative substitution is given a score between zero and 1. The scoring of conservative substitutions is calculated, e.g., implemented in the program PC/GENE (Intelligenetics, Mountain View, California).
(d) As used herein, "percentage of sequence identity" means the value determined by comparing two optimally aligned sequences over a comparison window, wherein the portion of the polynucleotide sequence in the comparison window may comprise additions or deletions (i.e., gaps) as compared to the reference sequence (which does not comprise additions or deletions) for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison, and multiplying the result by 100 to yield the percentage of sequence identity.
(e)(i) The term "substantial identity" of polynucleotide sequences means that a polynucleotide comprises a sequence that has at least 50% or 60% or 70%, 71%, 72%, 73%, 74%, 75%, 76%, 77%, 78%, or 79%, or at least 80%, 81%, 82%, 83%, 84%, 85%, 86%, 87%, 88%, or 89%, or at least 90%, 91%, 92%, 93%, or 94%, or at least 95%, 96%, 97%, 98%, or 99% sequence identity, compared to a reference sequence using one of the alignment programs described using standard parameters. One of skill in the art will recognize that these values can be appropriately adjusted to determine corresponding identity of proteins encoded by two nucleotide sequences by taking into account codon degeneracy, amino acid similarity, reading frame positioning, and the like. Substantial identity of amino acid sequences for these purposes normally means sequence identity of at least 50%, including at least 80%, 90%, and at least 95%.

Another indication that nucleotide sequences are substantially identical is if two molecules hybridize to each other under low, medium and/or stringent conditions (see below). Generally, stringent conditions are selected to be about 5°C lower than the thermal melting point (Tₘ) for the specific sequence at a defined ionic strength and pH. However, stringent conditions encompass temperatures in the range of about 1°C to about 20°C, depending upon the desired degree of stringency as otherwise qualified herein. Nucleic acids that do not hybridize to each other under stringent conditions are still substantially identical if the polypeptides they encode are substantially identical. This may occur, e.g., when a copy of a nucleic acid is created using the maximum codon degeneracy permitted by the genetic code. One indication that two nucleic acid sequences are substantially identical is when the polypeptide encoded by the first nucleic acid is immunologically cross reactive with the polypeptide encoded by the second nucleic acid.
(e)(ii) The term "substantial identity" in the context of a peptide indicates that a peptide comprises a sequence with at least 50% or 60% or 70%, 71%, 72%, 73%, 74%, 75%, 76%, 77%, 78%, or 79%, or at least 80%, 81%, 82%, 83%, 84%, 85%, 86%, 87%, 88%, or 89%, or at least 90%, 91%, 92%, 93%, or 94%, or at least 95%, 96%, 97%, 98% or 99%, sequence identity to the reference sequence over a specified comparison window. Preferably, optimal alignment is conducted using the homology alignment algorithm of Needleman and Wunsch (1970). An indication that two peptide sequences are substantially identical is that one peptide is immunologically reactive with antibodies raised against the second peptide. Thus, a peptide is substantially identical to a second peptide, for example where the two peptides differ only by a conservative substitution.

For sequence comparison, typically one sequence acts as a reference sequence to which test sequences are compared. When using a sequence comparison algorithm, test and reference sequences are input into a computer, subsequence coordinates are designated if necessary, and sequence algorithm program parameters are designated. The sequence comparison algorithm then calculates the percent sequence identity for the test sequence(s) relative to the reference sequence, based on the designated program parameters.

As noted above, another indication that two nucleic acid sequences are substantially identical is that the two molecules hybridize to each other under low, medium or stringent conditions. The phrase "hybridizing specifically to" refers to the binding, duplexing, or hybridizing of a molecule only to a particular nucleotide sequence under stringent conditions when that sequence is present in a complex mixture (e.g., total cellular) DNA or RNA. "Bind(s) substantially" refers to complementary hybridization between a probe nucleic acid and a target nucleic acid and embraces minor mismatches that can be accommodated by reducing the stringency of the hybridization media to achieve the desired detection of the target nucleic acid sequence.

"Stringent hybridization conditions" and "stringent hybridization wash conditions" in the context of nucleic acid hybridization experiments such as Southern and Northern hybridization are sequence dependent, and are different under different environmental parameters. The Tₘ is the temperature (under defined ionic strength and pH) at which 50% of the target sequence hybridizes to a perfectly matched probe. Specificity is typically the function of post-hybridization washes, the issues being the ionic strength and temperature of the final wash solution. For DNA-DNA hybrids, the Tₘ can be approximated from the equation of Meinkoth and Wahl, 1984; Tₘ 81.5°C + 16.6 (log M) +0.41 (%GC) - 0.61 (% form) - 500/L; where M is the molarity of monovalent cations, %GC is the percentage of guanosine and cytosine nucleotides in the DNA, % form is the percentage of formamide in the hybridization solution, and L is the length of the hybrid in base pairs. Tₘ is reduced by about 1 °C for each 1% of mismatching; thus, Tₘ, hybridization, and/or wash conditions can be adjusted to hybridize to sequences of the desired identity. For example, if sequences with >90% identity are sought, the Tₘ can be decreased 10°C. Generally, stringent conditions are selected to be about 5°C lower than the thermal melting point I for the specific sequence and its complement at a defined ionic strength and pH. However, severely stringent conditions can utilize a hybridization and/or wash at 1, 2, 3, or 4°C lower than the thermal melting point I; moderately stringent conditions can utilize a hybridization and/or wash at 6, 7, 8, 9, or 10°C lower than the thermal melting point I; low stringency conditions can utilize a hybridization and/or wash at 11, 12, 13, 14, 15, or 20°C lower than the thermal melting point I. Using the equation, hybridization and wash compositions, and desired T, those of ordinary skill will understand that variations in the stringency of hybridization and/or wash solutions are inherently described. If the desired degree of mismatching results in a T of less than 45°C (aqueous solution) or 32°C (formamide solution), it is preferred to increase the SSC concentration so that a higher temperature can be used. Generally, highly stringent hybridization and wash conditions are selected to be about 5°C lower than the thermal melting point Tₘ for the specific sequence at a defined ionic strength and pH.

An example of highly stringent wash conditions is 0.15 M NaCl at 72°C for about 15 minutes. An example of stringent wash conditions is a 0.2X SSC wash at 65°C for 15 minutes. Often, a high stringency wash is preceded by a low stringency wash to remove background probe signal. An example medium stringency wash for a duplex of, e.g., more than 100 nucleotides, is 1X SSC at 45°C for 15 minutes. An example low stringency wash for a duplex of, e.g., more than 100 nucleotides, is 4-6X SSC at 40°C for 15 minutes. For short probes (e.g., about 10 to 50 nucleotides), stringent conditions typically involve salt concentrations of less than about 1.5 M, more preferably about 0.01 to 1.0 M, Na ion concentration (or other salts) at pH 7.0 to 8.3, and the temperature is typically at least about 30°C and at least about 60°C for long robes (e.g., >50 nucleotides). Stringent conditions may also be achieved with the addition of destabilizing agents such as formamide. In general, a signal to noise ratio of 2X (or higher) than that observed for an unrelated probe in the particular hybridization assay indicates detection of a specific hybridization. Nucleic acids that do not hybridize to each other under stringent conditions are still substantially identical if the proteins that they encode are substantially identical. This occurs, e.g., when a copy of a nucleic acid is created using the maximum codon degeneracy permitted by the genetic code.

Very stringent conditions are selected to be equal to the Tₘ for a particular probe. An example of stringent conditions for hybridization of complementary nucleic acids which have more than 100 complementary residues on a filter in a Southern or Northern blot is 50% formamide, e.g., hybridization in 50% formamide, 1 M NaCl, 1% SDS at 37°C, and a wash in 0. 1X SSC at 60 to 65°C. Exemplary low stringency conditions include hybridization with a buffer solution of 30 to 35% formamide, 1 M NaCl, 1% SDS (sodium dodecyl sulphate) at 37°C, and a wash in 1X to 2X SSC (20X SSC = 3.0 M NaCl/0.3 M trisodium citrate) at 50 to 55°C. Exemplary moderate stringency conditions include hybridization in 40 to 45% formamide, 1.0 M NaCl, 1% SDS at 37°C, and a wash in 0.5X to 1X SSC at 55 to 60°C.

The following are examples of sets of hybridization/wash conditions that may be used to clone orthologous nucleotide sequences that are substantially identical to reference nucleotide sequences of the present invention: a reference nucleotide sequence preferably hybridizes to the reference nucleotide sequence in 7% sodium dodecyl sulfate (SDS), 0.5 M NaPO₄, 1 mM EDTA at 50°C with washing in 2X SSC, 0.1% SDS at 50°C, more desirably in 7% sodium dodecyl sulfate (SDS), 0.5 M NaPO₄, 1 mM EDTA at 50°C with washing in 1X SSC, 0.1% SDS at 50°C, more desirably still in 7% sodium dodecyl sulfate (SDS), 0.5 M NaPO₄, 1 mM EDTA at 50°C with washing in 0.5X SSC, 0.1% SDS at 50°C, preferably in 7% sodium dodecyl sulfate (SDS), 0.5 M NaPO₄, 1 mM EDTA at 50°C with washing in 0.1X SSC, 0.1% SDS at 50°C, more preferably in 7% sodium dodecyl sulfate (SDS), 0.5 M NaPO₄, 1 mM EDTA at 50°C with washing in 0.1X SSC, 0.1% SDS at 65°C.

As described in the Example herein below, MAPCs were differentiated into vascular endothelial cells *in vitro.* Briefly, MAPCs were cultured in medium containing VEGF₁₆₅ (for example, about 10 ng/ml to about 100 ng/ml) or VEGF₁₂₁ (for example, about 10 ng/ml to about 100 ng/ml), and optionally a Notch ligand (including, but not limited to, Dll-4 and Jagged-1 (for example, about 10 ng/ml to about 100 ng/ml)) and/or a patched ligand (including, but not limited to sonic hedgehog (for example, about 10 ng/ml to about 100 ng/ml)). Additionally, the cells can be exposed to one or more agents that modulates, including negatively or positively modulates, the Notch and/or patched pathways.

Methods of identifying and subsequently separating differentiated cells from their undifferentiated counterparts can be carried out by methods well known in the art and those described herein. Cells that have been induced to differentiate can be identified by selectively culturing cells under conditions whereby differentiated cells outnumber undifferentiated cells. Similarly, differentiated cells can be identified by morphological changes and characteristics that are not present on their undifferentiated counterparts, such as cell size, the number of cellular processes, or the complexity of intracellular organelle distribution.

Also contemplated are methods of identifying differentiated cells by their expression of specific cell-surface markers such as cellular receptors and transmembrane proteins. Monoclonal antibodies against these cell-surface markers can be used to identify differentiated cells. Detection of these cells can be achieved through fluorescence activated cell sorting (FACS) and enzyme-linked immunosorbent assay (ELISA). From the standpoint of transcriptional upregulation of specific genes, differentiated cells often display levels of gene expression that are different (increased or decreased expression of mRNA and/or protein) from undifferentiated cells.

In the present case, differentiated cells can be identified by gene expression levels, which are different from their undifferentiated counterparts, of arterial, venous and/or lymphatic markers, including, but not limited to, EphrinB1 (*e.g.*, accession no. NP_034240 or NM_004429; Bagley et al., 2003), EphrinB2 (*e.g.*, accession no. NP_004084 or NM_004093; Bagley et al., 2003), Dll-4 (*e.g.*, accession no. BAB18581 or AB043894; Shutter et al., 2000), Hey-2 (e.g., accession no. CAI20068 or AL078594; Gridlock zebrafish orthologue; Zhong et al., 2001; Zhong et al., 2000), Notch 1 (e.g., accession no. AAG33848 or AF308602.1) and 4 (e.g., NM_004557; Villa et al., 2001), Jagged-1 (e.g., accession no. AAC52020 or U73936) and 2 (e.g., accession no. AAB61285 or AF003521; Villa et al,. 2001) and connexin-40 (e.g., accession no. AAD37801 or AF151979 (arterial markers); Lefty-1 (e.g., accession no. AAD55580 (chicken) or ; AF179483 (chicken); Chi et al,. 2003), Lefty-2 (e.g., NP_571036 (zebrafish) and NM_130961 (zebrafish); Chi et al., 2003), COUP-TFII (e.g., accession no. AAA19854 (mouse) or U07635 (mouse); You et al., 2003) and MYO1-*β* (Chi et al., 2003), EphB4 (e.g., accession no. EAL23820) ; Bagley et al., 2003) (venous makers) and podoplanin (e.g., accession no. AAM73655 or AF390106), prox-1 (e.g., accession no. AAC50656 or U44060) and lyve-1 (e.g., accession no. AAD42764 or AF118108; Conway, 2001; Oettgen, 2001; Partanen, 2001) (lymphatic markers) (the citations of which are incorporated herein by reference for their description of the markers)). Reverse-transcription polymerase chain reaction (RT-PCR) can be used to monitor such changes in gene expression in response to differentiation. In addition, whole genome analysis using microarray technology can be used to identify differentiated cells.

Accordingly, once differentiated cells are identified, they can be separated from their undifferentiated counterparts, if necessary. The methods of identification detailed above also provide methods of separation, such as FACS, preferential cell culture methods, ELISA, magnetic beads, and combinations thereof. A preferred embodiment of the invention envisions the use of FACS to identify and separate cells based on cell-surface antigen expression.

### Additional Culture Methods

The density at which MAPCs are cultured can vary from about 100 cells/cm² or about 150 cells/cm² to about 10,000 cells/cm², including about 200 cells/cm² to about 1500 cells/cm² to about 2,000 cells/cm². The density can vary between species. Additionally, optimal density can vary depending on culture conditions and source of cells. It is within the skill of the ordinary artisan to determine the optimal density for a given set of culture conditions and cells.

Also, effective atmospheric oxygen concentrations of less than about 10%, including about 3 to about 5% , can be used at any time during the isolation, growth and differentiation of MAPCs in culture.

Isolating and culturing MAPCs at 5% O₂ was shown to result in fewer cytogenetic abnormalities. Additionally, it resulted in a slight change in the phenotype of MAPCs. When rodent MAPCs were isolated and maintained at 5% O₂, Oct-4 transcript levels approached those of embryonic stem (ES) cells (50-80%), and >90% of cells express nuclear Oct-4 protein by immunohistochemistry. 5%-O₂ derived rodent MAPCs also expressed Rex1 at levels approaching that of ES cells, suggesting that Oct-4 is functional within these cells. However, Nanog mRNA remained almost undetectable. Low-O₂ derived mouse MAPCs were Sca1, Thy1, CD34, CD31, MHC-class I and II, CD44 negative, but cKit positive. Although mouse MAPCs expressed Oct-4 mRNA at levels similar to ES cells, they did not form embryoid bodies or teratomas (5x10⁶ MAPCs grafted under the skin of 5 nude mice). (U.S. Provisional Patent Application No. 60/625,426 (filed November 4, 2004) is incorporated herein by reference for its description of MAPC and embryonic stem cell culture at low O₂ concentrations.)

MAPCs and embryonic stem cells can also be cultured in the presence of a GSK3 inhibitor (*e.g.*, at a concentration of about 100 nM to about 1 µM to about 2 µM). The presence of a GSK3 inhibitor allows one to culture the cells at high density without losing differentiation potential. For example, non-embryonic or embryonic stem cells can be cultured at a density of about 8,000 to about 50,000 cells/cm² in the presence of a GSK-3 inhibitor while retaining their ability to differentiate into cell types of more than one embryonic lineage (incorporated herein by reference are U.S. Provisional Patent Application Nos. 60/703,823 (filed July 29, 2005) and 60/704,169 (filed July 29, 2005) for the disclosure of culturing cells in the present of a GSK3 inhibitor). For example, MAPCs or embryonic stem cells can be cultured with a GSK-3 inhibitor of formula (I): 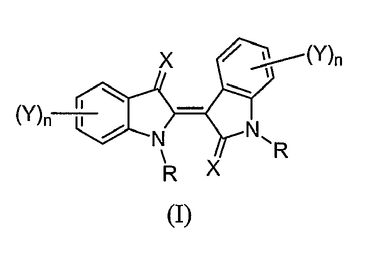 wherein each X is independently O, S, N-OH, N(Z), or two groups independently selected from H, NO₂ phenyl, and (C₁-C₆)alkyl;
each Y is independently H, (C₁-C₆)alkyl, phenyl, N(Z)(Z), sulfonyl, phosphonyl, F, Cl, Br, or I;
each Z is independently H, (C₁-C₆)alkyl, phenyl, benzyl, or both Z groups together with the nitrogen to which they are attached form 5, 6, or 7-membered heterocycloalkyl;
each n is independently 0, 1, 2, 3, or 4;
each R is independently H, (C₁-C₆)alkyl, phenyl, benzyl, or benzoyl; and
wherein alkyl is branched or straight-chain, optionally substituted with 1, 2, 3, 4, or 5 OH, N(Z)(Z), (C₁-C₆)alkyl, phenyl, benzyl, F, Cl, Br, or I; and
wherein any phenyl, benzyl, or benzoyl is optionally substituted with 1, 2, 3, 4, or 5 OH, N(Z)(Z), (C₁-C₆)alkyl, F, Cl, Br, or I; or a salt thereof.

For example, the compound of formula includes a 6-bromoindirubin compound, including but not limited to, 6-bromoindirubin-3'-oxime (BIO).

### Vascular Endothelial Cells of the Invention

The present invention relates to vascular endothelial cells and methods of preparation, culture, and use thereof. hMAPCs cultured in the presence of VEGF₁₆₅ were found to acquire endothelial cell markers, including VEGF-R1 and 2, Tie-1, Tie-2, KDR, Flt-1, CD26, CD105, *α*ᵥ*β*₃, CD34, VE-cadherin and von Willebrand Factor. They also increased expression for markers for arterial (Hey-2, Dll-4, EphrinB2 and EphrinB1) and venous (EphB4) endothelium. Expression of Hey-2, Dll-4, EphrinB1, EphrinB2 and EphB4 mRNA were found to be up-regulated in the present vascular endothelial cells obtained from hMAPCs, thus demonstrating the potential for arterial and venous endothelial differentiation of hMAPCs. A subset of the population of differentiated cells expressed smooth muscle actin, a marker of smooth muscle, showing that hMAPCs can differentiate into both endothelial cells and smooth muscle cells under the same conditions. Additionally, hMAPC derived endothelial cells were found to express low levels of proteins usually present in microvascular endothelium, such as CD36 and CD34, which indicate that most of the cells had a macrovascular phenotype. Endothelial cells derived from MAPCs were able to form tubes on matrigel and uptake acetylated-LDL which indicates their functional capacity as endothelial cells.

Thus, the invention provides a population of cells with increased expression of, as compared to undifferentiated MAPCs, Hey-2, Dll-4, EphrinB1, EphrinB2, and/or EphB4.

In one embodiment, the vascular endothelial cells are arterial endothelial cells. In another embodiment, the vascular endothelial cells are venous endothelial cells.

Generally, arterial cells express a transmembrane ligand of the Ephrin family of ligands, such as EphrinB2, whose receptor, EphB4, is expressed on venous cells (a member of the Eph family of receptors which are generally expressed on venous cells).

Additionally, the present invention provides a method for making lymphatic endothelial cells obtained from a population of cells enriched in MAPCs, comprising contacting the non-ES, non-EG, non-germ cells with VEGF₁₆₅, VEGF-C or a combination thereof, so that the MAPCs differentiate into lymphatic vascular endothelial cells. The lymphatic cells express podoplanin, lvye-1, and/or prox-1. Preferably, the cells are mammalian cells, including human cells.

Methods for culturing and preparing vascular endothelial cells derived from a population of cells enriched in MAPCs, comprising contacting the MAPCs with a vascular endothelial growth factor (VEGF), such as VEGF₁₆₅ or VEGF-C, and optionally with one or more notch ligands, patched ligands or a combination thereof, so that the MAPCs differentiate into vascular endothelial cells.

In vascular endothelial cells contacted with a VEGF, and one or more notch ligands, patch ligands or a combination thereof, Hey-2, EphrinB1 and EphrinB2 expression is increased and EphB4 expression is decreased when compared to endothelial cells derived from said non-ES, non-EG, non-germ cells which have not been contacted with a notch and/or patched ligand. This expression pattern shows that the vascular endothelial cells of the invention have an increased potential for terminally differentiating into arterial endothelial cells.

### Uses for Vascular Endothelial Cells of the Invention

The vascular endothelial cells of the invention and/or the MAPCs can be used to repopulate blood vessels (re-endothelialization or re-vascularization) or to create new ones by either direct introduction into the area of damage or by systemic administration, that allows the cells to home to the area of damage. Accordingly, the invention provides methods of treating a subject in need of vascular endothelial cells, treating a blood vessel, increasing vasculogenesis and/or angiogenesis comprising administering to a subject an effective amount of the vascular endothelial cells of the invention or MAPCs.

For the purposes described herein, either autologous, allogeneic or xenogeneic cells can be administered to a patient, either in undifferentiated, terminally differentiated or in a partially differentiated form, genetically altered or unaltered, by direct introduction to a site of interest, e.g., on or around the surface of an acceptable matrix ,or systemically, in combination with a pharmaceutically acceptable carrier so as to repair, replace or promote the growth of existing and/or new blood vessels.

Generally, the invention provides methods to treat a vascular condition, such as a condition associated with loss, injury or disruption of the vasculature within an anatomical site or system. The term "vascular condition" or "vascular disease" refers to a state of vascular tissue where blood flow has become impaired. Many pathological conditions can lead to vascular diseases that are associated with alterations in the normal vascular condition of the affected tissues and/or systems. Examples of vascular conditions or vascular diseases to which the methods of the invention apply are those in which the vasculature of the affected tissue or system is senescent or otherwise altered in some way such that blood flow to the tissue or system is reduced or in danger of being reduced. Examples of vascular conditions that can be treated with the compositions and methods of the invention include atherosclerosis, preeclampsia, peripheral vascular disease, erectile dysfunction, renal failure, heart disease, and stroke. Vascular, circulatory or hypoxic conditions to which the methods of the invention apply also include those associated with but not limited to maternal hypoxia (*e.g.*, placental hypoxia, preeclampsia), abnormal pregnancy, peripheral vascular disease (*e.g.*, arteriosclerosis), transplant accelerated arteriosclerosis, deep vein thrombosis, erectile dysfunction, renal failure, stroke, heart disease, sleep apnea, hypoxia during sleep, female sexual dysfunction, fetal hypoxia, smoking, anemia, hypertension, diabetes, vasculopathologies, surgery, endothelial dysfunction, regional perfusion deficits (*e.g.*, limb, gut, renal ischemia), myocardial infarction, stroke, thrombosis, frost bite, decubitus ulcers, asphyxiation, poisoning (*e.g.*, carbon monoxide, heavy metal), altitude sickness, pulmonary hypertension, sudden infant death syndrome (SIDS), asthma, chronic obstructive pulmonary disease (COPD), congenital circulatory abnormalities (*e.g.*, Tetralogy of Fallot) and Erythroblastosis (blue baby syndrome). Additionally, the cells can be used with organ transplants, vascular grafts or valves to enhance vascularization.

Thus, the invention is directed to methods of treating conditions or diseases such as ischemia, congestive heart failure, peripheral vasculature disorder, coronary vascular disease, hypertension, stroke, aneurysm, acute coronary syndromes including unstable angina, thrombosis and myocardial infarction, plaque rupture, both primary and secondary (in-stent) restenosis in coronary or peripheral arteries, transplantation-induced sclerosis, peripheral limb disease, diabetic complications (including ischemic heart disease, peripheral artery disease, congestive heart failure, retinopathy, neuropathy and nephropathy), thrombosis, arrhythmia, tachycardia, or surgical or physical trauma.

For example, the vascular condition or vascular disease may arise from damaged myocardium. As used herein "damaged myocardium" refers to myocardial cells that have been exposed to ischemic conditions, including those conditions created by disease and during surgical procedures or other trauma. These ischemic conditions may be caused by a myocardial infarction, or other cardiovascular disease. The lack of oxygen causes the death of the cells in the surrounding area, leaving an infarct that can eventually scar.

To treat damaged tissue, including damaged myocardium, the vascular endothelial cells of the invention or the MAPCs may be introduced into ischemic tissue in the heart or other muscle, where the cells can organize into tubules that will anastomose with existing cardiac vasculature to provide a blood supply to the diseased tissue. Other tissues may be vascularized or re-vascularized in the same manner. The cells may incorporate into neovascularization sites in the ischemic tissue and accelerate vascular development and anastomosis. It is intended that the invention be used to vascularize all sorts of tissues, including connective tissue, muscle tissue, nerve tissue, and organ tissue.

Additionally, the vascular endothelial cells of the invention or the MAPCs may also be used to help restore or repair cardiac vasculature following angioplasty. For example, a catheter can be used to deliver vascular endothelial cells to the surface of a blood vessel following angioplasty or before insertion of a stent. Alternatively, the stent may be seeded or infused with cells. In another embodiment, cells may be seeded into a polymeric sheet and wrapped around the outside of a blood vessel that has undergone angioplasty or stent insertion (Nugent, et al., 2001).

The cells can also be used in artificial vessel applications. For example, the cells may be seeded onto a tubular substrate. For example, a polymer matrix may be formed into a tube or network. Such tubes may be formed of natural or synthetic ECM materials or may come from natural sources, for example, decellularized tubular grafts. The cells can coat the inside or outside of the tube, forming an artificial channel (*e.g.*, artificial vessel) that can be used, for example, in heart bypass. In addition, use of the endothelial cells or MAPCs of the invention may reduce thrombosis post-implantation.

### Administration of Vascular Endothelial Cells or MAPCs of the Invention

For the purposes described herein, either autologous, allogeneic or xenogeneic MAPCs, or their differentiated progeny, can be administered to a subject, either in differentiated or undifferentiated form, genetically altered or unaltered, by direct injection to a tissue site, systemically, on a surface, on or around the surface of an acceptable matrix, encapsulated or in combination with a pharmaceutically acceptable carrier.

MAPCs, or their differentiated progeny, can be administered to a subject by a variety of methods available to the art, including but not limited to localized injection, catheter administration, systemic injection, intraperitoneal injection, parenteral administration, oral administration, intracranial injection, intra-arterial injection, intravenous injection, intraventricular infusion, intraplacental injection, intrauterine injection, surgical intramyocardial injection, transendocardial injection, transvascular injection, intracoronary injection, transvascular injection, intramuscular injection, surgical injection into a tissue of interest or via direct application to tissue surfaces (e.g., during surgery or on a wound).

MAPCs can be administered either peripherally or locally through the circulatory system. "Homing" of stem cells would concentrate the implanted cells in an environment favorable to their growth and function. Pre-treatment of a patient with cytokine(s) to promote homing is another alternative contemplated in the methods of the present invention. Certain cytokines (*e.g.*, cellular factors that induce or enhance cellular movement, such as homing of MAPCs or other stem cells, progenitor cells or differentiated cells) can enhance the migration of MAPCs or their progeny. Cytokines include, but are not limited to, stromal cell derived factor-1 (SDF-1), stem cell factor (SCF), angiopoietin-1, placenta-derived growth factor (PIGF) and granulocyte-colony stimulating factor (G-CSF). Cytokines also include any which promote the expression of endothelial adhesion molecules, such as ICAMs, VCAMs and others, which facilitate the homing process.

Factors promoting angiogenesis, including but not limited to, VEGF, aFGF, angiogenin, angiotensin-1 and -2, betacellulin, bFGF, Factor X and Xa, HB-EGF, PDGF, angiomodulin, angiotropin, angiopoetin-1, prostaglandin E1 and E2, steroids, heparin, 1-butyryl-glycerol and nicotinic amide, can also be used.

Factors that decrease apoptosis can also promote the formation of new vasculature. Factors that decrease apoptosis include but are not limited to β-blockers, angiotensin-converting enzyme inhibitors (ACE inhibitors), AKT, HIF, carvedilol, angiotensin II type 1 receptor antagonists, caspase inhibitors, cariporide and eniporide.

Exogenous factors (*e.g.*, cytokines, differentiation factors (*e.g.*, cellular factors, such as growth factors or angiogenic factors that induce lineage commitment), angiogenesis factors and anti-apoptosis factors) can be administered prior to, after or concomitantly with MAPCs or their differentiated progeny. For example, a form of concomitant administration would comprise combining a factor of interest in the MAPC suspension media prior to administration. Administrations are variable and may include an initial administration followed by subsequent administrations.

A method to potentially increase cell survival is to incorporate MAPCs or progeny into a biopolymer or synthetic polymer. Depending on the patient's condition, the site of injection might prove inhospitable for cell seeding and growth because of scarring or other impediments. Examples of biopolymer include, but are not limited to, fibronectin, fibrin, fibrinogen, thrombin, collagen and proteoglycans. This could be constructed with or without included cytokines, differentiation factors, angiogenesis factors or anti-apoptosis factors. Additionally, these could be in suspension. Another alternative is a three-dimensional gel with cells entrapped within the interstices of the cell biopolymer admixture. Again cytokines, differentiation factors, angiogenesis factors, anti-apoptosis factors or a combination thereof could be included within the gel. These could be deployed by injection via various routes described herein.

The cells could also be encapsulated with a capsule that is permeable to nutrients and oxygen while allowing appropriate cellular products to be released into the bloodstream or to adjacent tissues. In one embodiment, the capsular material is restrictive enough to exclude immune cells and antibodies that could reject and destroy the implant. Such encapsulation can be achieved using, for example, polymers (Chang, 2000). Such polymeric encapsulation systems include, but are not limited to, alginate (e.g., alginate bead), polysaccharide hydrogels, chitosan, calcium or barium alginate, a layered matrix of alginate and polylysine, a photopolymerizable poly(ethylene glycol) (PEG) polymer (Novocell, Inc.), a polyanionic material termed Biodritin (US Patent 6,281,341), polyacrylates, a photopolymerizable poly(ethylene glycol) polymer, and polymers such as hydroxyethyl methacrylate methyl methacrylate.

Another approach to encapsulate cells involves the use of photolithography techniques adapted from the semiconductor industry to encapsulate living cells in silicon capsules that have pores only a few nanometers wide (Desai 2002).

Also, suitable immune-compatible polycations, including but not limited to, poly-1-lysine (PLL) polycation or poly-1-ornithine or poly(methylene-co-guanidine) hydrochloride, may be used to encapsulate cells.

Additionally, cells can be encapsulated with biocompatible semipermeable membranes to surround encapsulated cells, sometimes within a capillary device, to create a miniature artificial organ. This is often called macroencapsulation. The membrane lets various agents pass in and out of the blood stream, and preferably keeps out the antibodies and T cells of the immune system, which may destroy the cells. Such membranes can be used in a perfusion device, a capsule that is grafted to an artery where it makes direct contact with the body's circulating blood; in this way, the device can draw nutrients from the blood and release factors to circulate throughout the body.

The quantity of cells to be administered will vary for the subject being treated. In a preferred embodiment, between about 10⁴ to about 10⁸, more preferably about 10⁵ to about 10⁷ and most preferably, about 3 x 10⁷ stem cells and optionally, about 50 to about 500 µg/kg per day of a cytokine can be administered to a human subject. However, the precise determination of what would be considered an effective dose may be based on factors individual to each patient, including their size, age, disease or injury, amount of damage, amount of time since the damage occurred and factors associated with the mode of delivery (direct injection-lower doses, intravenous - higher doses). Dosages can be readily ascertained by those skilled in the art from this disclosure and the knowledge in the art.

An issue regarding the use of stem cells or their progeny is the purity of the enriched or isolated cell population. Bone marrow cells, for example, comprise mixed populations of cells, which can be purified to a degree sufficient to produce a desired effect. Those skilled in the art can readily determine the percentage of MAPCs or progeny in a population using various well-known methods, such as fluorescence activated cell sorting (FACS). Preferable ranges of purity in populations comprising MAPCs, or their differentiated progeny, are about 50-55%, about 55-60%, and about 65-70%. More preferably the purity is about 70-75%, about 75-80%, about 80-85%; and most preferably the purity is about 85-90%, about 90-95%, and about 95-100%. However, populations with lower purity can also be useful, such as about 25-30%, about 30-35%, about 35-40%, about 40-45% and about 45-50%. Purity of MAPCs or their progeny can be determined according to the gene expression profile within a population. Dosages can be readily adjusted by those skilled in the art (*e.g.*, a decrease in purity may require an increase in dosage).

The skilled artisan can readily determine the amount of cells and optional additives, vehicles, or carrier in compositions to be administered in methods of the invention. Typically, additives (in addition to the active stem cell(s) or cytokine(s)) are present in an amount of about 0.001 to about 50 wt % solution in phosphate buffered saline, and the active ingredient is present in the order of micrograms to milligrams, such as about 0.0001 to about 5 wt %, preferably about 0.0001 to about 1 wt %, most preferably about 0.0001 to about 0.05 wt % or about 0.001 to about 20 wt %, preferably about 0.01 to about 10 wt %, and most preferably about 0.05 to about 5 wt %. Of course, for any composition to be administered to an animal or human, and for any particular method of administration, it is preferred to determine therefore: toxicity, such as by determining the lethal dose (LD) and LD₅₀ in a suitable animal model e.g., a rodent, such as mouse; and, the dosage of the composition(s), concentration of components therein and timing of administering the composition(s), which elicit a suitable response. Such determinations do not require undue experimentation from the knowledge of the skilled artisan, this disclosure and the documents cited herein. Additionally, the time for sequential administrations can be ascertained without undue experimentation.

When administering a therapeutic composition of the present invention, it will generally be formulated in a unit dosage injectable form (solution, suspension, emulsion). The pharmaceutical formulations suitable for injection include sterile aqueous solutions and dispersions. The carrier can be a solvent or dispersing medium containing, for example, water, saline, phosphate buffered saline, polyol (for example, glycerol, propylene glycol, liquid polyethylene glycol, and the like) and suitable mixtures thereof.

Additionally, various additives which enhance the stability, sterility, and isotonicity of the compositions, including antimicrobial preservatives, antioxidants, chelating agents and buffers, can be added. Prevention of the action of microorganisms can be ensured by various antibacterial and antifungal agents, for example, parabens, chlorobutanol, phenol, sorbic acid, and the like. In many cases, it will be desirable to include isotonic agents, for example, sugars, sodium chloride, and the like. Prolonged absorption of the injectable pharmaceutical form can be brought about by the use of agents delaying absorption, for example, aluminum monostearate and gelatin. Sterile injectable solutions can be prepared by incorporating the cells utilized in practicing the present invention in the required amount of the appropriate solvent with various amounts of the other ingredients, as desired.

In one embodiment, MAPCs, or differentiated progeny thereof, can be administered initially, and thereafter maintained by further administration of MAPCs or differentiated progeny thereof. For instance, MAPCs can be administered by one method of injection, and thereafter further administered by a different or the same type of method.

It is noted that human subjects are treated generally longer than canines or other experimental animals, such that treatment has a length proportional to the length of the disease process and effectiveness. The doses may be single doses or multiple doses over a period of several days. Thus, one of skill in the art can scale up from animal experiments, *e.g.*, rats, mice, canines and the like, to humans, by techniques from this disclosure and documents cited herein and the knowledge in the art, without undue experimentation. The treatment generally has a length proportional to the length of the disease process and drug effectiveness and the subject being treated.

Examples of compositions comprising MAPCs, or differentiated progeny thereof, include liquid preparations for administration, including suspensions, and, preparations for direct or intravenous administration (*e.g.*, injectable administration), such as sterile suspensions or emulsions. Such compositions may be in admixture with a suitable carrier, diluent, or excipient such as sterile water, physiological saline, glucose, dextrose, or the like. The compositions can also be lyophilized. The compositions can contain auxiliary substances such as wetting or emulsifying agents, pH buffering agents, gelling or viscosity enhancing additives, preservatives, flavoring agents, colors, and the like, depending upon the route of administration and the preparation desired. Standard texts, such as "REMINGTON'S PHARMACEUTICAL SCIENCE," 17th edition, 1985, incorporated herein by reference, may be consulted to prepare suitable preparations, without undue experimentation.

Compositions are conveniently provided as liquid preparations, *e.g.*, isotonic aqueous solutions, suspensions, emulsions or viscous compositions, which may be buffered to a selected pH. Liquid preparations are normally easier to prepare than gels, other viscous compositions and solid compositions. Additionally, liquid compositions are somewhat more convenient to administer, especially by injection. Viscous compositions, on the other hand, can be formulated within the appropriate viscosity range to provide longer contact periods with specific tissues.

The choice of suitable carriers and other additives will depend on the exact route of administration and the nature of the particular dosage form, *e.g.*, liquid dosage form (*e.g.*, whether the composition is to be formulated into a solution, a suspension, gel or another liquid form, such as a time release form or liquid-filled form).

Solutions, suspensions and gels normally contain a major amount of water (preferably purified, sterilized water) in addition to the cells. Minor amounts of other ingredients such as pH adjusters (*e.g.*, a base such as NaOH), emulsifiers or dispersing agents, buffering agents, preservatives, wetting agents and jelling agents (*e.g.*, methylcellulose), may also be present. The compositions can be isotonic, *i.e.*, they can have the same osmotic pressure as blood and lacrimal fluid.

The desired isotonicity of the compositions of this invention may be accomplished using sodium chloride, or other pharmaceutically acceptable agents such as dextrose, boric acid, sodium tartrate, propylene glycol or other inorganic or organic solutes. Sodium chloride is preferred particularly for buffers containing sodium ions.

Viscosity of the compositions, if desired, can be maintained at the selected level using a pharmaceutically acceptable thickening agent. Methylcellulose is preferred because it is readily and economically available and is easy to work with. Other suitable thickening agents include, for example, xanthan gum, carboxymethyl cellulose, hydroxypropyl cellulose, carbomer, and the like. The preferred concentration of the thickener will depend upon the agent selected and the desired viscosity. Viscous compositions are normally prepared from solutions by the addition of such thickening agents.

A pharmaceutically acceptable preservative or cell stabilizer can be employed to increase the life of the compositions. Preferably, if preservatives are necessary, it is well within the purview of the skilled artisan to select compositions that will not affect the viability or efficacy of the MAPCs or progeny as described in the present invention.

Those skilled in the art will recognize that the components of the compositions should be selected to be chemically inert. This will present no problem to those skilled in chemical and pharmaceutical principles, or problems can be readily avoided by reference to standard texts or simple experiments (not involving undue experimentation), from this disclosure and the documents cited herein.

Compositions can be administered in dosages and by techniques available to those skilled in the medical and veterinary arts taking into consideration such factors as the age, sex, weight and condition of the particular patient, and the composition form used for administration (e.g., solid vs. liquid). Dosages for humans or other animals can be determined without undue experimentation by the skilled artisan, from this disclosure, the documents cited herein, and the knowledge in the art.

Suitable regimes for initial administration and further doses or for sequential administrations also are variable, may include an initial administration followed by subsequent administrations; but nonetheless, can be ascertained by the skilled artisan, from this disclosure, the documents cited herein, and the knowledge in the art.

### Approaches for Transplantation to Prevent Immune Rejection

In some embodiments, it may be desired that the MAPCs (or differentiated progeny) be treated or otherwise altered prior to transplantation/administration in order to reduce the risk of stimulating host immunological response against the transplanted cells. Any method known in the art to reduce the risk of stimulating host immunological response may be employed. The following provides a few such examples.

### 1. Universal donor cells:

MAPCs can be manipulated to serve as universal donor cells. Although undifferentiated MAPCs do not express MHC-I or -II antigens, some differentiated progeny may express one or both of these antigens. MAPCs can be modified to serve as universal donor cells by eliminating MHC-I or MHC-II antigens, and potentially introducing the MHC-antigens from the prospective recipient so that the cells do not become easy targets for NK-mediated killing, or become susceptible to unlimited viral replication or malignant transformation. Elimination of MHC-antigens can be accomplished, for example, by homologous recombination or by introduction of point-mutations in the promoter region or by introduction of a point mutation in the initial exon of the antigen to introduce a stop-codon, such as with chimeroplasts. Transfer of the host MHC-antigen(s) can be achieved by retroviral, lentiviral, adeno associated virus or other viral transduction or by transfection of the target cells with the MHC-antigen cDNAs.

### 2. Intrauterine transplant to circumvent immune recognition:

MAPCs can be used in an intrauterine transplantation setting to correct genetic abnormalities, or to introduce cells that will be tolerated by the host prior to immune system development. This can be a way to make human cells in large quantities in animals or it could be used as a way to correct human embryo genetic defects by transplanting cells that make the correct protein or enzyme.

### 3. Hematopoietic Chimerism and Tolerance Induction

Benefit would be achieved through use of a stem cell, capable of reconstituting the immune system, that did not carry risk of graft-versus-host response. The graft-versus-host reaction is due to contaminating T cells inherent in the bone marrow graft. Although purification of hematopoietic stem cells from bone marrow is routine, their successful engraftment in the patient requires accompaniment by accessory T cells. Thus, a balance must be achieved between the beneficial engraftment value of T cells and the detrimental effect of graft-versus-host response.

MAPCs and ES cells represent a stem cell population which can be delivered without risk of graft-versus-host reactivity, as they can be expanded free of hematopoietic cell types, including T cells. This greatly reduces clinical risk. The transient elimination of NK cell activity during the acute phase of cell delivery increases the frequency of primitive stem cell engraftment and hematopoietic reconstitution to a clinically useful threshold without risk of long term immunosuppression.

As MAPC or ES engraft and contribute to hematopoiesis, the newly formed T cells undergo thymic and peripheral self versus non-self education consistent with host T cells as described above. Co-exposure of newly created naïve T cells of donor and host origin results reciprocal depletion of reactive cells, hence tolerance to T cells expressing allogeneic antigens derived from a MAPC or ES donor can be achieved. A patient can thus be rendered tolerant to the cellular and molecular components of the MAPC or ES donor immune system, and would accept a cell, tissue or organ graft without rejection.

### 4. Natural Killer (NK) Cell Function:

Any means, such as an agent, which inhibits NK cell function, including depleting NK cells from a population of cells, may also be administered to prevent immune rejection, increase engraftment or increase immune tolerance. Such an agent includes an anti-NK cell antibody, irradiation or any other method which can inhibit NK cell function. NK function inhibition is further described in PCT Application No. PCT/US2005/015740, filed May 5, 2005, which application is incorporated herein by reference for teaching methods of inhibiting NK cells to aid in stem cell persistence *in vivo.*

In one embodiment of the invention at least one means for inhibiting NK cell function, including inhibition of NK cell-mediated cytotoxicity, is administered. NK cell function can be negated by NK depletion using either genetic (recipients deficient in NK cells) or epigenetic (*in vivo* depletion/inactivation with, for example, an anti-NK antibody) means. Any material capable of inhibiting NK cell function can be used (*e.g.*, multimeric compounds that bind to P-Selectin Glycoprotein 1 (PSGL-1) on the surface of T cells or NK cells (U.S. Pat. Pub. No. 2004/0116333) or modulation of SH2-containing inositol phophatase (SHIP) expression or function (U.S. Pat. Pub. No. 2002/0165192)). Any means/agent including, but not limited to, chemical (*e.g.*, a chemical compound, including, but not limited to, a pharmaceutical, drug, small molecule), protein (e.g., anti-NK cell antibody), peptide, microorganism, biologic, nucleic acid (including genes coding for recombinant proteins, or antibodies), or genetic construct (*e.g.*, vectors, such as expression vectors, including but not limited to expression vectors which lead to expression of an antagonist against NK cell activity) can be used to inhibit NK cell function.

There are several antibodies available in the art which inhibit NK cell function, including but not limited to anti-human thymocyte globulin (ATG; U.S. Pat. No. 6,296,846), TM-ß1 (anti-IL-2 receptor ß chain Ab), anti-asialo-GM1 (immunogen is the glycolipid GA1), anti-NK1.1 antibodies or monoclonal anti-NK-cell antibodies (5E6; Pharmingen, Piscataway, NJ). Additionally, antibodies directed against, for example, a natural cytotoxicity receptor (NCR), including, for example, NKp46, or an antibodies directed against a leukocyte-associated Ig like receptor family, including, for example, LAIR-1, or antibodies directed against a member of the killer cell immunoglobulin-like receptor (KIR) family, including, for example, KIR2DL1, KIR2DL2 or KR2DL3 are available to the art worker or can be made by methods available to an art worker and are useful in the present invention.

Additionally, a means, such as an agent which can cross-link LAIR-1 molecules on NK cells may be used to inhibit NK cell function. Also, irradiation (lethal, sublethal, and/or localized or systemic irradiation) may be used to inhibit NK cell function. In one embodiment, the means for inhibiting NK cell function is an antibody which is reactive with Natural Killer cells. Additionally, a means for inhibiting NK cell function can include agents that modulate the immune system, such as those developed for immunosuppression. It should be noted that any of these means/agents can be used alone or in combination.

Thus, there is also provide herein a method to increase immunologic tolerance in a subject to MAPCs and other cells comprising administering a population of the MAPCs and an effective amount of an agent for inhibiting Natural Killer cell function to the subject, so that immunologic tolerance to the MAPCs increases compared to the method without administration of the inhibiting agent.

### 5. Gene Therapy:

MAPCs can be extracted and isolated from the body, grown in culture in the undifferentiated state or induced to differentiate in culture, and genetically altered using a variety of techniques. Uptake and expression of genetic material is demonstrable, and expression of foreign DNA is stable throughout development. Retroviral and other vectors for inserting foreign DNA into stem cells are available to those of skill in the art. (Mochizuki, H. et al. 1998; Robbins, P. et al. 1997; Bierhuizen, M. et al. 1997; Douglas, J. et al. 1999; Zhang, G. et al. 1996). Once transduced using a retroviral vector, enhanced green fluorescent protein (eGFP) expression persists in terminally differentiated muscle cells, endothelium and c-Kit positive cells derived from isolated MAPCs, demonstrating that expression of retroviral vectors introduced into MAPC persists throughout differentiation. Terminal differentiation was induced from cultures initiated with about 10 eGFP⁺ cells previously transduced by retroviral vector and sorted a few weeks into the initial MAPC culture period.

### Monitoring of Subject After Administration of MAPCs or Progeny Therefrom

Following transplantation, the growth or differentiation of the administered MAPCs or progeny or the therapeutic effect of the MAPCs or progeny may be monitored.

Following administration, the immunological tolerance of the subject to the MAPCs or progeny may be tested by various methods known in the art to assess the subject's immunological tolerance to MAPCs. In cases where the subject's tolerance of MAPCs or their differentiated progeny is suboptimal (*e.g.*, the subject's immune system is rejecting the exogenous MAPCs or their progeny), therapeutic adjunct immunosuppressive treatment (*e.g.*, cyclosporine), which is known in the art, of the subject may be performed.

### Genetically-Modified MAPCs and Vascular Endothelial Cells of the Invention

MAPCs or the vascular endothelial cells derived therefrom can be genetically altered *ex vivo,* eliminating one of the most significant barriers for gene therapy. For example, a subject's bone marrow aspirate is obtained, and from the aspirate MAPCs are isolated. The MAPCs are then genetically altered to express one or more desired gene products. The MAPCs can then be screened or selected *ex vivo* to identify those cells which have been successfully altered, and these cells can be introduced into the subject or can be differentiated into the vascular endothelial cells of the invention and introduced into the subject, either locally or systemically. Alternately, MAPCs can be differentiated into vascular endothelial cells of the invention and then the vascular endothelial cells of the invention can be genetically altered prior to administration. In either case, the transplanted cells provide a stably-transfected source of cells that can express a desired gene product. Especially where the patient's own tissue, such as bone marrow, is the source of the MAPCs, this method provides an immunologically safe method for producing cells for transplant.

### Methods for Genetically Altering MAPCs and Vascular Endothelial Cells of the Invention

Cells isolated by the methods described herein, or their differentiated progeny, can be genetically modified by introducing DNA or RNA into the cell by a variety of methods available to those of skill in the art. These methods are generally grouped into four major categories: (1) viral transfer, including the use of DNA or RNA viral vectors, such as retroviruses, including lentiviruses (Mochizuki, H., et al., 1998; Martin, F., et al. 1999; Robbins, et al. 1997; Salmons, B. and Gunzburg, W.H., 1993; Sutton, R., et al., 1998; Kafri, T., et al., 1999; Dull, T., et al., 1998), Simian virus 40 (SV40), adenovirus (see, for example, Davidson, B.L., et al., 1993; Wagner, E., et al., 1992; Wold, W., Adenovirus Methods and Protocols, Humana Methods in Molecular Medicine (1998), Blackwell Science, Ltd.; Molin, M., et al., 1998; Douglas, J., et al., 1999; Hofmann, C., et al., 1999; Schwarzenberger, P., et al., 1997), alpha virus, including Sindbis virus (U.S. Patent No. 5,843,723; Xiong, C., et al., 1989; Bredenbeek, P.J., et al., 1993; Frolov, I., et al., 1996), herpes virus (Laquerre, S., et al., 1998) and bovine papillomavirus, for example; (2) chemical transfer, including calcium phosphate transfection and DEAE dextran transfection methods; (3) membrane fusion transfer, using DNA-loaded membranous vesicles such as liposomes (Loeffler, J. and Behr, J., 1993), red blood cell ghosts and protoplasts, for example; and (4) physical transfer techniques, such as microinjection, microprojectile (see J. Wolff in "Gene Therapeutics" (1994) at page 195; Johnston, S.A., et al., 1993; Williams, R.S., et al., 1991; Yang, N.S., et al., 1990), electroporation, nucleofection or direct "naked" DNA transfer.

Cells can be genetically altered by insertion of pre-selected isolated DNA, by substitution of a segment of the cellular genome with pre-selected isolated DNA, or by deletion of or inactivation of at least a portion of the cellular genome of the cell. Deletion or inactivation of at least a portion of the cellular genome can be accomplished by a variety of means, including but not limited to genetic recombination, by antisense technology (which can include the use of peptide nucleic acids or PNAs), or by ribozyme technology, for example. Insertion of one or more pre-selected DNA sequences can be accomplished by homologous recombination or by viral integration into the host cell genome. Methods of non-homologous recombination are also known, for example, as described in U.S. Patent Nos. 6,623,958, 6,602,686, 6,541,221, 6,524,824, 6,524,818, 6,410,266, 6,361,972, the contents of which are specifically incorporated by reference for their entire disclosure relating to methods of non-homologous recombination.

The desired gene sequence can also be incorporated into the cell, particularly into its nucleus, using a plasmid expression vector and a nuclear localization sequence. Methods for directing polynucleotides to the nucleus have been described in the art. For example, signal peptides can be attached to plasmid DNA, as described by Sebestyen, et al. (1998), to direct the DNA to the nucleus for more efficient expression.

The genetic material can be introduced using promoters that will allow for the gene of interest to be positively or negatively induced using certain chemicals/drugs, to be eliminated following administration of a given drug/chemical, or can be tagged to allow induction by chemicals (including but not limited to the tamoxifen responsive mutated estrogen receptor) in specific cell compartments (including, but not limited to, the cell membrane).

Any of transfection or transduction technique can also be applied to introduce a transcriptional regulatory sequence into MAPCs or progeny to activate a desired endogenous gene. This can be done by both homologous (e.g., U.S. 5,641,670) or non-homologous (e.g., U.S. 6,602,686) recombination. These patents are incorporated by reference for teaching of methods of endogenous gene activation.

Successful transfection or transduction of target cells can be demonstrated using genetic markers, in a technique that is known to those of skill in the art. The green fluorescent protein of *Aequorea victoria,* for example, has been shown to be an effective marker for identifying and tracking genetically modified hematopoietic cells (Persons, D., et al., 1998). Alternative selectable markers include the β-Gal gene, the truncated nerve growth factor receptor, drug selectable markers (including but not limited to NEO, MTX, hygromycin).

### Protein Transduction

Proteins can be transferred directly to cells when they are linked to a protein transduction domain (PTD), small cationic peptide domains that can freely and rapidly cross cell membranes. Several PTDs such as poly-arginine (poly-arginine-mediated protein transduction) and HIV-derived Tat have been identified that allow a fused protein to efficiently cross cell membranes. A distinct advantage of protein transduction is that the transduced proteins are present in the cells only transiently, a feature which depends on the intrinsic turnover of the expressed protein. In addition, intracellular concentration of the transduced protein can be controlled by varying the amount of protein added.

The following examples are provided in order to demonstrate and further illustrate certain embodiments and aspects of the present invention and are not to be construed as limiting the scope thereof.

### Examples

The potential of human AC 133 positive cells and human bone marrow derived Multipotent Adult Progenitor Cells (hMAPC) to differentiate to arterial, venous or lymphatic endothelium, as was the role of cytokines implicated in arterial-venous and lymphatic decisions, was examined.

### Material and Methods for the Examples

### A. Cell Populations

All samples were obtained after informed consent had been obtained from the donor or the mother according to the guidelines from the Committee on the Use of Human Subjects in Research from the Clinica Universitaria, Pamplona, Spain. hAC133⁺ cells: BM and UCB mononuclear cells were separated by Ficoll Hypaque centrifugation (specific gravity, 1077) (Sigma, St. Louis, USA) and hAC133⁺ cells were selected using the autoMACS (Miltenyi Biotec, Germany) with the AC133 Isolation Kit (Miltenyi Biotec) as described (de Wynter et al., (1998)). AC133⁺ purity was greater than 90% in all samples as determined by flow cytometry. New hMAPC cultures were established and characterized as described herein and previously (Jiang et al., 2003; Jiang et al., 2002; Schwartz et al., 20021; Reyes et al., 2002; Reyes et al., 2001), using BMMNCs depleted of CD45⁺ and glycophorin-A⁺ cells by means of micromagnetic beads (Miltenyi Biotec) or directly after Ficoll-Hypaque.

### B. Human MAPC (hMAPC) cultures

hMAPC cultures were established as previously described in Reyes, 2001 and 2002, and these methods are incorporated by reference herein. Briefly, CD45⁻GlyA⁻ cells or BMMNCs were plated at a concentration of 2 x 10⁵ cells/cm² in expansion medium supplemented with 10 ng/mL EGF (Sigma) and 10 ng/mL PDGF-BB (R&D Systems) in wells coated with 20 ng/mL fibronectin (FN) (Sigma). Expansion medium consisted of 58% low-glucose DMEM (Gibco BRL), 40% MCDB-201 (Sigma), 2% fetal calf serum (FCS) (Biochrom), 1X insulin transferrin selenium, 1X linoleic acid bovine serum albumin (BSA) (Sigma), 10⁻⁸ M dexamethasone (Sigma), and 10⁻⁴ M ascorbic acid 2-phosphate (Sigma), 100 U penicillin, and 1000 U streptomycin (Gibco). Once adherent cells were more than 50% confluent, they were detached with 0.25% trypsin-EDTA (Biowithaker) and replated at a concentration of 2-5 x 10² under the same culture conditions. Cells were maintained at the same confluence throughout the life of the culture.

The MAPC cell lines were maintained for more than 50 to 80 population doublings (PDs). The phenotype of the majority of cells within these cultures was CD90⁺, CD13⁺, CD44^{low}, MCH-I⁻, αᵥβ₃⁻, CD73⁻, CD105⁻, MHC-II⁻, CD36⁻, CD45⁻ and CD34⁻ (Fig. 1a), consistent with previous publications (Reyes et al., 2001) (Fig. 1a and data not shown). RT-PCR demonstrated presence of the transcription factors Oct3/4, Rex-1 and nanog, as well as hTERT (Fig. 1b) and cells stained positive for SSEA-4, nanog, and Oct3/4, but not SSEA-1 proteins (Fig. 1c-f).

hMAPCs were isolated, cultured and differentiated *in vitro* at 20% O₂.

### C. In vitro differentiation of hMAPCs

The cells were further qualified by showing differentiation into mesodermal (ECs; Fig. 2j-r and Fig. 3; and SMCs Fig. 1g), endodermal (hepatocytes; Fig. 1h) and ectodermal (neurons; Fig. 1i) cells types.

### 1. Mesodermal differentiation

**Skeletal muscle differentiation:** hMAPCs were plated at 40 x 10³ cells/cm² in expansion media with 5*µ*M of 5-Azacytidine for 24 hours after which cells were kept in expansion media for 2 weeks.

**Smooth muscle differentiation:** hMAPCs were plated at 1 x 10³ cells/cm² in growth media (58% low-glucose DMEM (Gibco BRL), 40% MCDB-201 (Sigma), 2% fetal calf serum (FCS) (Biochrom), ITS+1 (Sigma), 10⁻⁸ M dexamethasone (Sigma), 10⁻⁸ M ascorbic acid 2-phosphate (Sigma), 100 U penicillin, 1000 U streptomycin (Gibco BRL) and 10 ng/mL each of PDGF-BB and EGF during 24 hours and then media was exchanged with the same basal media, without FCS, EGF but now with 10 ng/ml TGF-*β*1. Media was changed every 4-5 days.

Alternatively, 40 x 10³ cells/cm² hMAPCs were plated in differentiation media (expansion media lacking PDGF, EGF or FCS) with 20 ng/ml of PDGF-BB for 2 weeks.

**Chondrocyte differentiation:** 250 x 10³ hMAPCs were spun down at 400 g into a 15 ml Polystyrene Conical Tube and, without disrupting the pellet, 300 *µ*l of chondrocyte differentiation media was added and replaced every 4 days. Chondrocyte differentiation media was prepared with high glucose DMEM, 10 ng/ml TGF*β*-3, 50 mg/ml ITS+ premix, 40 µg/ml proline, 500 ng/ml BMP6, 50 µg/ml ascorbate-2-phospate, 0.1 µM dexamethasone and 1% P/S.

**Osteoblast differentiation:** Osteoblast differentiation media (alpha MEM supplemented with 10 mM *β*-glicerophosphate, 0.2 mM ascorbate-2-phosphate, 0.1 µM dexamethasone, 10 % FBS and 1% P/S.) was added to hMAPCs plated at 20 x 10³ cells/cm² and replaced every 4 days for 21 days.

**Adipocyte differentiation:** hMAPCs were plated at 20 x 10³ cells/cm² for 21 days in adipocyte differentiation media (alpha MEM supplemented with 50 µM indomethacin, 0.5 mM methyl-isobutylxanthine -IBMX, 1 µM dexamethasone, 10 % FBS and 1% P/S), media was exchanged every 4 days.

### 2. Endoderm differentiation

**Hepatocyte differentiation:** hMAPCs were plated at 30-40 x 10³ cells/cm² on 1% matrigel coated plates in differentiation media with 50 ng/ml FGF-4 and 50 ng/ml HGF. Media was changed every 4-5 days.

### 3. Ectoderm differentiation

**Neuronal differentiation:** hMAPCs were plated at 10-15 x 10³ cells/cm² in growth media (58% low-glucose DMEM (Gibco BRL), 40% MCDB-201 (Sigma), 2% fetal calf serum (FCS) (Biochrom), ITS+1 (Sigma), 10⁻⁸ M dexamethasone (Sigma), 10⁻⁸ M ascorbic acid 2-phosphate (Sigma), 100 U penicillin, 1000 U streptomycin (Gibco BRL) and 10 ng/mL each of PDGF-BB and EGF during 24 hours and then media was exchanged with the same basal media, without FCS, EGF and PDGF-BB but now with 100 ng/ml bFGF. Media was changed every 4-5 days. After one week, 100 ng/ml Shh and 50 ng/ml FGF8 were added to the differentiation media, and 50 ng/ml BDNF was added during the third week.

### 4. Endothelial differentiation of hAC133⁺ and hMAPCs

To induce differentiation of hAC133⁺ cells into endothelial cells, 1 x 10⁵ hAC133⁺ cells/cm² were plated on fibronectin coated flasks or wells (50 mg/ml) in IMDM (Gibco BRL) with 20% fetal calf serum (FCS; Gibco BRL) supplemented with 50 ng/ml VEGF₁₆₅ (R&DSystems, Minneapolis, MN) and 10 ng/ml bFGF (Sigma, St. Louis, MO) and 1% Penicillin/Streptomycin (P/S). Cultures were maintained by media exchange every 4-5 days.

Endothelial differentiation of hMAPCs was performed using expansion media without serum, EGF and PDGF-BB and supplemented with cytokines (differentiation media): hMAPCs were plated at 30-40 x 10³ cells/cm² with growth media (58% low-glucose DMEM (Gibco BRL), 40% MCDB-201 (Sigma), 2% fetal calf serum (FCS) (Biochrom), ITS+1 (Sigma), 10⁻⁸ M dexamethasone (Sigma), 10⁻⁸ M ascorbic acid 2-phosphate (Sigma), 100 U penicillin, 1000 U streptomycin (Gibco BRL) and 10 ng/mL each of PDGF-BB and EGF for 24 hours and then media was exchanged with the same basal media, without FCS, EGF and PDGF-BB, but now with 100 ng/ml VEGF₁₆₅. Media was changed every 4-5 days.

Arterial and/or venous differentiation was induced by addition of different combinations of the following growth factors: VEGF₁₂₁ was used at 100 ng/ml and VEGF₁₆₅ in different combinations with Delta-like 4 (D11-4), Jagged-1, or Shh (all from R&DSystems) all at 100 ng/ml. Lymphatic differentiation was induced by addition of 100 ng/ml VEGF-C (R&DSystems) and 20 ng/ml bFGF (Sigma). Except for VEGF121, which was added alone, the other factors were added at the same time as VEGF165 in the various described combinations.

### D. FACS analysis

For fluorescence-activated cell sorting analysis (FACS) cells were detached with 0.25 % trypsin-EDTA and washed with PBS. The following antibodies were used: CD31-PE, CD34-APC, αᵥβ₃-PE, CD73-PE, CD45-PerCP, CD90-APC, HLA-DR,DP,DQ-PE, HLA-A,B,C-PE, CD44-PE, CD13-PE, CD36-FITC (all from BD Pharmingen), CD105-PE (Ancell), and CD133/1-PE and APC (Miltenyi-Biotec), and their corresponding isotype controls (all from BD Pharmingen). Between 50,000 and 200,000 cells were incubated with primary antibody directly coupled to FITC, PerCP, APC or phycoerythrin (PE) for 15 minutes in the dark at room temperature. Cells were fixed with 4% paraformaldehyde at 4°C. Syto was used to determine cell viability when necessary.

The appropriate negative immunoglobulin controls were used. Data acquisition was performed with the CellQuest software. Forward Scatter was collected on log scale and Side Scatter on linear scale. The threshold was set on FL1. The Paint-α-Gate software was used for data analysis.

### E. Immunofluorescent and histochemistry staining

Antibodies against α-smooth muscle actin (α-SMC; DakoCytomation and Cy3 or FITC conjugated; Sigma), SSEA-1 (Chemicon), SSEA-4 (Chemicon), Oct3/4 (Santa Cruz), Nanog (R&DSystems), VEGFR-1 Flt (Santa Cruz), KDR (Santa Cruz), Tie-1 (Santa Cruz), Tie-2 (Santa Cruz), CD31 (Dako or Pharmingen), VE-Cadherin (Chemicon), HLA Class I (Santa Cruz), von Willebrand Factor (vWF; Santa Cruz), EphB4 (Santa Cruz) Hey-2 (Chemicon) EphrinB1 (Zymed), Fli-1 (BD), UEA lectin (biotin, TRITC or FITC conjugated; Sigma), BS-I lectin (Sigma), PCNA (Santa Cruz), albumin (Dako), NF200 (Santa Cruz) were used as primary antibodies. Secondary antibodies coupled to FITC or PE were from Molecular Probes.

For immunofluorescent staining of intracellular molecules, cells were fixed with 4% paraformaldehyde at 20°C for 15 minutes and permeabilized with 0.1% triton X-100 for 10 minutes. For cell surface receptors, cells were fixed with 4% paraformaldehyde at 20°C for 10 minutes. Blocking solution consisted of phosphate-buffered saline (PBS), 1 % BSA, and 10% donkey serum. Primary antibodies were diluted in blocking solution and cultured overnight. After incubation non-specific binding was washed with a solution of PBS and 0.1% tween 20. Secondary antibody was used at dilution 1:1000 in PBS for 1 hour at 4°. Non-adherent antibody was washed with PBS and 0.1% tween 20, after which cells were mounted using DAPI (Vector Laboratories) or Topro as nuclear marker. For controls, cells were label with unspecific immunoglobulins (Santa Cruz) follow by incubation with the secondary antibody. For immunohistochemistry staining Envision system (DAKO) and ABC (Vector Labs) were used. Sirius Red (Luttun et al., 2002) and orcein (Salvato, 2001) staining were performed as described. To quantify the percentage of cultured cells expressing arterial or venous markers, the number of positive cells in 20 randomly selected fields were scored and divided by the total number of cells.

### F. RNA isolation and real time quantitative RT-PCR

Total RNA was obtained using the Rneasy Mini Kit (Qiagen) extraction kit according to the manufacturer's instructions. The first-strand cDNA was synthesized using random primers and MMLV reverse transcriptase. For PCR amplification, Taq (Roche) was used in a 25 µl reaction mixture, including 0.2 mM dNTPs (Invitrogen) and 0.8 mM of each primer (Sigma). PCR program parameters were: 10 minutes initial denaturation at 94°, followed by 30-35 cycles of 94° 1 minute, annealing 1 minute and elongation at 72° 1 minute, followed by 7 minutes of extension at 72°. Subsequently, PCR products were visualized in 1.5% ethidium bromide-stained agarose gels.

For real time RT-PCR Syber Green and Taq Man® methods were used as described (Hong et al., 2004). GAPDH was used as a housekeeping control. Results are presented as fold increase in comparison with the expression of the gene in undifferentiated cells or as percentage of positive control (cord blood derived venous and arterial ECs) (Jaffe et al., 1973; Neuhaus et al., 2003). Primers used for both RT-PCR and real time PCR are shown below in Table 2.

### G. Ac-LDL-DiI uptake

To analyze the uptake of acetylated LDL, cells were washed and 10 µg/ml Ac-LDL-DiI (Biomedical Technologies) was added in IMDM or MAPC differentiation medium (see above). Cells were incubated for 2 hours at 37°C and then washed, fixed and viewed under a fluorescent microscope using DAPI as nuclear marker.

### H. In vitro vascular tube formation

For vascular tube formation, 1 mm cold (4°C) matrigel (BD Bioscience) was added to a culture flask and incubated for 30 minutes at 37° for gelification. After gelification, 30-50x10³ cells differentiated for 14 days were plated in differentiation media on matrigel. After 24-48 hours, tube formation was analyzed.

### I. Electron microscopy

For ultrastructural studies, cells were washed in PB and fixed with 2% glutaraldehyde. Samples were post-fixed with 1% osmium, rinsed, dehydrated and embedded in araldite (Durcupan, Fluka). Semi-thin sections (1.5 µm) were cut with a diamond knife and stained lightly with 1% toluidine blue. Then, semi-thin sections were re-embedded in an araldite block and detached from the glass slide by repeated freezing (liquid nitrogen) and thawing. The block with semi-thin sections was cut in ultra-thin (50-70nm) sections with a diamond knife, stained with lead citrate and examined under a Jeol JEM 1010 electron microscope.

| **Table 2.** | | | |
|---|---|---|---|
| | **Sense** | **Antisense** | **Probe** |
| Housekeeping | | | |
| GAPDH | TGGTATCGTGGAAGGACTCA TGAC (SEQ ID NO:1) | ATGCCAGTGAGCTTCCCGTTCAGC (SEQ ID NO:2) | CCCAGAGACTGTGGATGGCCCC (SEQ ID NO:3) |
| | | | |
| Pluripotency | | | |
| Oct-3a | CTTCGGATTTGCCCTTCTCG (SEQ ID NO:4) | CCTTGGAAGCTTAGCCAGGTC (SEQ ID NO:9) | |
| Nanog | TTGTTGGTTGTGCTAATCTTT GTAGA (SEQ ID NO:5) | GGTGAAATCAGGGTAAAATCAACT AAA (SEQ ID NO:6) | AGAGGTCTTGTATTTGCTGCATC GTAATGACATG (SEQ ID NO:7) |
| Rex-1 | Assay on Demand (Applied Biosystems) | | |
| Telomerase | ACGTCGTGGGAGCCAGAA (SEQ ID NO:8) | CGTAGTTGAGCACGCTGAACA (SEQ ID NO:10) | AGAAAAGAGGGCCGAGCGTCTC ACC (SEQ ID NO:11) |
| | | | |
| General Endothelium | | | |
| CD31 | ACTGCACAGCCTTCAACAGA (SEQ ID NO:12) | TTTCTTCCATGGGGCAAG (SEQ ID NO:13) | SYBER GREEN |
| CD34 | TCCAGAAACGGCCATTCAG (SEQ ID NO:15) | CCCCACCTAGCCGAGTCA (SEQ ID NO:16) | SYBER GREEN |
| KDR | TCCTGTATGGAGGAGGAGGA (SEQ ID NO:17) | CGGCTCTTTCGCTTACTGTT (SEQ ID NO:18) | SYBER GREEN |
| Flt-1 | GGACTGACAGCAAACCCAAG (SEQ ID NO:19) | CAGCCCCGACTCCTTACTTT (SEQ ID NO:20) | SYBER GREEN |
| Tie-2 | TGCCCAGATATTGGTGTCCT (SEQ ID NO:21) | CTCATAAAGCGTGGTATTCACGTA (SEQ ID NO:22) | SYBER GREEN |
| Von Willebrand (vWF) | GTCGAGCTGCACAGTGACAT (SEQ ID NO:23) | CCACGTAAGGAACAGAGACCA (SEQ ID NO:24) | SYBER GREEN |
| VE-Cadherin | GTTCACGCATCGGTTGTTC (SEQ ID NO:25) | TCTGCATCCACTGCTGTCA (SEQ ID NO:26) | SYBER GREEN |
| | | | |
| Artery-Venous | | | |
| Hey-2 | CCCCTGCGAGGAGACGA (SEQ ID NO:27) | ATCTAATCACAGAGCTAGTACTTTG CCC (SEQ ID NO:28) | CTCCGAGAGCGACATGGACGAG ACC (SEQ ID NO:29) |
| Ephrin B1 | [?] GTTCTCGACCCCAACGTGTT (SEQ ID NO:30) | CAGGCTTCCATTGGATGTTGA (SEQ ID NO:31) | TCACCTGCAATAGGCCAGAGCA GGAAATAC (SEQ ID NO:32) |
| Ephrin B2 | CTCCTCAACTGTGCCAAACC A (SEQ ID NO:33) | GGTTATCCAGGCCCTCCAAA (SEQ ID NO:34) | ACCAAGATATCAAATTCACCAT CAAGTTTCAAGAATTC (SEQ ID NO:35) |
| Eph B4 | GCCGCAGCTTTGGAAGAG (SEQ ID NO:36) | GGGAATGTCACCCACTTCAGA (SEQ ID NO:37) | CCCTGCTGAACACAAAATTGG (SEQ ID NO:38) |
| Dll-4 | ATGACCACTTCGGCCACTAT G (SEQ ID NO:39) | GCCCGAAAGACAGATAGGCTG (SEQ ID NO:40) | TCCTGCCTGCCCGGTTGGAC (SEQ ID NO:41) |
| C17 | CGACCTGCTACTCCCGCAT (SEQ ID NO:75) | CGCAGCTTGTCCAGCACA (SEQ ID NO:76) | TGAGCCAGGAGATCACCCGCGA (SEQ ID NO:77) |
| GDF-1 | GAAGCTTCTATGGCCACTCC A (SEQ ID NO:78) | AGCACAAGGATGCCCACATT (SEQ ID NO:79) | ATCCTCATCGTCTCCTCCTACGC CTTCC (SEQ ID NO:80) |
| Lefty-1 | GACTATGGAGCTCAGGGCGA (SEQ ID NO:81) | CACACTCATAAGCCAGGAAGCC (SEQ ID NO:82) | AAGTGGGCCGAGAACTGGGTGC TG (SEQ ID NO:83) |
| Lefty-2 | CTGGACCTCAGGGACTATGG AG (SEQ ID NO:84) | ACACTCGTAAGCCAGGAAGCC (SEQ ID NO:85) | TCAGGGCGACTGTGACCCTGAA GC (SEQ ID NO:86) |
| Myo1B | ATGGAGTGGATGATGCAGCA (SEQ ID NO:87) | TTCACTCGAGATTCGGGCTT (SEQ ID NO:88) | CGGAATGCCATGCAGATTGTGG G (SEQ ID NO:89) |
| Sema3F | GTGTGGGAACTTCGTCAGGC (SEQ ID NO:90) | CTCGAGTCGCTCAGGCTCC (SEQ ID NO:91) | CCTACAACCCCATGTGCACCTAT GTGAA (SEQ ID NO:92) |
| Jagged-2 | TCATCCCCTTCCAGTTCGC (SEQ ID NO:93) | AGGCTCTTCCAGCGGTCCT (SEQ ID NO:94) | TGGCCGCGCTCCTTTACCCTC (SEQ ID NO:95) |
| ALDH1A1 | GGAGTGTTGAGCGGGCTAAG (SEQ ID NO:96) | CCTCCACATTCCAGTTTGGC (SEQ ID NO:97) | CTCTGACCCCAGGAGTCACTCA AGGC (SEQ ID NO:98) |
| | | | |
| Lymphatic Endothelium | | | |
| Lyve-1 | CTTTGAAACTTGCAGCTATG GCT (SEQ ID NO:42) | TCAGGACACCCACCCCATT (SEQ ID NO:43) | AGGATTAGCCCAAACCCCAAGT GTGG (SEQ ID NO:44) |
| Prox-1 | CAGTACTGAAGAGCTGTCTA TAACCAGAG (SEQ ID NO:45) | TCTGAGCAACTTCCAGGAATCTC (SEQ ID NO:46) | CTGTACAGGGCTCTGAACATGC ACTACAATAAAGC (SEQ ID NO:47) |
| Podoplanin | CTCCAGGAACCAGCGAAGAC (SEQ ID NO:48) | AGTTGGCAGATCCTCGATGC (SEQ ID NO:49) | CTATAAGTCTGGCTTGACAACTC TGGTGGCA (SEQ ID NO:50) |
| | | | |
| Notch Pathway | | | |
| Notch-1 | CCACGGGCGACGTCACCC (SEQ ID NO:51) | TCCACTCTGGCGGGCACG (SEQ ID NO:52) | GAAAATATCGACGATTGTCCAG GAAACAA (SEQ ID NO:99) |
| Notch-2 | CTTGCAGCCCGCTACTCAC (SEQ ID NO:53) | GGTTGCGAATCAGAATCTGGA (SEQ ID NO:54) | ACATGGGCCGCTGTCCACTCCAT (SEQ ID NO:100) |
| Notch-3 | GCCGTGTGCTTCCATGG (SEQ ID NO:55) | CCGGATTTGTGTCACAGATAGC (SEQ ID NO:56) | [?] CCCATGGGCAAGACTGGCCTCC (SEQ ID NO:101) |
| Notch-4 | ATGTCTCAATGGCGGCTCC (SEQ ID NO:57) | GGAGAAGGTGCCAGGCCT (SEQ ID NO:58) | TGTGTCTGCCCCGTGCTTCAATG (SEQ ID NO:102) |
| Jagged-1 | CCAATGACTGCAGCCCTCAT (SEQ ID NO:59) | GCTCCAAAGGCACAAGGTGA (SEQ ID NO:60) | ATGGAGACAACTGGTACCGGTG CGA (SEQ ID NO:103) |
| Jagged-2 | TCATCCCCTTCCAGTTCGC (SEQ ID NO:61) | AGGCTCTTCCAGCGGTCCT (SEQ ID NO:62) | TGGCCGCGCTCCTTTACCCTC (SEQ ID NO:104) |
| Dll-1 | TTGAAGCTCTCCACACAGAT TCTC (SEQ ID NO:63) | CTCGCCCACCGTCAGGT (SEQ ID NO:64) | TGACCTCGCAACAGAAAACCCA GAAAGACT (SEQ ID NO:105) |
| Dll-3 | CTGATCTCCCACTGCCCG (SEQ ID NO:65) | TCCTAACTCCTCTCTCCAGGTTTC (SEQ ID NO:66) | CGGCCTCTTGCAGGTGCCCTT (SEQ ID NO:106) |
| Dll-4 | ATGACCACTTCGGCCACTAT G (SEQ ID NO:67) | GCCCGAAAGACAGATAGGCTG (SEQ ID NO:68) | TCCTGCCTGCCCGGTTGGAC (SEQ ID NO:107) |
| COUP-TFII | CGCCTCAAAAGTGCCTCA (SEQ ID NO:108) | GCATCCTGCCCCTCTGC (SEQ ID NO:109) | AGTGGGCATGAGACGGAAGCG (SEQ ID NO:110) |
| | | | |
| Sonic Pathway | | | |
| Patched-1 | CTGCCCTCTTCCGATCACA (SEQ ID NO:69) | TATGAGGAGGCCCACAACCA (SEQ ID NO:70) | TCGGGAAGGCTACTGGCCGGA (SEQ ID NO:111) |
| Patched-2 | CAGCTCGAACCGCAGCA (SEQ ID NO:71) | GTTCCAAGTTTGTCTCAATAATGGC (SEQ ID NO:72) | TCCACTCTGGCTTCGTGCTTACT TCCA (SEQ ID NO:112) |
| Shh | ACTCACCCCCAATTACAACC C (SEQ ID NO:73) | GGTCACCCGCAGTTTCACTC (SEQ ID NO:74) | ATGAAGAAAACACCGGAGCGGA CAGG (SEQ ID NO:113) |
| | | | |
| VEGF Pathway | | | |
| VEGF | ACCAAGGCCAGCACATAGGA (SEQ ID NO:114) | AGGCCCACAGGGATTTTCTT (SEQ ID NO:115) | AGATGAGCTTCCTACAGCACAA CAAATGTGAATG (SEQ ID NO:116) |
| Neuropilin-1 | TTTGCGCCAAAGATGTCAGA (SEQ ID NO:117) | AGTAACGCCCAATGTGAGGG (SEQ ID NO:118) | AAAGCTTTGACCTGGAGCCTGA CTCAAATC (SEQ ID NO:119) |
| | | | |
| Smooth muscle | | | |
| Smooth muscle α-actin | GCCTTGGTGTGTGACAATGG (SEQ ID NO:120) | CGTCACCCACGTAGCTGTCTT (SEQ ID NO:121) | TCTGTAAGGCCGGCTTTGCTGGG (SEQ ID NO:122) |
| Calponin | TCATCAAGGCCATCACCAAG T (SEQ ID NO:123) | CCCACGTTCACCTTGTTTCC (SEQ ID NO:124) | TGAAGCCCCACGACATTTTTGA GGC (SEQ ID NO:125) |
| Sm22α | TCTCAGCCAGCCACATCCA (SEQ ID NO:126) | GCGGCTCATGCCATAGGA (SEQ ID NO:127) | TGTAAGGGTGCAGGCGCCGG (SEQ ID NO:128) |
| Hepatocyte | | | |
| HNF1β | TCCCGCAGACTATGCTCATC (SEQ ID NO:129) | CTCCAGTGAGTCCGGGCT (SEQ ID NO:130) | CCAACCTGAGCGCCCTGGCC (SEQ ID NO:131) |
| HNF3β | CTACGCCAACATGAACTCCA (SEQ ID NO:132) | CTCGTACATCTCGCTCATCACC (SEQ ID NO:133) | ACGCACGCAAAGCCGCCC (SEQ ID NO:134) |
| CK19 | GAAGAGCTGGCCTACCTGAA (SEQ ID NO:135) | GACATGCGAAGCCAATATGA (SEQ ID NO:136) | AGGTGGATTCCGCTCCGGGC (SEQ ID NO:137) |
| α-fetoprotein | GCTGCCAAGGCCCAGGAA (SEQ ID NO:138) | CAAAAACTCGTGCTGCTTTG (SEQ ID NO:139) | AGGAAGTCTGCTTTGCTGAAGA GGGACAAA (SEQ ID NO:140) |
| CK18 | TATGAGGCCCTGCTGAACAT (SEQ ID NO:141) | AGCAACTCCATGCAAACCAT (SEQ ID NO:142) | ACCTACCGCCGCCTGCTAA (SEQ ID NO:143) |
| | | | |
| Neuronal | | | |
| Nestin | CAGGAGAAACAGGGCCTACA (SEQ ID NO:144) | GTCTTGGATCTTTGCTCCCA (SEQ ID NO:145) | CAGGAGAAACAGGGCCTACA (SEQ ID NO:146) |
| MAP2 | Assay on Demand (Applied Biosystems) | | |
| Tau | TCCAGTCGAAGATTGGGTCC (SEQ ID NO:147) | AAATAAAAAGATTGAAACCCACAA GC (SEQ ID NO:148) | TATCACCCACGTCCCTGGCGGA (SEQ ID NO:149) |
| Otx1 | AAGATCAACCTGCCGGAGT (SEQ ID NO:150) | AAGAAGTCCTCTCCAGTGCG (SEQ ID NO:151) | TTCAAGAACCGCCGCGCCA (SEQ ID NO:152) |
| Nurr1 | Assay on Demand (Applied Biosystems) | | |

### J. Karyotyping

Cells, subcultured at a 1:4 dilution 12 hours before harvesting, were collected with trypsin-EDTA and subjected to a 1.5-hour colcemid incubation followed by lysis with hypotonic KCL and fixation in acid/alcohol. Metaphases were analyzed after QFQ or GTG banding.

### K. ELISA

To assess cytokine production of undifferentiated cells, hMAPCs were plated in triplicate at 30-40 x 10³ cells/cm² at day 0 in cytokine-less expansion media and supernatant was collected 60 hours later and frozen. To assess cytokine production in differentiated cells, cells were plated in triplicate for endothelial differentiation as described above and media was collected after 7 and 14 days, and frozen. ELISA kits were from R&DSystems and the procedure was performed according to the manufacturer's recommendations.

### L. Blocking studies

Blocking of Patched and Notch pathways was performed using cyclopamine (Watkins et al., 2003) (Biomol) at 5 µM (added every 4-5 days with media change) and 1 µM of γ-secretase inhibitor L-685,458 (Bachem) (Dahlqvist et al., 2003), respectively. Exogenous VEGF was present in the media for the blocking experiments (no exogenous Shh or Dll-4).

### M. In vivo models

For the *in vivo* matrigel plug assay, 10 week-old nude mice were injected subcutaneously in the back with cold (4°C) 0.5 ml growth factor reduced matrigel containing general endothelial differentiation cytokines (300 ng/ml VEGF₁₆₅), or arterial EC differentiation cytokines (300 ng/ml VEGF₁₆₅, 100 ng/ml Shh and 100 ng/ml Dll-4), combined, or not, with 0.5 x 10⁶ undifferentiated hMAPCs or hAC133⁺ cells (unlabeled or labeled with CFSE, Molecular Probes or Resovist^{™} as described in Arbab et al. (2003). Ten and 30 days after injection, animals were perfusion fixed and matrigel plugs were removed and processed for paraffin or OCT embedding. Tissue sections were examined and photographed under a fluorescence microscope (Leica) or a confocal microscope. Ultrastructural analysis was performed as described above. *In vivo* live imaging was performed under anesthesia using a Leica Dissection microscope.

For the limb ischemia model, male nude mice were anesthetized via intraperitoneal injection of a combination of ketamine and xylazine. The left iliac artery was ligated and excised. 0.5 x 10⁶ undifferentiated hMAPCs were directly injected both in adductor and in quadriceps muscles. After 30 days, animals were euthanized and perfused with saline, followed by 4% paraformaldehyde. Muscle tissues were frozen and 3 µm sections were analyzed by confocal imaging after staining with the appropriate antibodies.

### N. Mouse and Rat Protocols

### 1) Isolation

MAPCs were derived from newborn to six-week-old mice. When newborn mice were sacrificed, the hindlimbs were removed, and the muscle was detached from them. The bones were minced into very small pieces and placed in a tube with 20-30 mL of 0.2% collagenase (Worthington). The tube was gently shaken for 45 minutes to an hour on a shaker. Cells were passed through a 40-µm nylon mesh cell strainer (Falcon), and were then triturated through a 21-gage needle. 10% serum was added to inactivate the collagenase, and the cells were centrifuged at 1800 rpm for 6 minutes. Cells were washed three times, using approximately 10-15 mL phosphate buffered saline (PBS; 1X, without calcium and magnesium, Cellgro) each time.

For adult mice, the tibia and femur bones were used. The ends of the bones were included and the muscle was removed. The bones were flushed very forcefully, using a 23-gage needle and Media 199 (1X, Gibco), into a small Petrie dish. Approximately 15-20mL of media was used in flushing the cells. Cells were filtered in a 40-µm cell strainer, triturated, and washed with PBS similar to newborn cells.

The plating procedure is used for both types of mice. After the final washing, cells are suspended in media and counted using a haemocytometer. They are plated at 6 million cells per well on a fibronectin (FN) coated 6 well plate (Corning), in 1.5mL media per well. After 3 days, another 1.5mL media is added to each well. For the rest of the first week, half of the media is changed every other day. The second week, two thirds of the media is changed at the same time interval. Beginning the third week, cells are replated at 80% confluence. Once they grow to 100% confluent, they are replated at 80% confluence.

### 2. Culture

Cells were cultured in 10cm plates (Nunc) coated with 100 ng/mL mouse fibronectin (Sigma). Coating was done for at least one hour at 37°C, two hours at room temperature, or overnight at 4°C.

Every 36-48 hours, cells were either split or the media was changed. When cells were split, the media was removed and saved for trypsin deactivation. Cells were washed once with phosphate buffered saline. 1 mL of 0.05% trypsin (Cellgro) was added to each plate for less than 30 seconds, at which time the plate was tapped to detach the cells. Cells were centrifuged at 1800 rpm for 6 minutes. Cells were resuspended in 6 mL of media per plate.

For the first two weeks after isolation, cells were kept about 100% confluent, about 3x10⁴ cells/cm². The following two weeks, they were kept at about 70-80% confluence, or about 2x10⁴ cells/cm². After cells were cultured for approximately a month, column depletion was done to remove CD45⁺ and Terr119⁺ cells from the culture. Following depletion, cells were kept at a significantly lower density of about 1-2x10² cells/cm². Mouse and rat MAPCs were isolated and cultured at 5% O₂.

### 3. Media

For rat cell culture, the media contained 60% low glucose DMEM (Gibco BRL), 40% MCDB-201 (Sigma), 1X insulin-transferrin-selenium (ITS; Sigma), 1X linoleic acid bovine serum albumin (LA-BSA; Sigma), 10⁻⁹M dexamethasone (Sigma), 10⁻⁴M ascorbic acid 3-phosphate (Sigma), 100 units of penicillin, 1,000 units of streptomycin (Gibco), 2% fetal bovine serum (FBS; HyClone), 10 ng/mL human platelet derived growth factor (R&D Systems), 10 ng/mL mouse epidermal growth factor (Sigma), and 1000 units/mL mouse leukaemia inhibitory factor (Chemicon). Mouse cells were cultured in similar media with the following modifications: 1X SITE (Sigma) was used instead of ITS, a combination of 0.2 mg/mL LA-BSA and 0.8 mg/mL powdered bovine serum albumin (BSA; Sigma) was added instead of using only LA-BSA, 1X chemically defined lipid concentrate (Gibco) was included, and dexamethasone was not included. 1 µg/mL β-mercaptoethanol was added freshly to both types of media. Media was sterilized using a 22-µm filter (Millipore), and was kept in glass bottles for up to two weeks.

### 4. Column Depletion

Depletion was done on a MACS column with a minimum of 2-3 million cells. Before adding cells, the column was washed with 30 mL of buffer, consisting of 0.5-1% BSA in PBS. For this, an 18 to 20-gage needle was attached. Cells were centrifuged, and then resuspended in about 80µL buffer. Added to this was microbeads for mouse CD45 and mouse Terr119 (Miltenyi Biotec) at 10 µL each per 5 million cells. This solution was incubated on ice for 15-20 minutes. After incubation, cells were washed twice, by suspending them in 10 mL buffer and centrifuging. Following the second wash, cells were suspended in 500 µL buffer, added to the column, and washed through in at least 30 mL buffer. At this time, a 23 to 30-gage needle was used. The solution should drip slowly, approximately 1 drop every 2-3 seconds.

Cells were collected in at least three separate 10 mL fractions, centrifuged, and counted. Each fraction was plated in 96 well plates at varying densities in order for clones to grow out. Densities of 1 and 10 cells/well worked well for single clone growth in each well. After a minimum of 1 week, clones were observed. When 30-50 cells were seen in a well, they were removed and plated again in 1 well of a 96 well plate. Clones were then removed from the 96 well plates and transferred into progressively larger plates when they begin to contact each other.

### 5. Differentiation endothelial cells from mouse and rat MAPCs

Glass 4-well chambers with were coated with 1 µg/ml human fibronectin (Sigma, F0895) for about 30 minutes to about 1 hour in the 37°C incubator. The cells were collected (with the use of 0.05% trypsin), counted and resuspended at 120K/ml in mouse MAPC culture media (see above). The fibronectin was taken off and the cells were seeded (1ml per well) (= Day 0). The cells were then incubated at 37°C, 5% 02 overnight. After about 16-20 hours, the wells were rinsed gently with PBS once, followed by one rinse with basal media (60% low glucose DMEM (Gibco BRL), 40% MCDB-201 (Sigma), 1X insulin-transferrin-selenium (ITS; Sigma), 1X linoleic acid bovine serum albumin (LA-BSA; Sigma), 10⁻⁹M dexamethasone (Sigma), 10⁻⁴M ascorbic acid 3-phosphate (Sigma), 100 units of penicillin, 1,000 units of streptomycin (Gibco)) and add 1 ml differentiation media (basal media with 10 ng/ml VEGF-A from R&D Systems) (= Day 1). The media was changed 50% (using differentiation media) on day 4 and from then on, media changed 50% every other day. For rat cells, the differentiation media contained 2% serum.

### Example 1

### In vitro analysis hMAPCs

### Phenotypic and Functional Endothelial Potential of hAC133⁺ cells and hMAPCs

Having established and characterized hMAPCs capable of differentiating into mesoderm, endoderm and ectoderm derived tissues, the endothelial potential of hMAPCs was compared with that of hAC133⁺, a cell population previously shown to be enriched for endothelial, neuronal and hematopoietic progenitors (Gehling, 2000; Asahara, 1997). Culture of hAC133⁺ cells in the presence of VEGF₁₆₅ induced downregulation of hematopoietic markers (CD34 and CD45) and up-regulation of mature endothelial markers (CD36, CD105 and αᵥβ₃) (Fig. 2a-b), as has been described Gehling et al. (2000). The majority of the cells in 21-day cultures (which are further designated as "hAC133-ECs) expressed VEGF receptors 1 (Flt-1) and 2 (KDR), angiopoietin receptors (Tie-1 and Tie-2; Fig. 2c-g) and were functional as demonstrated by uptake of acetylated-LDL (AcLDL; Fig. 2h) and vascular tube formation on matrigel (Fig. 2i).

Likewise, the majority of hMAPC cultured in the presence of VEGF₁₆₅ acquired endothelial cell markers, including KDR, Tie-1, Tie-2, CD34, CD105, VE-cadherin, CD31, and von Willebrand Factor (vWF) and αᵥβ₃ (Fig. 2j-p and Fig. 3) and the resultant endothelial cells (ECs) were functional as shown by AcLDL uptake (Fig. 2q) and vascular tube formation on matrigel (Fig. 2r). Notably, ECs derived from MAPCs had a different morphology and formed more elaborate vascular tubes as compared to hAC133-ECs (Fig. 2c-i and 1-r). Under these conditions, some of the cells in the differentiated cultures, most obviously in hAC133⁺ derived cultures (Fig. 2c-g), did not express mature EC markers, suggesting the presence of immature precursors or other cell types. Unlike in hMAPC-EC cultures, 23% of the cells remained CD45⁺ in hAC133-EC cultures (Fig. 2a). While no SMC α-actin⁺ cells were observed in hAC133⁺ cell-derived cultures, hMAPCs gave rise to SMC α-actin⁺ cells; however, these cells represented less than 5% of the differentiated cells (not shown). For hMAPCs, the increase in mRNA expression varied between the different genes from 5-fold (CD31) to over 600-fold (Flt-1) (Fig. 3).

### VEGF₁₆₅ induces arterial specification of hMAPCs but not hAC133⁺ cells

To study whether VEGF₁₆₅ was able to induce arterial EC differentiation from hMAPCs or hAC133⁺ cells *in vitro,* it was determined, using quantitative (Q)-RT-PCR and/or immunofluorescence, if the arterial markers Hey-2, Dll-4, EphrinB2 and EphrinB1, and the venous marker, EphB4, were expressed in hMAPC-ECs and hAC133-ECs generated in the presence of VEGF₁₆₅. Low levels of transcripts for arterial and venous specific genes were detected in both cell populations before differentiation. Although VEGF₁₆₅ induced an increase in general EC markers in both cell populations, VEGF₁₆₅ treatment suppressed arterial markers in hAC133⁺ cells while venous markers remained stable, but significantly induced the arterial markers Hey-2, Dll-4, EphrinB1, EphrinB2 as well as the venous marker EphB4, in hMAPCs (Fig. 4a). Another venous marker, COUP-TFII was expressed in undifferentiated hMAPCs and did not change significantly after differentiation (data not shown). At the protein level, determined by immunofluorescence, expression of arterial Hey-2 (48.3 ± 3.5 of the cells were Hey2⁺), EphrinB1 (65.8 ± 4) and venous EphB4 (31.2 ± 2.8) was found in hMAPC-ECs at day 14 (Fig. 4b-d), while no protein expression was found at baseline (data not shown). Interestingly, while the majority of hAC133-ECs expressed CD36, suggesting a microvascular phenotype, hMAPC-ECs were mostly CD36 negative, suggesting a macrovascular phenotype (Petzelbauer et al., 1993; Ades et al., 1992; Swerlick et al., 1992) (Fig. 4e). Together, this suggests a unique ability of hMAPCs to differentiate into arterial ECs in addition to venous ECs.

### Notch/patched pathway members are differentially expressed in hMAPCs and hAC133⁺ cells

The expression of Notch, and its ligands Jagged and Dll, and Shh, and its receptor patched (ptc), was compared in undifferentiated hMAPCs and hAC133⁺ cells by Q-RT-PCR. Expression of Shh was restricted to hMAPCs (Fig. 5a) and the expression of its receptors ptc1 and ptc2 was significantly higher - the former potentially driven by a positive feedback loop (Marigo et al., 1996; Pola et al., 2001) - in hMAPCs compared with hAC133⁺ cells (Fig. 5a). Likewise, Notch-1 was uniquely expressed in hMAPCs (Fig. 5b), Dll-3, Jagged-1 and Notch-3 were more highly expressed in hMAPCs than hAC133⁺ cells (Fig. 5b), while Dll-1 and Notch-4 were expressed preferentially in hAC133⁺ cells (Fig. 5b). Expression of Jagged-2, Notch-2, and Dll-4 was not significantly different between the two cell populations (Fig.5b). Baseline endogenous VEGF₁₆₅ expression was similar in both cell populations (Fig. 5c). To determine if the different response to VEGF₁₆₅ in hMAPCs and hAC133⁺ cells could be due to differences in VEGF receptor expression, their expression level was compared by Q-RT-PCR. While Flt-1 and KDR were expressed in both cell populations (data not shown), only hMAPCs expressed neuropilin-1 (NP-1), a receptor which may serve as a co-receptor in VEGF₁₆₅ induced arterial EC differentiation (Mukouyama et al., 2005) (Fig. 5c). Thus, the expression of Shh and ptc, several of the Notch ligands and receptors as well as NP-1 in hMAPCs, but not hAC133⁺ cells, may account for the ability of hMAPCs, but not hAC133⁺ cells, to differentiate along the arterial EC lineage.

### Shh or Notch pathway blocking attenuates arterial EC differentiation in hMAPCs

To investigate the involvement of Notch, Shh/Ptc and/or NP-1 in arterial EC differentiation from hMAPCs, each of them was manipulated separately. Blocking of Shh signaling with cyclopamine-mediated inhibition of the ptc receptor complex (Watkins et al., 2003) significantly decreased expression of the arterial EC markers Hey-2, EphrinB1 and EphrinB2 and simultaneously slightly increased expression of the venous marker EphB4 (Fig. 6a). An even more pronounced attenuation of arterial EC marker expression was observed by blocking the Notch pathway (Dahlqvist et al., 2003) using an inhibitor for γ-secretase (Fig. 6a). The combination of cyclopamine and γ -secretase inhibitor gave an additive effect (Fig. 3a). VEGF¹²¹, an isoform that does not bind NP-1 (Soker et al., 1998) increased arterial EC marker expression to the same extent as VEGF₁₆₅ (Fig. 3b). Since there was a rapid downregulation of endogenous VEGF₁₆₅ expression during differentiation, the latter did not confound the results obtained with VEGF₁₂₁ (data not shown). All together, this demonstrates that arterial specification in hMAPCs in the presence of VEGF₁₆₅ is at least in part mediated by the patched and Notch pathways.

### Simultaneous Notch and ptc activation boosts arterial EC fate in hMAPCs, but not in hAC133⁺ cells

To further evaluate the role of Notch and ptc in arterial specification of hMAPCs, the effect of VEGF₁₆₅ alone or combined with either Dll-4 or Jagged-1, and Shh, alone or in combination was evaluated. All conditions induced a significant increase in arterial EC markers in hMAPCs, but not hAC133⁺ cells (Fig. 7a). Out of all combinations tested, addition of Dll-4 and Shh most efficiently increased expression of Hey-2 along with downregulation of the venous marker EphB4, indicating a preferential differentiation towards arterial endothelium (Fig. 7a). The latter condition (VEGF₁₆₅+Shh+Dll-4) was examined in more detail by examining the expression of additional arterial or venous specific genes. In agreement with previous work in HUVECs (Chi et al., 2003), increased Hey-2 mRNA levels were associated with increased levels of the arterial EC specific genes, ALDH1A1 and Jagged-2, and decreased levels of the venous specific Lefty-1 and Lefty-2 transcripts (Fig. 7b). Expression of other arterial or venous transcripts (Sema3f, C17 for arterial endothelium; GDF-1 and Myo1β for venous endothelium; data not shown; (Chi et al., 2003)) were unaffected.

### Lymphatic potential of hMAPC and AC133⁺ cells

To determine the lymphatic potential of hMAPC, the expression of specific genes of lymphatic endothelium, Prox-1, Lyve-1 and podoplanin, was quantified after culture in the presence of VEGF-C, bFGF and/or VEGF₁₆₅ (VEGF-C and bFGF have recently been demonstrated to play a role in lymphatic differentiation). Up-regulation of mRNA expression of these genes suggests that hMAPC can also give rise to lymphatic endothelium, whereas VEGF-C alone or with VEGF₁₆₅ does not enhance expression of these genes more than VEGF₁₆₅ alone (Fig. 8).

### Rat and mouse MAPCs

Undifferentiated rat and mouse MAPCs express VEGF-A and its receptors VEGF-R1 and 2, NP-1, Shh and its receptors patched (Ptc) 1 and 2, Notch receptors 1 and 4 and their ligands Jagged (Jgd) 1 and 2 and D114, as well as COUPTF-11 and Prox-1.

Mouse and rat MAPCs, in the presence of VEGF-A alone, differentiated into venous, lymphatic and arterial ECs, as demonstrated by the increased expression levels, as determined by qRT-PCR, of venous (NP-2), arterial (Alk-1, NP-1, D114 and Jgd2) and lymphatic (Prox-1, LYVE-1, Flt-4, Integrin 9α and Mmr) EC markers as compared to undifferentiated cells (data not shown). Since the resulting cell populations bind Bandereia Simplifolica (BS)-I lectin (Sigma) and form tubes in matrigel (BD Biosciences), these cells functionally behave as ECs (data not shown).

Undifferentiated rat and mouse MAPCs express VEGF-A and its receptors VEGF-R1 and 2, NP-1, Shh and its receptors patched (Ptc) 1 and 2, Notch receptors 1 and 4 and their ligands Jagged (Jgd)1 and 2 and D114, as well as COUPTF-II and Pox-1, possibly explaining their ability to form the three EC types. Addition of either a Shh-antibody (final concentration of 0.5 micrograms/ml) or cyclopamine (final concentration of 10 micromolar) (both of which block the Ptc receptors) were able to decrease the arterial component in the cell cultures.

### Example 2

### In vivo analysis of hMAPCs

### Shh and D11-4 induce arterial hMAPC-EC differentiation and arterial-like vessel growth in vivo

To determine whether the same factors could also induce hMAPC differentiation into arterial endothelium *in vivo,* 0.5x10⁶ undifferentiated hMAPCs in growth factor reduced matrigel containing either VEGF₁₆₅ ("standard media"), or VEGF₁₆₅+Shh+D11-4 ("arterial media") was injected under the skin of nude mice (N=6 per group). To account for the effects of the admixed cytokines on host cells, the corresponding "cytokine-alone" groups were also included. In order to track the cells following implantation, hMAPCs were labeled with CFSE or iron particles (Resovist™; Arbab et al., 2003) before injection. Irrespective of the cytokine cocktail used, localized areas of CFSE-labeled cells (Fig. 9a) and single Resovist-labeled (Arbab et al., 2003) hMAPC-derived cells (Fig. 9b) persisted for at least 10 days in the matrigel plug as determined by *in vivo* live imaging and electron microscopy, respectively. Most implanted cells expressed (human) CD31 and (human) VE-cadherin and Fli-1 (data not shown) showing their EC identity (Fig. 9c-d). Despite their intrinsic ability to differentiate into SMCs (Fig. 1g), hMAPCs, under the present conditions, did not generate SMCs, as α-actin⁺ cells with human nuclei were not located (not shown). hMAPC-ECs generated in the presence of the arterial cytokine combination, but not with VEGF₁₆₅ alone, expressed the arterial markers Hey-2 and EphrinB1, as shown by immunohistochemistry (Fig. 9e-f) and double immunofluorescence confocal microscopy (Fig. 9g-h), demonstrating differentiation of hMAPCs *in vivo* to arterial endothelium. While hAC133⁺ cells were capable of *in vivo* differentiation into (human) CD31⁺ and UEA lectin⁺ ECs (Fig. 10a-d), they did not give rise to arterial ECs, shown by the lack of EphrinB1 and Hey-2 staining (Fig. 10e-h).

Not only did hMAPCs differentiate into arterial ECs *in vivo,* these arterial ECs contributed to vessels functionally connected to the host vasculature as demonstrated by the presence of erythrocytes in their lumen (Fig. 9f). In addition, in two animals, one per group, TRITC-labeled UEA lectin (that specifically binds to human ECs) was injected in the tail vein, 30 minutes before sacrifice. Co-labeling of TRITC with CFSE (with which the hMAPCs were labeled) confirmed that the hMAPC-EC containing vessels were connected to the host vasculature (Fig. 9i).

The arterial cytokine mix also induced the formation of arterial-like vessels in which both implanted and host cells participated. Indeed, coating with host α-actin⁺ SMCs of human EC-containing vessels was observed in the matrigel plugs containing the arterial cytokine combination, as shown by double confocal immunofluorescence (Fig. 9j). Not only did the arterial media induce a significant increase in total number of vessels (number of lectin⁺ vessels/mm²: 124±16 in arterial media versus 74±10 in standard media; P<0.05), but also significantly increased the fraction of vessels coated with SMCs (32±5% in arterial media versus 15±4% in standard media; *P*<0.05; Fig. 9k-m) as well as the diameter of these vessels (diameter (µm): 20.1±4.2 versus 14.7±3.7; *P*=0.01). While the two cytokine cocktails did not differentially affect hMAPC-EC proliferation, the increased number of vessels with the arterial mix, as well as the enhanced coating with SMCs was at least in part due to an increase in host vascular (EC + SMC) proliferation (number of PCNA⁺BS-I lectin⁺ (host EC), PCNA⁺/α-actin⁺ (host SMC) cells and PCNA⁺/UEA lectin⁺ (hMAPC-ECs): 7±3, 38±16 and 40±9 in standard media versus 18±4, 85±13 and 46±10 in arterial media; *P*<0.05; *P*<0.05; *P*=NS; Fig. 11). Consistent with a possible effect of the cytokine mix on host ECs and SMCs, more α-actin coated vessels were also seen in matrigels containing arterial versus standard media, but without hMAPCs. However, when hMAPCs were coimplanted, the arterial mix was much more effective (Fig. 9m). Significantly more deposition of Sirius Red⁺ fibrillar collagen (Fig. 9n-p) and orcein⁺ elastin (Fig. 12), both characteristics of arteries, could be detected surrounding the newly formed EC channels (EM micrograph shows that the collagen/elastin is associated with an SMC around the endothelium; Fig. 9q) when the arterial cytokine combination was used. Again, in the absence of hMAPCs, there was more collagen deposition with the arterial mix than with standard media, although collagen deposition was significantly lower than when hMAPCs were coimplanted (Fig. 9p). Electron microscopic analysis further confirmed the differences in complexity and caliber between vessels formed in matrigel plugs with standard versus arterial media (Fig. 13a-d).

### hMAPCs differentiate into arterial ECs in ischemic hind limbs

Undifferentiated hMAPCs were intramuscularly injected into mouse limbs, immediately after induction of limb ischemia. One month after injection, upon histological analysis of the quadriceps muscle, capillaries containing hMAPC-derived UEA lectin⁺ and human CD31⁺ ECs and arterioles containing hMAPC-derived EphrinB1⁺ UEA⁺ arterial ECs were detected (Fig. 14), demonstrating that hMAPCs participated in (arterial) EC growth in ischemic mouse limbs.

### In vivo mouse MAPCs

Mouse MAPCs to contributed to vessel formation and thereby functionally improved limb function in a model of mouse hind limb ischemia (Fig. 16a). Injection of 0.5 million (in left adductor or left gastrocnemic muscle) GFP-overexpressing MAPCs (GFP-overexpressing MAPCs were derived, according to the above mentioned protocol, from GFP-transgenic mice (expressing the GFP-gene under control of the chicken beta-actin promoter)) in the left adductor and gastrocnemius muscle, immediately following bilateral femoral artery ligation (Fig. 16a), resulted in stable engraftment on the left side (Fig. 16b,d) and functional improvement of both hind limbs and favorably affected the energetic status of the muscle as evidenced by their increased swimming performance and better magnetic resonance imaging (MRI) spectrum, respectively, as compared to mice injected with vehicle (PBS) only (Fig. 16e-i). As evident from the MRI recording, the non-injected contra-lateral muscle also showed improvement (Fig. 16i), despite the absence of engrafted cells (Fig. 16b).

Further, histological analysis revealed that undifferentiated (Fig. 16j,k) MAPCs contribute to ECs and SMCs *in vivo,* as evidenced by co-localization with BS-I lectin and SMC alpha-actin, respectively. In agreement with this, GFP-DAB staining revealed that both the endothelium and smooth muscle layer of some arteries was positive, indicating that they were derived from the transplanted cells (Fig. 16l). In addition, undifferentiated MAPCs also contributed to regeneration of skeletal muscle, as evidenced by the presence of GFP-positive regenerating muscle fibers (Fig. 16m).

Analysis of the injected muscles showed robust and stable (up to 5 weeks) engraftment of the cells, indicating their *in vivo* contribution. Co-localization studies showed differentiation to ECs (Fig. 16j,l), indicating that MAPCs are capable of contributing to new vessels by vasculogenesis. The cells appeared to incorporate into small and large arteries, suggesting the ability to selectively differentiate into arterial ECs (Fig. 16l). In addition, MAPCs also differentiated to SMCs (Fig. 16k,l), suggesting that they also contributed to vessel formation by arteriogenesis. Finally, as suggested by the functional improvement of the contra-lateral muscle, the cells may also contribute by secreting soluble (angiogenic) factors, thereby affecting proliferation of the endogenous host vasculature, i.e., angiogenesis. Therefore, we determined, by ELISA, the levels of VEGF protein. Undifferentiated cells secreted high levels of VEGF (3 ng/100,000 cells), suggesting that they may contribute to blood vessel growth by inducing angiogenesis.

### Bibliography

Abe, A., et al. (1998). J Virol 72:6159-6163.
Adams, R. H., W. L. Maxwell, et al. (2003). "Molecular control of arterial-venous blood vessel identity Localisation of calcium ions and calcium-ATPase activity within myelinated nerve fibres of the adult guinea-pig optic nerve. The effect of posture on diffusion into lumbar intervertebral discs. The canine vomeronasal organ. The lateral nasal gland of dog: its structure and secretory content. Surface coatings of cells in the oral epithelium of the human fetus. In memoriam. William Edgar Adams, Ph.D., D.Sc., B.Med.Sc., M.B., Ch.B., F.R.S.N.Z. The surface layer of human foetal skin and oral mucosa: a study by scanning and transmission electron microscopy. Observations on the periderm layer of human foetal oral mucosa. The blood supply of the pituitary gland of the ferret with special reference to infarction after stalk section. The venous drainage of the rat spleen. The carotid sinus complex in the hedgehog, Erinaceus europaeus. The vascularization of the rat pancreas and the effect of ischaemia on the islets of Langerhans." J Anat 202(1):105-12.
Ades, E.W. et al. (1992) "HMEC-1: establishment of an immortalized human microvascular endothelial cell line." J Invest Dermatol 99: 683-690.
Arbab, A.S. et al. (2003). "Intracytoplasmic tagging of cells with ferumoxides and transfection agent for cellular magnetic resonance imaging after cell transplantation: methods and techniques." Transplantation 76: 1123-1130.
Arbab, A.S. et al. (2003) "Characterization of biophysical and metabolic properties of cells labeled with superparamagnetic iron oxide nanoparticles and transfection agent for cellular MR imaging." Radiology 229: 838-846.
Asahara, T., H. Masuda, et al. (1999). "Bone marrow origin of endothelial progenitor cells responsible for postnatal vasculogenesis in physiological and pathological neovascularization." Circ Res 85(3): 221-8.
Asahara, T., T. Murohara, et al. (1997). "Isolation of putative progenitor endothelial cells for angiogenesis." Science 275(5302): 964-7.
Bagley, R. G. et al. (2003). "Endothelial precursor cells as a model of tumor endothelium: characterization and comparison with mature endothelial cells." Cancer Res 63(18): 5866-73.
Batinic, D., et al. (1990). Bone Marrow Transplant 6(2):103-7.
Benedito, R. & Duarte, A. (2005). "Expression of D114 during mouse embryogenesis suggests multiple developmental roles." Gene Expr Patterns. 5(6):750-5.
Bhatia, M. (2001) "AC133 expression in human stem cells." Leukemia 15: 1685-8.
Bredenbeek, P.J., et al. (1993). J. Virol 67:6439-6446.
Cao, R., A. Eriksson, et al. (2004). "Comparative evaluation of FGF-2-, VEGF-A-, and VEGF-C-induced angiogenesis, lymphangiogenesis, vascular fenestrations, and permeability." Circ Res 94(5): 664-70.
Carlson, T.R. et al. (2005) "Endothelial expression of constitutively active Notch4 elicits reversible arteriovenous malformations in adult mice." Proc Natl Acad Sci U S A.102(28):9884-9.
Carmeliet, P. (2004). "Manipulating angiogenesis in medicine." J Intern Med 255(5): 538-61.
Chi, J. T., H. Y. Chang, et al. (2003). "Endothelial cell diversity revealed by global expression profiling." Proc Natl Acad Sci U S A 100(19): 10623-8.
Chuong et al. (2000). "Sonic hedgehog signaling pathway in vertebrate epithelial appendage morphogenesis: perspectives in development and evolution." CMLS Cell. Mol. Life Sci.: 1672-1681.
Cleaver, O. & Krieg, P.A. (1998). "VEGF mediates angioblast migration during development of the dorsal aorta in Xenopus." Development 125:3905-3914.
Cohen, M.M. Jr. (2004). "The hedgehog signaling network." Am J Med Genet. 124A: 439-40.
Conway, E. M. and P. Carmeliet (2004). "The diversity of endothelial cells: a challenge for therapeutic angiogenesis." Genome Biol 5(2): 207.
Conway, E. M., D. Collen, et al. (2001). "Molecular mechanisms of blood vessel growth." Cardiovasc Res 49(3): 507-21.
Corbeil, D., et al. (1998) "AC133 hematopoietic stem cell antigen: human homologue of mouse kidney prominin or distinct member of a novel protein family?" Blood 91:2625-6.
Dahlqvist, C. et al. (2003). "Functional Notch signaling is required for BMP4-induced inhibition of myogenic differentiation." Development 130: 6089-6099.
Dalrymple-Hay, M. J., et al. (1998). "Postinfarction Ventricular Septal Rupture: the Wessex Experience," Semin Thorac Cardiovasc Surg 10(2): 111-116.
Davidson, B.L., et al. (1993). Nature Genetics 3:219-223.
de Wynter, E.A. et al. (1998) "CD34+AC133+ cells isolated from cord blood are highly enriched in long-term culture-initiating cells, NOD/SCID-repopulating cells and dendritic cell progenitors." Stem Cells 16: 387-396.
Douglas, J., et al. (1999). Nature Biotech 17:470-475.
Duarte, A., M. Hirashima, et al. (2004). "Dosage-sensitive requirement for mouse D114 in artery development." Genes Dev 18(20): 2474-8.
Dull, T., et al. (1998). J. Virol 72:8463-8471.
Fischer, A. et al. (2004). "The Notch target genes Heyl and Hey2 are required for embryonic vascular development." Genes Dev 18: 901-911.
Frolov, I., et al. (1996). Proc. Natl. Acad. Sci. USA 93:11371-11377.
Gale, N.W. et al. (2001) "Ephrin-B2 selectively marks arterial vessels and neovascularization sites in the adult, with expression in both endothelial and smooth-muscle cells." Dev Biol 230: 151-160.
Gehling, U. M., S. Ergun, et al. (2000). "In vitro differentiation of endothelial cells from AC133-positive progenitor cells." Blood 95(10): 3106-12.
Harvey, N.L. & Oliver, G. (2004) "Choose your fate: artery, vein or lymphatic vessel?" Curr Oρin Genet Dev 14: 499-505.
Hirashima, M. E. et al. (1999). "Maturation of ES cells into endothelial cells in an in vitro model ofvasculogenesis." Blood 93:1253-1263.
Hofmann, C., et al. (1999). J. Virol 73:6930-6936.
Holash, J. et al. (1999). "Vessel cooption, regression, and growth in tumors mediated by angiopoietins and VEGF." Science 284:994-998.
Hong, Y. K. et al. (2004). "Lymphatic reprogramming of blood vascular endothelium by Kaposi sarcoma-associated herpesvirus." Nat Genet 36(7): 683-5.
Iso, T. et al. (2003). "Notch signaling in vascular development." Arterioscler Thromb Vasc Biol 23: 543-553.
Jaffe, E.A. et al. (1973). "Culture of human endothelial cells derived from umbilical veins. Identification by morphologic and immunologic criteria." J Clin Invest 52: 2745-2756.
Jahagirdar, B.N. et al., (2001). Exp Hematol 29(5):543-56.
Jain, R. K. (2003). "Molecular regulation of vessel maturation." Nat Med 9(6): 685-93.
Jawien, A., et al. (1992). "Platelet-derived growth factor promotes smooth muscle migration and intimal thickening in a rat model of balloon angioplasty." J Clin Invest 89: 507-11.
Jiang, Y., B. N. Jahagirdar, et al. (2002). "Pluripotency of mesenchymal stem cells derived from adult marrow." Nature 418(6893): 41-9.
Jiang, Y., et al. (2002). Exp Hematol 30(8):896-904.
Jiang, Y. et al. (2003). "Neuroectodermal differentiation from mouse multipotent adult progenitor cells." Proc Natl Acad Sci U.S.A. 100(Suppl 1): 11854-11860.
Johnston, S.A., et al. (1993). Genet. Eng. (NY) 15:225-236.
Kafri, T., et al. (1998). J. Virol 73:576-584.
Kalka, C., H. Masuda, et al. (2000). "Transplantation of ex vivo expanded endothelial progenitor cells for therapeutic neovascularization." Proc Natl Acad Sci USA 97(7): 3422-7.
Kawamoto, A., T. Tkebuchava, et al. (2003). "Intramyocardial transplantation of autologous endothelial progenitor cells for therapeutic neovascularization of myocardial ischemia." Circulation 107(3): 461-8.
Kinnaird, T. et al. (2004). "Marrow-derived stromal cells express genes encoding a broad spectrum of arteriogenic cytokines and promote in vitro and in vivo arteriogenesis through paracrine mechanisms." Circ Res 94: 678-685.
Kusano, K.F. et al. (2004). "Sonic hedgehog induces arteriogenesis in diabetic vasa nervorum and restores function in diabetic neuropathy." Arterioscler Thromb Vasc Biol 24: 2102-2107.
Lacorre, D. A., E. S. Baekkevold, et al. (2004). "Plasticity of endothelial cells: rapid dedifferentiation of freshly isolated high endothelial venule endothelial cells outside the lymphoid tissue microenvironment." Blood 103(11): 4164-72.
Laquerre, S., et al. (1998). J. Virol 72: 9683-9697.
Lawson, N. D. et al. (2001). "Notch signaling is required for arterial-venous differentiation during embryonic vascular development." Development 128(19): 3675-83.
Lawson, N. D., A. M. Vogel, et al. (2002). "Sonic hedgehog and vascular endothelial growth factor act upstream of the Notch pathway during arterial endothelial differentiation." Dev Cell 3(1): 127-36.
le Noble, F. et al. (2004) "Flow regulates arterial-venous differentiation in the chick embryo yolk sac." Development 131:361-375.
Line, Y. et al. (2000). "Origins of circulating endothelial cells and endothelial outgrowth from blood." J Clin Invest 105:71-7.
Liu, Z.J. et al. (2003). "Regulation of Notch1 and D114 by vascular endothelial growth factor in arterial endothelial cells: implications for modulating arteriogenesis and angiogenesis." Mol Cell Biol 23: 14-25.
Loeffler, J. and Behr, J. (1993). Methods in Enzymology 217:599-618.
Luttun, A. et al. (2002). "Lack of plasminogen activator inhibitor-1 promotes growth and abnormal matrix remodeling of advanced atherosclerotic plaques in apolipoprotein E-deficient mice." Arterioscler Thromb Vasc Biol 22: 499-505.
Masood, R. et al. (2005). "Ephrin B2 expression in Kaposi sarcoma is induced by human herpesvirus type 8: phenotype switch from venous to arterial endothelium." Blood 105: 1310-1318.
Marigo, V. et al. (1996). "Sonic hedgehog differentially regulates expression of GLI and GLI3 during limb development." Dev Biol 180: 273-283.
Martin, F., et al. (1999). J. Virol 73:6923-6929.
Melo, L.G. et al. (2004) "Molecular and cell-based therapies for protection, rescue, and repair of ischemic myocardium: reasons for cautious optimism." Circulation 109: 2386-2393.
Miller, A.D., and C. Buttimore (1986) in Mol. Cell. Biol 6: 2895-2902. Mochizuki, H., et al. (1988). J. Virol. 72:8873-8883.
Molin, M., et al. (1998). J. Virol.72:8358-8361.
Moyon, D., L. Pardanaud, et al. (2001). "Selective expression of angiopoietin 1 and 2 in mesenchymal cells surrounding veins and arteries of the avian embryo." Mech Dev 106(1-2): 133-6.
Mukouyama, Y.S. et al. (2002) "Sensory nerves determine the pattern of arterial differentiation and blood vessel branching in the skin." Cell 109:693-705.
Mukouyama, Y.S. et al. (2005). "Peripheral nerve-derived VEGF promotes arterial differentiation via neuropilin 1-mediated positive feedback" Development 132:941-952 (2005).
Muschler, G.F., et al. (1997). J. Bone Joint Surg. Am 79(11):1699-709.
Neuhaus, T. et al. (2003). "The use of suppression subtractive hybridization for the study of SDF-1 alpha induced gene-expression in human endothelial cells." Mol Cell Probes 17, 245-252.
Noseda, M. et al. (2004). "Notch activation results in phenotypic and functional changes consistent with endothelial-to-mesenchymal transformation." Circ Res 94: 910-917.
Nugent, et al. (2001). J Surg. Res. 99: 228-234.
Oettgen, P. (2001). "Transcriptional regulation of vascular development." Circ Res 89(5): 380-8.
Othman-Hassan, K. et al. (2001). "Arterial identity of endothelial cells is controlled by local cues." Dev Biol 237:398-409.
Owens, G.K., et al. (2004) "Molecular regulation of vascular smooth muscle cell differentiation in development and disease." Physiol Rev 84:767-801.
Partanen, T. A. and K. Paavonen (2001). "Lymphatic versus blood vascular endothelial growth factors and receptors in humans." Microsc Res Tech 55(2): 108-21.
Peichev, M. et al. (2000). "Expression of VEGFR2 and AC133 by circulating human CD34(+) cells identifies a population of functional endothelial precursors." Blood 95:952-958.
Pelosi, E. et al. (2002). "Identification of the hemangioblast in postnatal life." Blood 100(9): 3203-8.
Persons, D., et al. (1998). Nature Medicine 4:1201-1205.
Petzelbauer, P. et al. (1993) "Heterogeneity of dermal microvascular endothelial cell antigen expression and cytokine responsiveness in situ and in cell culture." J Immunol 151: 5062-5072.
Pola, R. et al. (2001). "The morphogen Sonic hedgehog is an indirect angiogenic agent upregulating two families of angiogenic growth factors." Nat Med 7: 706-711.
Quirici, N. et al. (2001) "Differentiation and expansion of endothelial cells from human bone marrow CD133(+) cells." Br J Haematol 115:186-94.
Rafii, S. and D. Lyden (2003). "Therapeutic stem and progenitor cell transplantation for organ vascularization and regeneration." Nat Med 9(6): 702-12.
Reyes, M et al. (2002). J. Clin. Invest. 109:337-346.
Reyes, M. et al. (2001). Blood 98:2615-2625.
Reyes, M. and C.M. Verfaillie (2001). Ann N Y Acad Sci 938:231-5. Robbins, et al. (1997). J. Virol. 71(12):9466-9474.
Robbinson, C.J. and Stringer, S.E. (2001). "The splice variants of vascular endothelial growth factor (VEGF) and their receptors." J. Cell Sci. 114:853-865.
Ruiz i Altaba, A. et al. (2002). "Gli and hedgehog in cancer: tumours, embryos and stem cells." Nat Rev Cancer 2: 361-372.
Salven, P., S. Mustjoki, et al. (2003). "VEGFR-3 and CD133 identify a population of CD34+ lymphatic/vascular endothelial precursor cells." Blood 101(1): 168-72.
Salmons, B. and Gunzburg, W.H. (1993) "Targeting of Retroviral Vectors for Gene Therapy." Hum. Gene Therapy 4:129-141.
Salvato, G. (2001). "Quantitative and morphological analysis of the vascular bed in bronchial biopsy specimens from asthmatic and non-asthmatic subjects." Thorax 56: 902-906.
Schatteman, G. C., H. D. Hanlon, et al. (2000). "Blood-derived angioblasts accelerate blood-flow restoration in diabetic mice." J Clin Invest 106(4): 571-8.
Schwarzenberger, P., et al. (1997). J. Virol 71:8563-8571.
Schwartz, R.E. et al. (2002) "Multipotent adult progenitor cells from bone marrow differentiate into functional hepatocyte-like cells." J Clin Invest 109: 1291-1302.
Sebestyen, et al. (1998). Nature Biotech 16:80-85.
Seki, T., K. H. Hong, et al. (2004). "Isolation of a regulatory region of activin receptor-like kinase 1 gene sufficient for arterial endothelium-specific expression." Circ Res 94(8): e72-7.
Seki, T., J. Yun, et al. (2003). "Arterial endothelium-specific activin receptor-like kinase 1 expression suggests its role in arterialization and vascular remodeling." Circ Res 93(7): 682-9.
Shawber, C.J. et al. (2003). "Notch signaling in primary endothelial cells" Ann N Y Acad Sci 995: 162-170.
Shawber, C.J. & Kitajewski, J. (2004). "Notch function in the vasculature: insights from zebrafish, mouse and man." Bioessays 26: 225-234.
Shi, Q. et al. (1998). "Evidence for circulating bone marrow-derived endothelial cells." Blood 92:362-367.
Shima, D.T. and Mailhos, C. (2000) "Vascular developmental biology: getting nervous." Opin. Gen. Dev. 10: 536-542.
Shintani, S., T. Murohara, et al. (2001). "Augmentation of postnatal neovascularization with autologous bone marrow transplantation." Circulation 103(6): 897-903.
Shutter, J. R. et al. (2000). "D114, a novel Notch ligand expressed in arterial endothelium." Genes Dev 14(11): 1313-8.
Soker, S. et al. (1998) "Neuropilin-1 is expressed by endothelial and tumor cells as an isoform-specific receptor for vascular endothelial growth factor." Cell 92: 735-745.
Sorensen, L. K. et al. (2003). "Loss of distinct arterial and venous boundaries in mice lacking endoglin, a vascular-specific TGFbeta coreceptor." Dev Biol 261(1): 235-50.
Stalmans, I., Y. S. Ng, et al. (2002). "Arteriolar and venular patterning in retinas of mice selectively expressing VEGF isoforms." J Clin Invest 109(3): 327-36.
Stamm, C., B. Westphal, et al. (2003). "Autologous bone-marrow stem-cell transplantation for myocardial regeneration." Lancet 361(9351): 45-6.
Sutton, R., et al. (1998). J. Virol. 72:5781-5788.
Swerlick, R.A. et al. (1992). "Human dermal microvascular endothelial but not human umbilical vein endothelial cells express CD36 in vivo and in vitro." J Immunol 148: 78-83.
Tomlinson, J. E. and J. N. Topper (2003). "New insights into endothelial diversity." Curr Atheroscler Rep 5(3): 223-9.
Torres-Vazquez, J., et al. (2003) "Molecular distinction between arteries and veins." Cell Tissue Res. 314: 43-59.
Uchida, N. et al. (2000) "Direct isolation of human central nervous system stem cells." Proc Natl Acad Sci USA 97:14720-5.
Verfaillie, C.M. (2002). Trends Cell Biol 12(11):502-8.
Villa, N., L. Walker, et al. (2001). "Vascular expression of Notch pathway receptors and ligands is restricted to arterial vessels." Mech Dev 108(1-2): 161-4.
Visconti, R.P. et al. "Orchestration of angiogenesis and arteriovenous contribution by angiopoietins and vascular endothelial growth factor (VEGF)." Proc Natl Acad Sci U S A 99: 8219-8224.
Wagner, E., et al. (1992). Proc. Natl. Acad. Sci. USA 89:6099-6103.
Wang, H. U., Z. F. Chen, et al. (1998). "Molecular distinction and angiogenic interaction between embryonic arteries and veins revealed by ephrin-B2 and its receptor Eph-B4." Cell 93(5): 741-53.
Waite, K.A. & Eng, C. (2003) From developmental disorder to heritable cancer: it's all in the BMP/TGF-beta family." Nat Rev Genet 4:63-773.
Wang, Y., J. Liu, et al. (2001). "Expression of a truncated first exon BCR sequence in chronic myelogenous leukemia cells blocks cell growth and induces cell death." Cancer Res 61(1): 138-44.
Watkins, D.N. et al. (2003). "Hedgehog signaling within airway epithelial progenitors and in small-cell lung cancer." Nature 422: 313-317.
Williams, R.S., et al. (1991). Proc. Natl. Acad. Sci. USA 88:2726-2730.
Williams, C.K. et al. (2006). "Upregulation of the Notch ligand Delta-like 4 inhibits VEGF-induced endothelial cell function." Blood. 107(3):931-9.
Wold, W. (1998) Adenovirus Methods and Protocols, Humana Methods in Molecular Medicine, Blackwell Science, Ltd.
Yancopoulos, G.D., et al. (2000) "Vascular-specific growth factors and blood vessel formation." Nature 407: 242-248.
Yang, J. et al. (1998). "FGF and Shh signals control dopaminergic and serotonergic cell fate in the anterior neural plate." Cell 93:755-66.
Yang, N.S., et al. (1990). Proc. Natl. Acad. Sci. USA 87:9568-9572.
Yang, C., et al. (2004) "Enhancement of neovascularization with cord blood CD133+ cell-derived endothelial progenitor cell transplantation." Thromb Haemost 91:1202-12.
You, L.R. et al. (2005). "Suppression of Notch signalling by the COUP-TFII transcription factor regulates vein identity." Nature 435:98-104.
Xiong, C., et al. (1989). Science 243:1188-1191.
Zhang, Z. G. et al. (2002). "Bone marrow-derived endothelial progenitor cells participate in cerebral neovascularization after focal cerebral ischemia in the adult mouse." Circ Res 90(3): 284-8.
Zhong, T.P. et al. (2001). "Gridlock signalling pathway fashions the first embryonic artery." Nature 414:216-220.
Zhong, T.P. et al. (2000). "gridlock, an HLH gene required for assembly of the aorta in zebrafish." Science 287:1820-1824.

All publications, patents and patent applications are incorporated herein by reference. While in the foregoing specification this invention has been described in relation to certain preferred embodiments thereof, and many details have been set forth for purposes of illustration, it will be apparent to those skilled in the art that the invention is susceptible to additional embodiments and that certain of the details described herein may be varied considerably without departing from the basic principles of the invention.

### STATEMENTS

1. A method to differentiate cells comprising contacting an enriched population of stem cells that differentiate into more than one embryonic lineage with VEGF₁₆₅, VEGF₁₂₁ or a combination thereof, so as to increase expression of one or more of Hey-2, EphrinB1, EphrinB2 or EphB4 relative to the initial population.
2. A method to differentiate cells comprising contacting an enriched population of stem cells that differentiate into more than one embryonic lineage with (a) at least one vascular endothelial growth factor (VEGF) and (b) one or more notch ligands, one or more patched ligands or a combination thereof, so as to increase expression of one or more of Hey-2, Ephrin B1 or EphrinB2 relative to the initial population.
3. A method to differentiate cells comprising contacting an enriched population of stem cells that differentiate into more than one embryonic lineage with VEGF₁₆₅, VEGF-C, VEGF-D or a combination thereof, so as to increase expression of one or more of prox-1, podoplanin or lyve-1 relative to the initial population.
4. The method of any one of claims 1-3, wherein the enriched population of cells are embryonic stem cells.
5. The method of any one of claim 1-3, wherein the enriched population of cells are non-embryonic stem, non-germ, non-embryonic germ cells.
6. The method of claim 2 further comprising a decrease in the expression of EphB4 relative to the initial population.
7. The method of any one of claims 1-6, wherein the enriched population of stem cells are mammalian cells.
8. The method of claim 2, wherein the VEGF comprises VEGF₁₆₅, VEGF₁₂₁ or a combination thereof.
9. The method of claim 2, wherein the notch ligand comprises DII-1, Dll-3, Dll-4, Jagged-1, Jagged-2 or a combination thereof.
10. The method of claim 2, wherein the patched ligand comprises sonic hedgehog.
11. The method of claim 2, wherein the VEGF comprises VEGF₁₆₅, the notch ligand comprises DII-4 and the patched ligand comprises sonic hedgehog.
12. The method of any one of claims 1-11 further comprising admixing the differentiated cells with a pharmaceutically acceptable carrier.
13. The method of any one of claims 1-12 further comprising administering the differentiated cells to a subject.
14. A composition comprising VEGF₁₆₅, VEGF₁₂₁ or a combination thereof and an enriched population of stem cells that differentiate into more than one embryonic lineage.
15. A composition comprising at least one vascular endothelial growth factor (VEGF) and at least one notch ligand, at least one patched ligand or a combination thereof and an enriched population of stem cells that differentiate into more than one embryonic lineage.
16. The composition of claim 14 further comprising cells differentiated from the enriched population of cells, wherein the expression of one or more of Hey-2, EphrinB1, EphrinB2 or EphB4 is increased in the differentiated cells.
17. The composition of any one of claims 14-16, wherein the enriched population of cells are embryonic stem cells.
18. The composition of any one of claims 14-16, wherein the enriched population of cells are non-embryonic stem, non-germ, non-embryonic germ cells.
19. A composition comprising the cells differentiated from an enriched population of stem cells by the method of any one of claims 1-13 and cell culture media or a pharmaceutically acceptable carrier.
20. The composition of any one of claims 14-19, wherein the enriched population of stem cells are mammalian.
21. The composition of any one of claims 15,17-20, wherein the VEGF comprises VEGF₁₂₁, VEGF₁₆₅ or a combination thereof.
22. The composition of any one of claims 15-21, wherein the notch ligand comprises DII-1, DII-3, DII-4, Jagged-1, Jagged-2 or a combination thereof.
23. The composition of any one of claims 15-22, wherein the patched ligand comprises sonic hedgehog.
24. The composition of any one of claims 15-23, wherein the VEGF comprises VEGF₁₆₅, the notch ligand comprises DII-4, and the patched ligand comprises sonic hedgehog.
25. The composition of any one of claims 14-24 further comprising a pharmaceutically acceptable carrier or cell culture media,
26. A method to prepare a composition comprising admixing VEGF₁₆₅, VEGF_{12I} or a combination thereof and an enriched population stem cells that differentiate into at least one embryonic lineage.
27. A method to prepare a composition comprising admixing at least one vascular endothelial growth factor (VEGF) and at least one notch ligand, at least one patched ligand or a combination thereof, and an enriched population of stem cells that differentiate into at least one embryonic lineage.
28. The method of claim 26 further comprising cells differentiated from the enriched population of stem cells, wherein the expression of one or more of Hey-2, EphrinB1, EphrinB2 or EphB4 is increased in the differentiated cells relative to the initial population.
29. A method to prepare a composition comprising admixing the cells differentiated from the enriched population of stem cells by the method of any one of claims 1-13 and a carrier.
30. The method of any one of claims 26-28 further comprising admixing a culture medium.
31. The method of any one of claims 26-28 further comprising admixing a pharmaceutically acceptable carrier.
32. The method of claim 29, wherein the carrier is a cell culture medium or a pharmaceutically acceptable carrier.

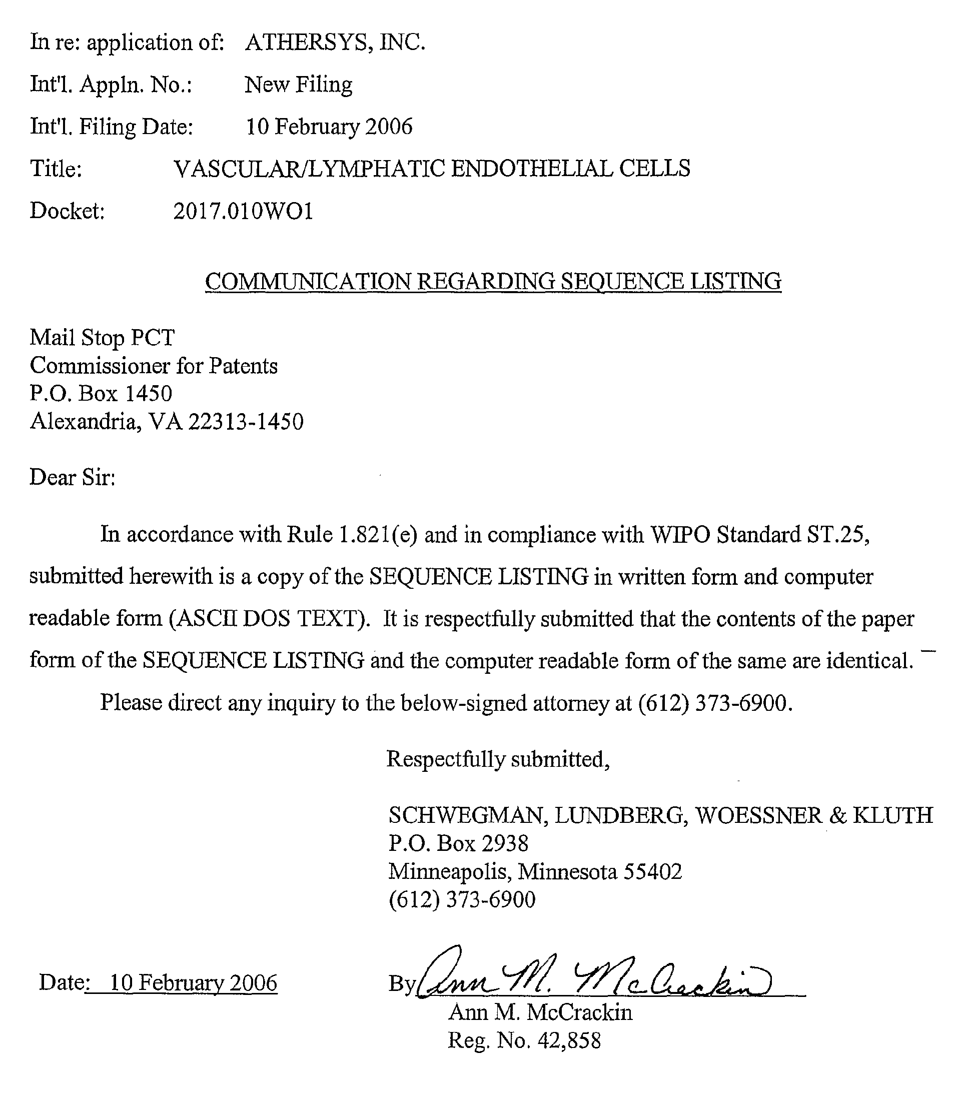
In re: application of: ATHERSYS, INC.
Int'l. Appln. No.: New Filing
Int'l. Filing Date: 10 February 2006
Title: VASCULAR/LYMPHATIC ENDOTHELIAL CELLS
Docket: 2017.010WO1

COMMUNICATION REGARDING SEQUENCE LISTING

Mail Stop PCT
Commissioner for Patents
P.O. Box 1450
Alexandria, VA 22313-1450

Dear Sir:

In accordance with Rule 1.821(e) and in compliance with WIPO Standard ST.25,
submitted herewith is a copy of the SEQUENCE LISTING in written form and computer
readable form (ASCII DOS TEXT). It is respectfully submitted that the contents of the paper
form of the SEQUENCE LISTING and the computer readable form of the same are identical.
Please direct any inquiry to the below-signed attorney at (612) 373-6900.

Respectfully submitted,

SCHWEGMAN, LUNDBERG, WOESSNER & KLUTH
P.O. Box 2938
Minneapolis, Minnesota 55402
(612) 373-6900

Date: 10 February 2006
By
Ann M. McCrackin
Reg. No. 42,858

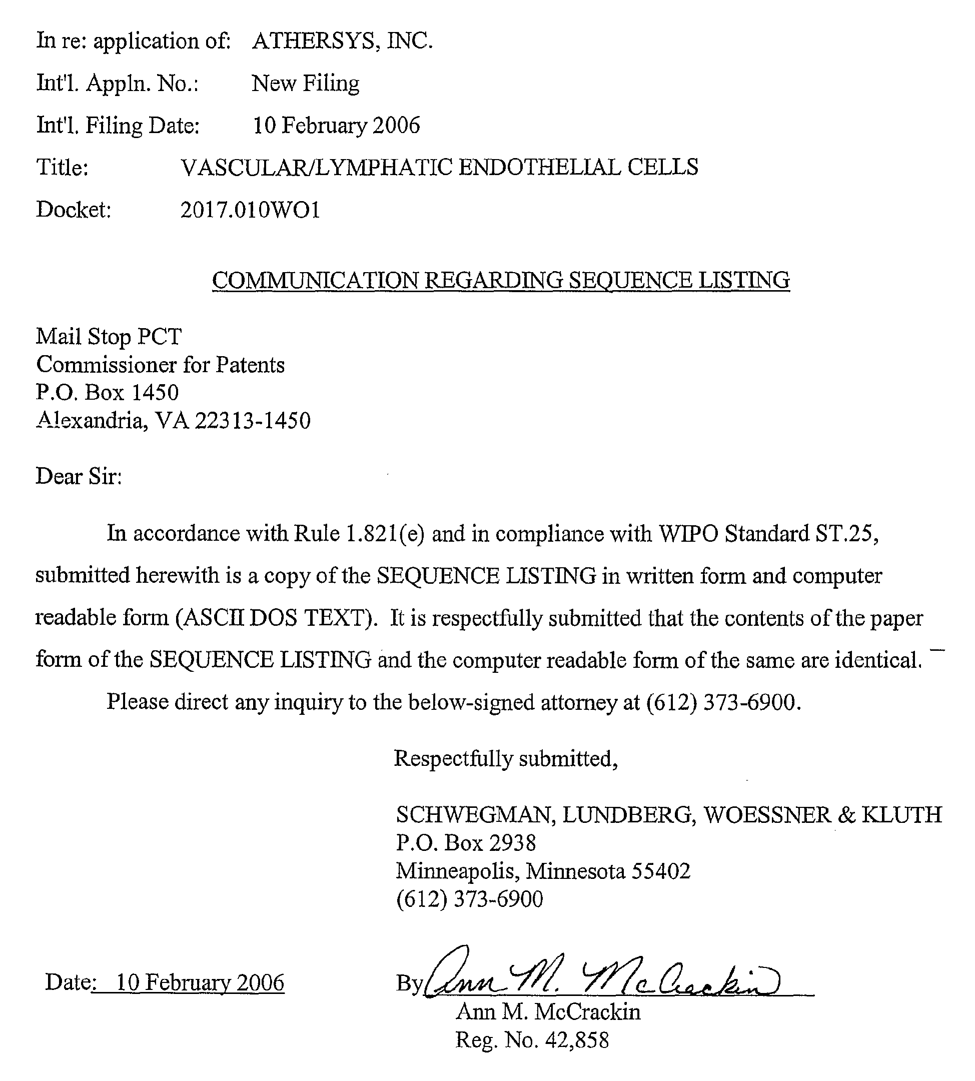
In re: application of: ATHERSYS, INC.
Int'l. Appln. No.: New Filing
Int'l. Filing Date: 10 February 2006
Title: VASCULAR/LYMPHATIC ENDOTHELIAL CELLS
Docket: 2017.010WO1

COMMUNICATION REGARDING SEQUENCE LISTING

Mail Stop PCT
Commissioner for Patents
P.O. Box 1450
Alexandria, VA 22313-1450

Dear Sir:

In accordance with Rule 1.821(e) and in compliance with WIPO Standard ST.25,
submitted herewith is a copy of the SEQUENCE LISTING in written form and computer
readable form (ASCII DOS TEXT). It is respectfully submitted that the contents of the paper
form of the SEQUENCE LISTING and the computer readable form of the same are identical.
Please direct any inquiry to the below-signed attorney at (612) 373-6900.

Respectfully submitted,

SCHWEGMAN, LUNDBERG, WOESSNER & KLUTH
P.O. Box 2938
Minneapolis, Minnesota 55402
(612) 373-6900

Date: 10 February 2006
By
Ann M. McCrackin
Reg. No. 42,858

## Claims

1. A method to differentiate cells comprising contacting an enriched population of stem cells that differentiate into more than one embryonic lineage with VEGF₁₆₅, VEGF-C, VEGF-D or a combination thereof, so as to increase expression of one or more of prox-1, podoplanin or lyve-1 relative to the initial population.

2. The method of claim 1, wherein the enriched population of cells are embryonic stem cells.

3. The method claim 1, wherein the enriched population of cells are non-embryonic stem, non-germ, non-embryonic germ cells.

4. The method of any one of claims 1-3, wherein the enriched population of stem cells are mammalian cells.

5. The method of any one of claims 1- 4 further comprising admixing the differentiated cells with a pharmaceutically acceptable carrier.

6. A composition comprising VEGF₁₆₅, VEGF-C, VEGF-D or a combination thereof and an enriched population of stem cells that differentiate into more than one embryonic lineage.

7. The composition claim 6, wherein the enriched population of cells are embryonic stem cells.

8. The composition of claim 6, wherein the enriched population of cells are non-embryonic stem, non-germ, non-embryonic germ cells.

9. A composition comprising the cells differentiated from an enriched population of stem cells by the method of any one of claims 1-4 and cell culture media or a pharmaceutically acceptable carrier.

10. The composition of any one of claims 6-9, wherein the enriched population of stem cells are mammalian.

11. The composition of any one of claims 6-8 further comprising a pharmaceutically acceptable carrier or cell culture media.

12. A medical device comprising the cells differentiated from the enriched population of cells by the method of any one of claims 1 to 5.

13. The device of claim 12 which comprises a valve, stent, shunt or graft such as an artificial vessel graft.

14. A composition comprising the cells differentiated from the enriched population of cells by the method of any of claims 1 to 5 for use in a method for the treatment of a vascular condition including ischemia atherosclerosis, congestive heart failure, peripheral vasculature disorder, coronary vascular disease, hypertension, stroke, aneurysm, thrombosis, arrhythmia or tachycardia or surgical or physical trauma.

15. The method of claim 1 wherein when the cells are contacted with VEGF₁₆₅, VEGF₁₂₁ is also added.


SEQUENCE LISTING

<110> Regents of the University of Minnesota
      PROYECTO DE BIOMEDICINA CIMA S.L.
      Catherine M. Verfaillie
      Felipe Prosper
      Xabier Lopez Aranguren
      Carlos Clavel Claver
      Aernout Luttun

<120> Vascular/Lymphatic Endothelial Cells

<130> 2017.010WO1

<150> US 60/652,015
<151> 2005-02-10

<160> 152

<170> FastSEQ for Windows Version 4.0

<210> 1
<211> 24
<212> DNA
<213> Artificial Sequence

<220>
<223> A synthetic primer

<400> 1
tggtatcgtg gaaggactca tgac                                              24

<210> 2
<211> 24
<212> DNA
<213> Artificial Sequence

<220>
<223> A synthetic primer

<400> 2
atgccagtga gcttcccgtt cagc                                              24

<210> 3
<211> 22
<212> DNA
<213> Artificial Sequence

<220>
<223> A synthetic primer

<400> 3
cccagagact gtggatggcc cc                                                22

<210> 4
<211> 20
<212> DNA
<213> Artificial Sequence

<220>
<223> A synthetic primer

<400> 4
cttcggattt gcccttctcg                                                   20

<210> 5
<211> 26
<212> DNA
<213> Artificial Sequence

<220>
<223> A synthetic primer

<400> 5
ttgttggttg tgctaatctt tgtaga                                            26

<210> 6
<211> 27
<212> DNA
<213> Artificial Sequence

<220>
<223> A synthetic primer

<400> 6
ggtgaaatca gggtaaaatc aactaaa                                          27

<210> 7
<211> 34
<212> DNA
<213> Artificial Sequence

<220>
<223> A synthetic primer

<400> 7
agaggtcttg tatttgctgc atcgtaatga catg                                  34

<210> 8
<211> 18
<212> DNA
<213> Artificial Sequence

<220>
<223> A synthetic primer

<400> 8
acgtcgtggg agccagaa                                                    18

<210> 9
<211> 21
<212> DNA
<213> Artificial Sequence

<220>
<223> A synthetic primer

<400> 9
ccttggaagc ttagccaggt c                                                21

<210> 10
<211> 21
<212> DNA
<213> Artificial Sequence

<220>
<223> A synthetic primer

<400> 10
cgtagttgag cacgctgaac a                                                21

<210> 11
<211> 25
<212> DNA
<213> Artificial Sequence

<220>
<223> A synthetic primer

<400> 11
agaaaagagg gccgagcgtc tcacc                                            25

<210> 12
<211> 20
<212> DNA
<213> Artificial Sequence

<220>
<223> A synthetic primer

<400> 12
actgcacagc cttcaacaga                                                  20

<210> 13
<211> 18
<212> DNA
<213> Artificial Sequence

<220>
<223> A synthetic primer

<400> 13
tttcttccat ggggcaag                                                    18

<210> 14

<220>
<223> A synthetic primer

<400> 14
 000

<210> 15
<211> 19
<212> DNA
<213> Artificial Sequence

<220>
<223> A synthetic primer

<400> 15
tccagaaacg gccattcag                                                   19

<210> 16
<211> 18
<212> DNA
<213> Artificial Sequence

<220>
<223> A synthetic primer

<400> 16
ccccacctag ccgagtca                                                    18

<210> 17
<211> 20
<212> DNA
<213> Artificial Sequence

<220>
<223> A synthetic primer

<400> 17
tcctgtatgg aggaggagga                                                    20

<210> 18
<211> 20
<212> DNA
<213> Artificial Sequence

<220>
<223> A synthetic primer

<400> 18
cggctctttc gcttactgtt                                                    20

<210> 19
<211> 20
<212> DNA
<213> Artificial Sequence

<220>
<223> A synthetic primer

<400> 19
ggactgacag caaacccaag                                                    20

<210> 20
<211> 20
<212> DNA
<213> Artificial Sequence

<220>
<223> A synthetic primer

<400> 20
cagccccgac tccttacttt                                                    20

<210> 21
<211> 20
<212> DNA
<213> Artificial Sequence

<220>
<223> A synthetic primer

<400> 21
tgcccagata ttggtgtcct                                                    20

<210> 22
<211> 24
<212> DNA
<213> Artificial Sequence

<220>
<223> A synthetic primer

<400> 22
ctcataaagc gtggtattca cgta                                               24

<210> 23
<211> 20
<212> DNA
<213> Artificial Sequence

<220>
<223> A synthetic primer

<400> 23
gtcgagctgc acagtgacat                                                    20

<210> 24
<211> 21
<212> DNA
<213> Artificial Sequence

<220>
<223> A synthetic primer

<400> 24
ccacgtaagg aacagagacc a                                                  21

<210> 25
<211> 19
<212> DNA
<213> Artificial Sequence

<220>
<223> A synthetic primer

<400> 25
gttcacgcat cggttgttc                                                    19

<210> 26
<211> 19
<212> DNA
<213> Artificial Sequence

<220>
<223> A synthetic primer

<400> 26
tctgcatcca ctgctgtca                                                    19

<210> 27
<211> 17
<212> DNA
<213> Artificial Sequence

<220>
<223> A synthetic primer

<400> 27
cccctgcgag gagacga                                                      17

<210> 28
<211> 28
<212> DNA
<213> Artificial Sequence

<220>
<223> A synthetic primer

<400> 28
atctaatcac agagctagta ctttgccc                                          28

<210> 29
<211> 25
<212> DNA
<213> Artificial Sequence

<220>
<223> A synthetic primer

<400> 29
ctccgagagc gacatggacg agacc                                             25

<210> 30
<211> 20
<212> DNA
<213> Artificial Sequence

<220>
<223> A synthetic primer

<400> 30
gttctcgacc ccaacgtgtt                                                   20

<210> 31
<211> 21
<212> DNA
<213> Artificial Sequence

<220>
<223> A synthetic primer

<400> 31
[?]aggcttcca ttggatgttg a                                               21

<210> 32
<211> 30
<212> DNA
<213> Artificial Sequence

<220>
<223> A synthetic primer

<400> 32
tcacctgcaa taggccagag caggaaatac                                        30

<210> 33
<211> 21
<212> DNA
<213> Artificial Sequence

<220>
<223> A synthetic primer

<400> 33
ctcctcaact gtgccaaacc a                                                 21

<210> 34
<211> 20
<212> DNA
<213> Artificial Sequence

<220>
<223> A synthetic primer

<400> 34
ggttatccag gccctccaaa                                                   20

<210> 35
<211> 38
<212> DNA
<213> Artificial Sequence

<220>
<223> A synthetic primer

<400> 35
accaagatat caaattcacc atcaagtttc aagaattc                               38

<210> 36
<211> 18
<212> DNA
<213> Artificial Sequence

<220>
<223> A synthetic primer

<400> 36
gccgcagctt tggaagag                                                    18

<210> 37
<211> 21
<212> DNA
<213> Artificial Sequence

<220>
<223> A synthetic primer

<400> 37
gggaatgtca cccacttcag a                                                21

<210> 38
<211> 21
<212> DNA
<213> Artificial Sequence

<220>
<223> A synthetic primer

<400> 38
ccctgctgaa cacaaaattg g                                                21

<210> 39
<211> 21
<212> DNA
<213> Artificial Sequence

<220>
<223> A synthetic primer

<400> 39
atgaccactt cggccactat g                                                21

<210> 40
<211> 21
<212> DNA
<213> Artificial Sequence

<220>
<223> A synthetic primer

<400> 40
gcccgaaaga cagataggct g                                                21

<210> 41
<211> 20
<212> DNA
<213> Artificial Sequence

<220>
<223> A synthetic primer

<400> 41
tcctgcctgc ccggttggac                                                  20

<210> 42
<211> 23
<212> DNA
<213> Artificial Sequence

<220>
<223> A synthetic primer

<400> 42
ctttgaaact tgcagctatg gct                                              23

<210> 43
<211> 19
<212> DNA
<213> Artificial Sequence

<220>
<223> A synthetic primer

<400> 43
tcaggacacc caccccatt                                                    19

<210> 44
<211> 26
<212> DNA
<213> Artificial Sequence

<220>
<223> A synthetic primer

<400> 44
aggattagcc caaaccccaa gtgtgg                                            26

<210> 45
<211> 29
<212> DNA
<213> Artificial Sequence

<220>
<223> A synthetic primer

<400> 45
cagtactgaa gagctgtcta taaccagag                                         29

<210> 46
<211> 23
<212> DNA
<213> Artificial Sequence

<220>
<223> A synthetic primer

<400> 46
tctgagcaac ttccaggaat ctc                                               23

<210> 47
<211> 35
<212> DNA
<213> Artificial Sequence

<220>
<223> A synthetic primer

<400> 47
ctgtacaggg ctctgaacat gcactacaat aaagc                                35

<210> 48
<211> 20
<212> DNA
<213> Artificial Sequence

<220>
<223> A synthetic primer

<400> 48
ctccaggaac cagcgaagac                                                 20

<210> 49
<211> 20
<212> DNA
<213> Artificial Sequence

<220>
<223> A synthetic primer

<400> 49
agttggcaga tcctcgatgc                                                 20

<210> 50
<211> 31
<212> DNA
<213> Artificial Sequence

<220>
<223> A synthetic primer

<400> 50
ctataagtct ggcttgacaa ctctggtggc a                                    31

<210> 51
<211> 18
<212> DNA
<213> Artificial Sequence

<220>
<223> A synthetic primer

<400> 51
ccacgggcga cgtcaccc                                                   18

<210> 52
<211> 18
<212> DNA
<213> Artificial Sequence

<220>
<223> A synthetic primer

<400> 52
tccactctgg cgggcacg                                                   18

<210> 53
<211> 19
<212> DNA
<213> Artificial Sequence

<220>
<223> A synthetic primer

<400> 53
cttgcagccc gctactcac                                                  19

<210> 54
<211> 21
<212> DNA
<213> Artificial Sequence

<220>
<223> A synthetic primer

<400> 54
ggttgcgaat cagaatctgg a                                                21

<210> 55
<211> 17
<212> DNA
<213> Artificial Sequence

<220>
<223> A synthetic primer

<400> 55
gccgtgtgct tccatgg                                                     17

<210> 56
<211> 22
<212> DNA
<213> Artificial Sequence

<220>
<223> A synthetic primer

<400> 56
ccggatttgt gtcacagata gc                                               22

<210> 57
<211> 19
<212> DNA
<213> Artificial Sequence

<220>
<223> A synthetic primer

<400> 57
atgtctcaat ggcggctcc                                                   19

<210> 58
<211> 18
<212> DNA
<213> Artificial Sequence

<220>
<223> A synthetic primer

<400> 58
ggagaaggtg ccaggcct                                                    18

<210> 59
<211> 20
<212> DNA
<213> Artificial Sequence

<220>
<223> A synthetic primer

<400> 59
ccaatgactg cagccctcat                                                  20

<210> 60
<211> 20
<212> DNA
<213> Artificial Sequence

<220>
<223> A synthetic primer

<400> 60
gctccaaagg cacaaggtga                                                  20

<210> 61
<211> 19
<212> DNA
<213> Artificial Sequence

<220>
<223> A synthetic primer

<400> 61
tcatcccctt ccagttcgc                                                    19

<210> 62
<211> 19
<212> DNA
<213> Artificial Sequence

<220>
<223> A synthetic primer

<400> 62
aggctcttcc agcggtcct                                                    19

<210> 63
<211> 24
<212> DNA
<213> Artificial Sequence

<220>
<223> A synthetic primer

<400> 63
ttgaagctct ccacacagat tctc                                              24

<210> 64
<211> 17
<212> DNA
<213> Artificial Sequence

<220>
<223> A synthetic primer

<400> 64
ctcgcccacc gtcaggt                                                      17

<210> 65
<211> 18
<212> DNA
<213> Artificial Sequence

<220>
<223> A synthetic primer

<400> 65
ctgatctccc actgcccg                                                    18

<210> 66
<211> 24
<212> DNA
<213> Artificial Sequence

<220>
<223> A synthetic primer

<400> 66
tcctaactcc tctctccagg tttc                                             24

<210> 67
<211> 21
<212> DNA
<213> Artificial Sequence

<220>
<223> A synthetic primer

<400> 67
atgaccactt cggccactat g                                                21

<210> 68
<211> 21
<212> DNA
<213> Artificial Sequence

<220>
<223> A synthetic primer

<400> 68
gcccgaaaga cagataggct g                                                21

<210> 69
<211> 19
<212> DNA
<213> Artificial Sequence

<220>
<223> A synthetic primer

<400> 69
ctgccctctt ccgatcaca                                                    19

<210> 70
<211> 20
<212> DNA
<213> Artificial Sequence

<220>
<223> A synthetic primer

<400> 70
tatgaggagg cccacaacca                                                   20

<210> 71
<211> 17
<212> DNA
<213> Artificial Sequence

<220>
<223> A synthetic primer

<400> 71
cagctcgaac cgcagca                                                      17

<210> 72
<211> 25
<212> DNA
<213> Artificial Sequence

<220>
<223> A synthetic primer

<400> 72
gttccaagtt tgtctcaata atggc                                          25

<210> 73
<211> 21
<212> DNA
<213> Artificial Sequence

<220>
<223> A synthetic primer

<400> 73
actcaccccc aattacaacc c                                              21

<210> 74
<211> 20
<212> DNA
<213> Artificial Sequence

<220>
<223> A synthetic primer

<400> 74
ggtcacccgc agtttcactc                                                20

<210> 75
<211> 19
<212> DNA
<213> Artificial Sequence

<220>
<223> A synthetic primer

<400> 75
cgacctgcta ctcccgcat                                                 19

<210> 76
<211> 18
<212> DNA
<213> Artificial Sequence

<220>
<223> A synthetic primer

<400> 76
   cgcagcttgt ccagcaca                                                    18

<210> 77
<211> 22
<212> DNA
<213> Artificial Sequence

<220>
<223> A synthetic primer

<400> 77
tgagccagga gatcacccgc ga                                                22

<210> 78
<211> 21
<212> DNA
<213> Artificial Sequence

<220>
<223> A synthetic primer

<400> 78
gaagcttcta tggccactcc a                                                 21

<210> 79
<211> 20
<212> DNA
<213> Artificial Sequence

<220>
<223> A synthetic primer

<400> 79
agcacaagga tgcccacatt                                                   20

<210> 80
<211> 28
<212> DNA
<213> Artificial Sequence

<220>
<223> A synthetic primer

<400> 80
atcctcatcg tctcctccta cgccttcc                                          28

<210> 81
<211> 20
<212> DNA
<213> Artificial Sequence

<220>
<223> A synthetic primer

<400> 81
gactatggag ctcagggcga                                                   20

<210> 82
<211> 22
<212> DNA
<213> Artificial Sequence

<220>
<223> A synthetic primer

<400> 82
cacactcata agccaggaag cc                                                22

<210> 83
<211> 24
<212> DNA
<213> Artificial Sequence

<220>
<223> A synthetic primer

<400> 83
aagtgggccg agaactgggt gctg                                              24

<210> 84
<211> 22
<212> DNA
<213> Artificial Sequence

<220>
<223> A synthetic primer

<400> 84
ctggacctca gggactatgg ag                                                22

<210> 85
<211> 21
<212> DNA
<213> Artificial Sequence

<220>
<223> A synthetic primer

<400> 85
acactcgtaa gccaggaagc c                                                 21

<210> 86
<211> 24
<212> DNA
<213> Artificial Sequence

<220>
<223> A synthetic primer

<400> 86
tcagggcgac tgtgaccctg aagc                                              24

<210> 87
<211> 20
<212> DNA
<213> Artificial Sequence

<220>
<223> A synthetic primer

<400> 87
atggagtgga tgatgcagca                                                    20

<210> 88
<211> 20
<212> DNA
<213> Artificial Sequence

<220>
<223> A synthetic primer

<400> 88
ttcactcgag attcgggctt                                                    20

<210> 89
<211> 23
<212> DNA
<213> Artificial Sequence

<220>
<223> A synthetic primer

<400> 89
cggaatgcca tgcagattgt ggg                                                23

<210> 90
<211> 20
<212> DNA
<213> Artificial Sequence

<220>
<223> A synthetic primer

<400> 90
gtgtgggaac ttcgtcaggc                                                    20

<210> 91
<211> 19
<212> DNA
<213> Artificial Sequence

<220>
<223> A synthetic primer

<400> 91
ctcgagtcgc tcaggctcc 19

<210> 92
<211> 28
<212> DNA
<213> Artificial Sequence

<220>
<223> A synthetic primer

<400> 92
cctacaaccc catgtgcacc tatgtgaa 28

<210> 93
<211> 19
<212> DNA
<213> Artificial Sequence

<220>
<223> A synthetic primer

<400> 93
tcatcccctt ccagttcgc 19

<210> 94
<211> 19
<212> DNA
<213> Artificial Sequence

<220>
<223> A synthetic primer

<400> 94
aggctcttcc agcggtcct                                                    19

<210> 95
<211> 21
<212> DNA
<213> Artificial Sequence

<220>
<223> A synthetic primer

<400> 95
tggccgcgct cctttaccct c                                                 21

<210> 96
<211> 20
<212> DNA
<213> Artificial Sequence

<220>
<223> A synthetic primer

<400> 96
ggagtgttga gcgggctaag                                                   20

<210> 97
<211> 20
<212> DNA
<213> Artificial Sequence

<220>
<223> A synthetic primer

<400> 97
cctccacatt ccagtttggc                                                   20

<210> 98
<211> 26
<212> DNA
<213> Artificial Sequence

<220>
<223> A synthetic primer

<400> 98
ctctgacccc aggagtcact caaggc                                          26

<210> 99
<211> 29
<212> DNA
<213> Artificial Sequence

<220>
<223> A synthetic primer

<400> 99
gaaaatatcg acgattgtcc aggaaacaa                                       29

<210> 100
<211> 23
<212> DNA
<213> Artificial Sequence

<220>
<223> A synthetic primer

<400> 100
acatgggccg ctgtccactc cat                                             23

<210> 101
<211> 22
<212> DNA
<213> Artificial Sequence

<220>
<223> A synthetic primer

<400> 101
cccatgggca agactggcct cc                                              22

<210> 102
<211> 23
<212> DNA
<213> Artificial Sequence

<220>
<223> A synthetic primer

<400> 102
tgtgtctgcc ccgtgcttca atg                                              23

<210> 103
<211> 25
<212> DNA
<213> Artificial Sequence

<220>
<223> A synthetic primer

<400> 103
atggagacaa ctggtaccgg tgcga                                            25

<210> 104
<211> 21
<212> DNA
<213> Artificial Sequence

<220>
<223> A synthetic primer

<400> 104
tggccgcgct cctttaccct c                                                21

<210> 105
<211> 30
<212> DNA
<213> Artificial Sequence

<220>
<223> A synthetic primer

<400> 105
tgacctcgca acagaaaacc cagaaagact                                        30

<210> 106
<211> 21
<212> DNA
<213> Artificial Sequence

<220>
<223> A synthetic primer

<400> 106
cggcctcttg caggtgccct t                                                 21

<210> 107
<211> 20
<212> DNA
<213> Artificial Sequence

<220>
<223> A synthetic primer

<400> 107
tcctgcctgc ccggttggac                                                   20

<210> 108
<211> 19
<212> DNA
<213> Artificial Sequence

<220>
<223> A synthetic primer

<400> 108
cgcctcaaaa agtgcctca                                                    19

<210> 109
<211> 17
<212> DNA
<213> Artificial Sequence

<220>
<223> A synthetic primer

<400> 109
gcatcctgcc cctctgc                                                    17

<210> 110
<211> 22
<212> DNA
<213> Artificial Sequence

<220>
<223> A synthetic primer

<400> 110
agtgggcatg agacgggaag cg                                              22

<210> 111
<211> 21
<212> DNA
<213> Artificial Sequence

<220>
<223> A synthetic primer

<400> 111
tcgggaaggc tactggccgg a                                               21

<210> 112
<211> 27
<212> DNA
<213> Artificial Sequence

<220>
<223> A synthetic primer

<400> 112
tccactctgg cttcgtgctt acttcca                                         27

<210> 113
<211> 26
<212> DNA
<213> Artificial Sequence

<220>
<223> A synthetic primer

<400> 113
atgaagaaaa caccggagcg gacagg                                          26

<210> 114
<211> 20
<212> DNA
<213> Artificial Sequence

<220>
<223> A synthetic primer

<400> 114
accaaggcca gcacatagga                                                 20

<210> 115
<211> 20
<212> DNA
<213> Artificial Sequence

<220>
<223> A synthetic primer

<400> 115
aggcccacag ggattttctt                                                 20

<210> 116
<211> 34
<212> DNA
<213> Artificial Sequence

<220>
<223> A synthetic primer

<400> 116
agatgagctt cctacagcac aacaaatgtg aatg                                34

<210> 117
<211> 20
<212> DNA
<213> Artificial Sequence

<220>
<223> A synthetic primer

<400> 117
tttgcgccaa agatgtcaga                                                20

<210> 118
<211> 20
<212> DNA
<213> Artificial Sequence

<220>
<223> A synthetic primer

<400> 118
agtaacgccc aatgtgaggg                                                20

<210> 119
<211> 30
<212> DNA
<213> Artificial Sequence

<220>
<223> A synthetic primer

<400> 119
aaagctttga cctggagcct gactcaaatc                                     30

<210> 120
<211> 20
<212> DNA
<213> Artificial Sequence

<220>
<223> A synthetic primer

<400> 120
gccttggtgt gtgacaatgg                                                    20

<210> 121
<211> 21
<212> DNA
<213> Artificial Sequence

<220>
<223> A synthetic primer

<400> 121
cgtcacccac gtagctgtct t                                                  21

<210> 122
<211> 23
<212> DNA
<213> Artificial Sequence

<220>
<223> A synthetic primer

<400> 122
tctgtaaggc cggctttgct ggg                                                23

<210> 123
<211> 21
<212> DNA
<213> Artificial Sequence

<220>
<223> A synthetic primer

<400> 123
tcatcaaggc catcaccaag t                                                  21

<210> 124
<211> 20
<212> DNA
<213> Artificial Sequence

<220>
<223> A synthetic primer

<400> 124
cccacgttca ccttgtttcc                                                    20

<210> 125
<211> 25
<212> DNA
<213> Artificial Sequence

<220>
<223> A synthetic primer

<400> 125
tgaagcccca cgacattttt gaggc                                              25

<210> 126
<211> 19
<212> DNA
<213> Artificial Sequence

<220>
<223> A synthetic primer

<400> 126
tctcagccag ccacatcca                                                     19

<210> 127
<211> 18
<212> DNA
<213> Artificial Sequence

<220>
<223> A synthetic primer

<400> 127
gcggctcatg ccatagga                                                    18

<210> 128
<211> 20
<212> DNA
<213> Artificial Sequence

<220>
<223> A synthetic primer

<400> 128
tgtaagggtg caggcgccgg                                                  20

<210> 129
<211> 20
<212> DNA
<213> Artificial Sequence

<220>
<223> A synthetic primer

<400> 129
tcccgcagac tatgctcatc                                                  20

<210> 130
<211> 18
<212> DNA
<213> Artificial Sequence

<220>
<223> A synthetic primer

<400> 130
ctccagtgag tccgggct                                                    18

<210> 131
<211> 20
<212> DNA
<213> Artificial Sequence

<220>
<223> A synthetic primer

<400> 131
ccaacctgag cgccctggcc                                                    20

<210> 132
<211> 20
<212> DNA
<213> Artificial Sequence

<220>
<223> A synthetic primer

<400> 132
ctacgccaac atgaactcca                                                    20

<210> 133
<211> 22
<212> DNA
<213> Artificial Sequence

<220>
<223> A synthetic primer

<400> 133
ctcgtacatc tcgctcatca cc                                                 22

<210> 134
<211> 18
<212> DNA
<213> Artificial Sequence

<220>
<223> A synthetic primer

<400> 134
acgcacgcaa agccgccc                                                      18

<210> 135
<211> 20
<212> DNA
<213> Artificial Sequence

<220>
<223> A synthetic primer

<400> 135
gaagagctgg cctacctgaa                                                    20

<210> 136
<211> 20
<212> DNA
<213> Artificial Sequence

<220>
<223> A synthetic primer

<400> 136
gacatgcgaa gccaatatga                                                    20

<210> 137
<211> 20
<212> DNA
<213> Artificial Sequence

<220>
<223> A synthetic primer

<400> 137
aggtggattc cgctccgggc                                                    20

<210> 138
<211> 18
<212> DNA
<213> Artificial Sequence

<220>
<223> A synthetic primer

<400> 138
gctgccaagg cccaggaa                                                    18

<210> 139
<211> 20
<212> DNA
<213> Artificial Sequence

<220>
<223> A synthetic primer

<400> 139
caaaaactcg tgctgctttg                                                  20

<210> 140
<211> 30
<212> DNA
<213> Artificial Sequence

<220>
<223> A synthetic primer

<400> 140
aggaagtctg ctttgctgaa gagggacaaa                                       30

<210> 141
<211> 20
<212> DNA
<213> Artificial Sequence

<220>
<223> A synthetic primer

<400> 141
tatgaggccc tgctgaacat                                                  20

<210> 142
<211> 20
<212> DNA
<213> Artificial Sequence

<220>
<223> A synthetic primer

<400> 142
agcaactcca tgcaaaccat                                                    20

<210> 143
<211> 19
<212> DNA
<213> Artificial Sequence

<220>
<223> A synthetic primer

<400> 143
acctaccgcc gcctgctaa                                                     19

<210> 144
<211> 20
<212> DNA
<213> Artificial Sequence

<220>
<223> A synthetic primer

<400> 144
caggagaaac agggcctaca                                                    20

<210> 145
<211> 20
<212> DNA
<213> Artificial Sequence

<220>
<223> A synthetic primer

<400> 145
gtcttggatc tttgctccca                                                    20

<210> 146
<211> 20
<212> DNA
<213> Artificial Sequence

<220>
<223> A synthetic primer

<400> 146
caggagaaac agggcctaca                                                    20

<210> 147
<211> 20
<212> DNA
<213> Artificial Sequence

<220>
<223> A synthetic primer

<400> 147
tccagtcgaa gattgggtcc                                                    20

<210> 148
<211> 26
<212> DNA
<213> Artificial Sequence

<220>
<223> A synthetic primer

<400> 148
aaataaaaag attgaaaccc acaagc                                             26

<210> 149
<211> 22
<212> DNA
<213> Artificial Sequence

<220>
<223> A synthetic primer

<400> 149
tatcacccac gtccctggcg ga                                                22

<210> 150
<211> 19
<212> DNA
<213> Artificial Sequence

<220>
<223> A synthetic primer

<400> 150
aagatcaacc tgccggagt                                                    19

<210> 151
<211> 20
<212> DNA
<213> Artificial Sequence

<220>
<223> A synthetic primer

<400> 151
aagaagtcct ctccagtgcg                                                   20

<210> 152
<211> 19
<212> DNA
<213> Artificial Sequence

<220>
<223> A synthetic primer

<400> 152
ttcaagaacc gccgcgcca                                                    19

SEQUENCE LISTING

<110> Regents of the University of Minnesota
      PROYECTO DE BIOMEDICINA CIMA S.L.
      Catherine M. Verfaillie
      Felipe Prosper
      Xabier Lopez Aranguren
      Carlos Clavel Claver
      Aernout Luttun

<120> Vascular/Lymphatic Endothelial Cells

<130> 2017.010WO1

<150> US 60/652,015
<151> 2005-02-10

<160> 152

<170> FastSEQ for Windows Version 4.0

<210> 1
<211> 24
<212> DNA
<213> Artificial Sequence

<220>
<223> A synthetic primer

<400> 1
tggtatcgtg gaaggactca tgac                                              24

<210> 2
<211> 24
<212> DNA
<213> Artificial Sequence

<220>
<223> A synthetic primer

<400> 2
atgccagtga gcttcccgtt cagc                                              24

<210> 3
<211> 22
<212> DNA
<213> Artificial Sequence

<220>
<223> A synthetic primer

<400> 3
cccagagact gtggatggcc cc                                                22

<210> 4
<211> 20
<212> DNA
<213> Artificial Sequence

<220>
<223> A synthetic primer

<400> 4
cttcggattt gcccttctcg                                                   20

<210> 5
<211> 26
<212> DNA
<213> Artificial Sequence

<220>
<223> A synthetic primer

<400> 5
ttgttggttg tgctaatctt tgtaga                                            26

<210> 6
<211> 27
<212> DNA
<213> Artificial Sequence

<220>
<223> A synthetic primer

<400> 6
ggtgaaatca gggtaaaatc aactaaa                                          27

<210> 7
<211> 34
<212> DNA
<213> Artificial Sequence

<220>
<223> A synthetic primer

<400> 7
agaggtcttg tatttgctgc atcgtaatga catg                                  34

<210> 8
<211> 18
<212> DNA
<213> Artificial Sequence

<220>
<223> A synthetic primer

<400> 8
acgtcgtggg agccagaa                                                    18

<210> 9
<211> 21
<212> DNA
<213> Artificial Sequence

<220>
<223> A synthetic primer

<400> 9
ccttggaagc ttagccaggt c                                                21

<210> 10
<211> 21
<212> DNA
<213> Artificial Sequence

<220>
<223> A synthetic primer

<400> 10
cgtagttgag cacgctgaac a                                                21

<210> 11
<211> 25
<212> DNA
<213> Artificial Sequence

<220>
<223> A synthetic primer

<400> 11
agaaaagagg gccgagcgtc tcacc                                            25

<210> 12
<211> 20
<212> DNA
<213> Artificial Sequence

<220>
<223> A synthetic primer

<400> 12
actgcacagc cttcaacaga                                                  20

<210> 13
<211> 18
<212> DNA
<213> Artificial Sequence

<220>
<223> A synthetic primer

<400> 13
tttcttccat ggggcaag                                                    18

<210> 14

<220>
<223> A synthetic primer

<400> 14
 000

<210> 15
<211> 19
<212> DNA
<213> Artificial Sequence

<220>
<223> A synthetic primer

<400> 15
tccagaaacg gccattcag                                                   19

<210> 16
<211> 18
<212> DNA
<213> Artificial Sequence

<220>
<223> A synthetic primer

<400> 16
ccccacctag ccgagtca                                                    18

<210> 17
<211> 20
<212> DNA
<213> Artificial Sequence

<220>
<223> A synthetic primer

<400> 17
tcctgtatgg aggaggagga                                                    20

<210> 18
<211> 20
<212> DNA
<213> Artificial Sequence

<220>
<223> A synthetic primer

<400> 18
cggctctttc gcttactgtt                                                    20

<210> 19
<211> 20
<212> DNA
<213> Artificial Sequence

<220>
<223> A synthetic primer

<400> 19
ggactgacag caaacccaag                                                    20

<210> 20
<211> 20
<212> DNA
<213> Artificial Sequence

<220>
<223> A synthetic primer

<400> 20
   cagccccgac tccttacttt                                                    20

<210> 21
<211> 20
<212> DNA
<213> Artificial Sequence

<220>
<223> A synthetic primer

<400> 21
tgcccagata ttggtgtcct                                                    20

<210> 22
<211> 24
<212> DNA
<213> Artificial Sequence

<220>
<223> A synthetic primer

<400> 22
ctcataaagc gtggtattca cgta                                               24

<210> 23
<211> 20
<212> DNA
<213> Artificial Sequence

<220>
<223> A synthetic primer

<400> 23
gtcgagctgc acagtgacat                                                    20

<210> 24
<211> 21
<212> DNA
<213> Artificial Sequence

<220>
<223> A synthetic primer

<400> 24
ccacgtaagg aacagagacc a                                                  21

<210> 25
<211> 19
<212> DNA
<213> Artificial Sequence

<220>
<223> A synthetic primer

<400> 25
gttcacgcat cggttgttc                                                    19

<210> 26
<211> 19
<212> DNA
<213> Artificial Sequence

<220>
<223> A synthetic primer

<400> 26
tctgcatcca ctgctgtca                                                    19

<210> 27
<211> 17
<212> DNA
<213> Artificial Sequence

<220>
<223> A synthetic primer

<400> 27
cccctgcgag gagacga                                                      17

<210> 28
<211> 28
<212> DNA
<213> Artificial Sequence

<220>
<223> A synthetic primer

<400> 28
atctaatcac agagctagta ctttgccc                                          28

<210> 29
<211> 25
<212> DNA
<213> Artificial Sequence

<220>
<223> A synthetic primer

<400> 29
ctccgagagc gacatggacg agacc                                             25

<210> 30
<211> 20
<212> DNA
<213> Artificial Sequence

<220>
<223> A synthetic primer

<400> 30
gttctcgacc ccaacgtgtt                                                   20

<210> 31
<211> 21
<212> DNA
<213> Artificial Sequence

<220>
<223> A synthetic primer

<400> 31
caggcttcca ttggatgttg a                                                 21

<210> 32
<211> 30
<212> DNA
<213> Artificial Sequence

<220>
<223> A synthetic primer

<400> 32
tcacctgcaa taggccagag caggaaatac                                        30

<210> 33
<211> 21
<212> DNA
<213> Artificial Sequence

<220>
<223> A synthetic primer

<400> 33
ctcctcaact gtgccaaacc a                                                 21

<210> 34
<211> 20
<212> DNA
<213> Artificial Sequence

<220>
<223> A synthetic primer

<400> 34
ggttatccag gccctccaaa                                                   20

<210> 35
<211> 38
<212> DNA
<213> Artificial Sequence

<220>
<223> A synthetic primer

<400> 35
accaagatat caaattcacc atcaagtttc aagaattc                               38

<210> 36
<211> 18
<212> DNA
<213> Artificial Sequence

<220>
<223> A synthetic primer

<400> 36
gccgcagctt tggaagag                                                    18

<210> 37
<211> 21
<212> DNA
<213> Artificial Sequence

<220>
<223> A synthetic primer

<400> 37
gggaatgtca cccacttcag a                                                21

<210> 38
<211> 21
<212> DNA
<213> Artificial Sequence

<220>
<223> A synthetic primer

<400> 38
ccctgctgaa cacaaaattg g                                                21

<210> 39
<211> 21
<212> DNA
<213> Artificial Sequence

<220>
<223> A synthetic primer

<400> 39
atgaccactt cggccactat g                                                21

<210> 40
<211> 21
<212> DNA
<213> Artificial Sequence

<220>
<223> A synthetic primer

<400> 40
gcccgaaaga cagataggct g                                                21

<210> 41
<211> 20
<212> DNA
<213> Artificial Sequence

<220>
<223> A synthetic primer

<400> 41
tcctgcctgc ccggttggac                                                  20

<210> 42
<211> 23
<212> DNA
<213> Artificial Sequence

<220>
<223> A synthetic primer

<400> 42
ctttgaaact tgcagctatg gct                                              23

<210> 43
<211> 19
<212> DNA
<213> Artificial Sequence

<220>
<223> A synthetic primer

<400> 43
tcaggacacc caccccatt                                                    19

<210> 44
<211> 26
<212> DNA
<213> Artificial Sequence

<220>
<223> A synthetic primer

<400> 44
aggattagcc caaaccccaa gtgtgg                                            26

<210> 45
<211> 29
<212> DNA
<213> Artificial Sequence

<220>
<223> A synthetic primer

<400> 45
cagtactgaa gagctgtcta taaccagag                                         29

<210> 46
<211> 23
<212> DNA
<213> Artificial Sequence

<220>
<223> A synthetic primer

<400> 46
tctgagcaac ttccaggaat ctc                                               23

<210> 47
<211> 35
<212> DNA
<213> Artificial Sequence

<220>
<223> A synthetic primer

<400> 47
ctgtacaggg ctctgaacat gcactacaat aaagc 35

<210> 48
<211> 20
<212> DNA
<213> Artificial Sequence

<220>
<223> A synthetic primer

<400> 48
ctccaggaac cagcgaagac 20

<210> 49
<211> 20
<212> DNA
<213> Artificial Sequence

<220>
<223> A synthetic primer

<400> 49
agttggcaga tcctcgatgc 20

<210> 50
<211> 31
<212> DNA
<213> Artificial Sequence

<220>
<223> A synthetic primer

<400> 50
ctataagtct ggcttgacaa ctctggtggc a                                    31

<210> 51
<211> 18
<212> DNA
<213> Artificial Sequence

<220>
<223> A synthetic primer

<400> 51
ccacgggcga cgtcaccc                                                   18

<210> 52
<211> 18
<212> DNA
<213> Artificial Sequence

<220>
<223> A synthetic primer

<400> 52
tccactctgg cgggcacg                                                   18

<210> 53
<211> 19
<212> DNA
<213> Artificial Sequence

<220>
<223> A synthetic primer

<400> 53
cttgcagccc gctactcac                                                  19

<210> 54
<211> 21
<212> DNA
<213> Artificial Sequence

<220>
<223> A synthetic primer

<400> 54
ggttgcgaat cagaatctgg a                                                21

<210> 55
<211> 17
<212> DNA
<213> Artificial Sequence

<220>
<223> A synthetic primer

<400> 55
gccgtgtgct tccatgg                                                     17

<210> 56
<211> 22
<212> DNA
<213> Artificial Sequence

<220>
<223> A synthetic primer

<400> 56
ccggatttgt gtcacagata gc                                               22

<210> 57
<211> 19
<212> DNA
<213> Artificial Sequence

<220>
<223> A synthetic primer

<400> 57
atgtctcaat ggcggctcc                                                   19

<210> 58
<211> 18
<212> DNA
<213> Artificial Sequence

<220>
<223> A synthetic primer

<400> 58
ggagaaggtg ccaggcct                                                    18

<210> 59
<211> 20
<212> DNA
<213> Artificial Sequence

<220>
<223> A synthetic primer

<400> 59
ccaatgactg cagccctcat                                                  20

<210> 60
<211> 20
<212> DNA
<213> Artificial Sequence

<220>
<223> A synthetic primer

<400> 60
gctccaaagg cacaaggtga                                                  20

<210> 61
<211> 19
<212> DNA
<213> Artificial Sequence

<220>
<223> A synthetic primer

<400> 61
tcatcccctt ccagttcgc                                                    19

<210> 62
<211> 19
<212> DNA
<213> Artificial Sequence

<220>
<223> A synthetic primer

<400> 62
aggctcttcc agcggtcct                                                    19

<210> 63
<211> 24
<212> DNA
<213> Artificial Sequence

<220>
<223> A synthetic primer

<400> 63
ttgaagctct ccacacagat tctc                                              24

<210> 64
<211> 17
<212> DNA
<213> Artificial Sequence

<220>
<223> A synthetic primer

<400> 64
ctcgcccacc gtcaggt                                                      17

<210> 65
<211> 18
<212> DNA
<213> Artificial Sequence

<220>
<223> A synthetic primer

<400> 65
ctgatctccc actgcccg                                                    18

<210> 66
<211> 24
<212> DNA
<213> Artificial Sequence

<220>
<223> A synthetic primer

<400> 66
tcctaactcc tctctccagg tttc                                             24

<210> 67
<211> 21
<212> DNA
<213> Artificial Sequence

<220>
<223> A synthetic primer

<400> 67
atgaccactt cggccactat g                                                21

<210> 68
<211> 21
<212> DNA
<213> Artificial Sequence

<220>
<223> A synthetic primer

<400> 68
gcccgaaaga cagataggct g                                                21

<210> 69
<211> 19
<212> DNA
<213> Artificial Sequence

<220>
<223> A synthetic primer

<400> 69
ctgccctctt ccgatcaca                                                    19

<210> 70
<211> 20
<212> DNA
<213> Artificial Sequence

<220>
<223> A synthetic primer

<400> 70
tatgaggagg cccacaacca                                                   20

<210> 71
<211> 17
<212> DNA
<213> Artificial Sequence

<220>
<223> A synthetic primer

<400> 71
cagctcgaac cgcagca                                                      17

<210> 72
<211> 25
<212> DNA
<213> Artificial Sequence

<220>
<223> A synthetic primer

<400> 72
gttccaagtt tgtctcaata atggc                                          25

<210> 73
<211> 21
<212> DNA
<213> Artificial Sequence

<220>
<223> A synthetic primer

<400> 73
actcaccccc aattacaacc c                                              21

<210> 74
<211> 20
<212> DNA
<213> Artificial Sequence

<220>
<223> A synthetic primer

<400> 74
ggtcacccgc agtttcactc                                                20

<210> 75
<211> 19
<212> DNA
<213> Artificial Sequence

<220>
<223> A synthetic primer

<400> 75
cgacctgcta ctcccgcat                                                 19

<210> 76
<211> 18
<212> DNA
<213> Artificial Sequence

<220>
<223> A synthetic primer

<400> 76
   cgcagcttgt ccagcaca                                                    18

<210> 77
<211> 22
<212> DNA
<213> Artificial Sequence

<220>
<223> A synthetic primer

<400> 77
tgagccagga gatcacccgc ga                                                22

<210> 78
<211> 21
<212> DNA
<213> Artificial Sequence

<220>
<223> A synthetic primer

<400> 78
gaagcttcta tggccactcc a                                                 21

<210> 79
<211> 20
<212> DNA
<213> Artificial Sequence

<220>
<223> A synthetic primer

<400> 79
agcacaagga tgcccacatt                                                   20

<210> 80
<211> 28
<212> DNA
<213> Artificial Sequence

<220>
<223> A synthetic primer

<400> 80
atcctcatcg tctcctccta cgccttcc 28

<210> 81
<211> 20
<212> DNA
<213> Artificial Sequence

<220>
<223> A synthetic primer

<400> 81
gactatggag ctcagggcga 20

<210> 82
<211> 22
<212> DNA
<213> Artificial Sequence

<220>
<223> A synthetic primer

<400> 82
cacactcata agccaggaag cc 22

<210> 83
<211> 24
<212> DNA
<213> Artificial Sequence

<220>
<223> A synthetic primer

<400> 83
aagtgggccg agaactgggt gctg                                              24

<210> 84
<211> 22
<212> DNA
<213> Artificial Sequence

<220>
<223> A synthetic primer

<400> 84
ctggacctca gggactatgg ag                                                22

<210> 85
<211> 21
<212> DNA
<213> Artificial Sequence

<220>
<223> A synthetic primer

<400> 85
acactcgtaa gccaggaagc c                                                 21

<210> 86
<211> 24
<212> DNA
<213> Artificial Sequence

<220>
<223> A synthetic primer

<400> 86
tcagggcgac tgtgaccctg aagc                                              24

<210> 87
<211> 20
<212> DNA
<213> Artificial Sequence

<220>
<223> A synthetic primer

<400> 87
atggagtgga tgatgcagca                                                    20

<210> 88
<211> 20
<212> DNA
<213> Artificial Sequence

<220>
<223> A synthetic primer

<400> 88
ttcactcgag attcgggctt                                                    20

<210> 89
<211> 23
<212> DNA
<213> Artificial Sequence

<220>
<223> A synthetic primer

<400> 89
cggaatgcca tgcagattgt ggg                                                23

<210> 90
<211> 20
<212> DNA
<213> Artificial Sequence

<220>
<223> A synthetic primer

<400> 90
gtgtgggaac ttcgtcaggc                                                    20

<210> 91
<211> 19
<212> DNA
<213> Artificial Sequence

<220>
<223> A synthetic primer

<400> 91
ctcgagtcgc tcaggctcc                                                    19

<210> 92
<211> 28
<212> DNA
<213> Artificial Sequence

<220>
<223> A synthetic primer

<400> 92
cctacaaccc catgtgcacc tatgtgaa                                          28

<210> 93
<211> 19
<212> DNA
<213> Artificial Sequence

<220>
<223> A synthetic primer

<400> 93
tcatcccctt ccagttcgc                                                    19

<210> 94
<211> 19
<212> DNA
<213> Artificial Sequence

<220>
<223> A synthetic primer

<400> 94
aggctcttcc agcggtcct                                                    19

<210> 95
<211> 21
<212> DNA
<213> Artificial Sequence

<220>
<223> A synthetic primer

<400> 95
tggccgcgct cctttaccct c                                                 21

<210> 96
<211> 20
<212> DNA
<213> Artificial Sequence

<220>
<223> A synthetic primer

<400> 96
ggagtgttga gcgggctaag                                                   20

<210> 97
<211> 20
<212> DNA
<213> Artificial Sequence

<220>
<223> A synthetic primer

<400> 97
cctccacatt ccagtttggc                                                   20

<210> 98
<211> 26
<212> DNA
<213> Artificial Sequence

<220>
<223> A synthetic primer

<400> 98
ctctgacccc aggagtcact caaggc                                          26

<210> 99
<211> 29
<212> DNA
<213> Artificial Sequence

<220>
<223> A synthetic primer

<400> 99
gaaaatatcg acgattgtcc aggaaacaa                                       29

<210> 100
<211> 23
<212> DNA
<213> Artificial Sequence

<220>
<223> A synthetic primer

<400> 100
acatgggccg ctgtccactc cat                                             23

<210> 101
<211> 22
<212> DNA
<213> Artificial Sequence

<220>
<223> A synthetic primer

<400> 101
cccatgggca agactggcct cc                                              22

<210> 102
<211> 23
<212> DNA
<213> Artificial Sequence

<220>
<223> A synthetic primer

<400> 102
tgtgtctgcc ccgtgcttca atg                                              23

<210> 103
<211> 25
<212> DNA
<213> Artificial Sequence

<220>
<223> A synthetic primer

<400> 103
atggagacaa ctggtaccgg tgcga                                            25

<210> 104
<211> 21
<212> DNA
<213> Artificial Sequence

<220>
<223> A synthetic primer

<400> 104
tggccgcgct cctttaccct c                                                21

<210> 105
<211> 30
<212> DNA
<213> Artificial Sequence

<220>
<223> A synthetic primer

<400> 105
tgacctcgca acagaaaacc cagaaagact                                        30

<210> 106
<211> 21
<212> DNA
<213> Artificial Sequence

<220>
<223> A synthetic primer

<400> 106
cggcctcttg caggtgccct t                                                 21

<210> 107
<211> 20
<212> DNA
<213> Artificial Sequence

<220>
<223> A synthetic primer

<400> 107
tcctgcctgc ccggttggac                                                   20

<210> 108
<211> 19
<212> DNA
<213> Artificial Sequence

<220>
<223> A synthetic primer

<400> 108
cgcctcaaaa agtgcctca                                                    19

<210> 109
<211> 17
<212> DNA
<213> Artificial Sequence

<220>
<223> A synthetic primer

<400> 109
gcatcctgcc cctctgc                                                    17

<210> 110
<211> 22
<212> DNA
<213> Artificial Sequence

<220>
<223> A synthetic primer

<400> 110
agtgggcatg agacgggaag cg                                              22

<210> 111
<211> 21
<212> DNA
<213> Artificial Sequence

<220>
<223> A synthetic primer

<400> 111
tcgggaaggc tactggccgg a                                               21

<210> 112
<211> 27
<212> DNA
<213> Artificial Sequence

<220>
<223> A synthetic primer

<400> 112
tccactctgg cttcgtgctt acttcca                                         27

<210> 113
<211> 26
<212> DNA
<213> Artificial Sequence

<220>
<223> A synthetic primer

<400> 113
atgaagaaaa caccggagcg gacagg                                          26

<210> 114
<211> 20
<212> DNA
<213> Artificial Sequence

<220>
<223> A synthetic primer

<400> 114
accaaggcca gcacatagga                                                 20

<210> 115
<211> 20
<212> DNA
<213> Artificial Sequence

<220>
<223> A synthetic primer

<400> 115
aggcccacag ggattttctt                                                 20

<210> 116
<211> 34
<212> DNA
<213> Artificial Sequence

<220>
<223> A synthetic primer

<400> 116
agatgagctt cctacagcac aacaaatgtg aatg                                34

<210> 117
<211> 20
<212> DNA
<213> Artificial Sequence

<220>
<223> A synthetic primer

<400> 117
tttgcgccaa agatgtcaga                                                20

<210> 118
<211> 20
<212> DNA
<213> Artificial Sequence

<220>
<223> A synthetic primer

<400> 118
agtaacgccc aatgtgaggg                                                20

<210> 119
<211> 30
<212> DNA
<213> Artificial Sequence

<220>
<223> A synthetic primer

<400> 119
aaagctttga cctggagcct gactcaaatc                                     30

<210> 120
<211> 20
<212> DNA
<213> Artificial Sequence

<220>
<223> A synthetic primer

<400> 120
gccttggtgt gtgacaatgg                                                    20

<210> 121
<211> 21
<212> DNA
<213> Artificial Sequence

<220>
<223> A synthetic primer

<400> 121
cgtcacccac gtagctgtct t                                                  21

<210> 122
<211> 23
<212> DNA
<213> Artificial Sequence

<220>
<223> A synthetic primer

<400> 122
tctgtaaggc cggctttgct ggg                                                23

<210> 123
<211> 21
<212> DNA
<213> Artificial Sequence

<220>
<223> A synthetic primer

<400> 123
tcatcaaggc catcaccaag t                                                  21

<210> 124
<211> 20
<212> DNA
<213> Artificial Sequence

<220>
<223> A synthetic primer

<400> 124
cccacgttca ccttgtttcc                                                    20

<210> 125
<211> 25
<212> DNA
<213> Artificial Sequence

<220>
<223> A synthetic primer

<400> 125
tgaagcccca cgacattttt gaggc                                              25

<210> 126
<211> 19
<212> DNA
<213> Artificial Sequence

<220>
<223> A synthetic primer

<400> 126
tctcagccag ccacatcca                                                     19

<210> 127
<211> 18
<212> DNA
<213> Artificial Sequence

<220>
<223> A synthetic primer

<400> 127
gcggctcatg ccatagga                                                    18

<210> 128
<211> 20
<212> DNA
<213> Artificial Sequence

<220>
<223> A synthetic primer

<400> 128
tgtaagggtg caggcgccgg                                                  20

<210> 129
<211> 20
<212> DNA
<213> Artificial Sequence

<220>
<223> A synthetic primer

<400> 129
tcccgcagac tatgctcatc                                                  20

<210> 130
<211> 18
<212> DNA
<213> Artificial Sequence

<220>
<223> A synthetic primer

<400> 130
ctccagtgag tccgggct                                                    18

<210> 131
<211> 20
<212> DNA
<213> Artificial Sequence

<220>
<223> A synthetic primer

<400> 131
[?]caacctgag cgccctggcc                                                20

<210> 132
<211> 20
<212> DNA
<213> Artificial Sequence

<220>
<223> A synthetic primer

<400> 132
ctacgccaac atgaactcca                                                20

<210> 133
<211> 22
<212> DNA
<213> Artificial Sequence

<220>
<223> A synthetic primer

<400> 133
ctcgtacatc tcgctcatca cc                                             22

<210> 134
<211> 18
<212> DNA
<213> Artificial Sequence

<220>
<223> A synthetic primer

<400> 134
acgcacgcaa agccgccc                                                  18

<210> 135
<211> 20
<212> DNA
<213> Artificial Sequence

<220>
<223> A synthetic primer

<400> 135
gaagagctgg cctacctgaa                                                    20

<210> 136
<211> 20
<212> DNA
<213> Artificial Sequence

<220>
<223> A synthetic primer

<400> 136
gacatgcgaa gccaatatga                                                    20

<210> 137
<211> 20
<212> DNA
<213> Artificial Sequence

<220>
<223> A synthetic primer

<400> 137
aggtggattc cgctccgggc                                                    20

<210> 138
<211> 18
<212> DNA
<213> Artificial Sequence

<220>
<223> A synthetic primer

<400> 138
gctgccaagg cccaggaa                                                    18

<210> 139
<211> 20
<212> DNA
<213> Artificial Sequence

<220>
<223> A synthetic primer

<400> 139
caaaaactcg tgctgctttg                                                  20

<210> 140
<211> 30
<212> DNA
<213> Artificial Sequence

<220>
<223> A synthetic primer

<400> 140
aggaagtctg ctttgctgaa gagggacaaa                                       30

<210> 141
<211> 20
<212> DNA
<213> Artificial Sequence

<220>
<223> A synthetic primer

<400> 141
tatgaggccc tgctgaacat                                                  20

<210> 142
<211> 20
<212> DNA
<213> Artificial Sequence

<220>
<223> A synthetic primer

<400> 142
agcaactcca tgcaaaccat                                                    20

<210> 143
<211> 19
<212> DNA
<213> Artificial Sequence

<220>
<223> A synthetic primer

<400> 143
acctaccgcc gcctgctaa                                                     19

<210> 144
<211> 20
<212> DNA
<213> Artificial Sequence

<220>
<223> A synthetic primer

<400> 144
caggagaaac agggcctaca                                                    20

<210> 145
<211> 20
<212> DNA
<213> Artificial Sequence

<220>
<223> A synthetic primer

<400> 145
gtcttggatc tttgctccca                                                    20

<210> 146
<211> 20
<212> DNA
<213> Artificial Sequence

<220>
<223> A synthetic primer

<400> 146
caggagaaac agggcctaca                                                    20

<210> 147
<211> 20
<212> DNA
<213> Artificial Sequence

<220>
<223> A synthetic primer

<400> 147
tccagtcgaa gattgggtcc                                                    20

<210> 148
<211> 26
<212> DNA
<213> Artificial Sequence

<220>
<223> A synthetic primer

<400> 148
aaataaaaag attgaaaccc acaagc                                             26

<210> 149
<211> 22
<212> DNA
<213> Artificial Sequence

<220>
<223> A synthetic primer

<400> 149
tatcacccac gtccctggcg ga                                                22

<210> 150
<211> 19
<212> DNA
<213> Artificial Sequence

<220>
<223> A synthetic primer

<400> 150
aagatcaacc tgccggagt                                                    19

<210> 151
<211> 20
<212> DNA
<213> Artificial Sequence

<220>
<223> A synthetic primer

<400> 151
aagaagtcct ctccagtgcg                                                   20

<210> 152
<211> 19
<212> DNA
<213> Artificial Sequence

<220>
<223> A synthetic primer

<400> 152
ttcaagaacc gccgcgcca                                                    19